# EUROPEAN PATENT APPLICATION

(11) **EP 1 491 209 A1**
(43) Date of publication of application: **29.12.2004**
(21) Application number: 03743073.3
(22) Date of filing: 28.02.2003
(51) Int. Cl.: A61K 39/395, A61K 38/17, A61P 11/00, G01N 33/50

(54) **DIAGNOSTICS AND REMEDIES FOR INTERSTITIAL PNEUMONIA**

(30) Priority: 28.02.2002 JP 2002054764
(71) Applicant: KYOWA HAKKO KOGYO CO., LTD., Chiyoda-ku, Tokyo 100-8185 (JP)
(72) Inventor: UEDA, Ryuzo, Nagoya-shi, Aichi 465-0051 (JP); SATO, Shigeki, Room 303, Mezon Shougetsu, Nagoya-shi, Aichi 467-0004 (JP); YOSHINOUCHI, Takeo, Room 202, Yakumo Shitihausu, Nagoya-shi, Aichi 466-0823 (JP); NIIMI, Takashi, Room 102, Yagotoharuyama Hiruzu, Nagoya-shi, Aichi 467-0024 (JP); SHIMIZU, Shigeki, Room 501, Komaba Koopu, Nagoya-shi, Aichi 467-0807 (JP); EIMOTO, Tadaaki, Nagoya-shi, Aichi 468-0063 (JP)
(74) Representative: VOSSIUS & PARTNER
(86) International application number: PCT/JP2003/002372
(87) International publication number: WO 2003/072134

(57) **Abstract**

A preventive agent, a diagnostic agent or a therapeutic agent for interstitial pneumonia, which comprises an anti-CCR4 antibody and/or an anti-CXCR3 antibody as an active ingredient, a diagnostic agent for discriminating between usual idiopathic interstitial pneumonia and non-specific idiopathic interstitial pneumonia which comprises an anti-CCR4 antibody and an anti-CXCR3 antibody as an active ingredient, and a method for discriminating between usual idiopathic interstitial pneumonia and non-specific idiopathic interstitial pneumonia, which comprises detecting and/or determining a Th2 cell and a Th1 cell in a sample by using an anti-CCR4 antibody and an anti-CXCR3 antibody.

## Description

### Technical Field

The present invention relates to a preventive agent, a diagnostic agent or a therapeutic agent for interstitial pneumonia, which comprises an anti-CCR4 (CC chemokine receptor 4) antibody and/or an anti-CXCR3 (CRC chemokine receptor 3) antibody as the active ingredient, and a diagnostic agent and a discrimination method for discriminating between usual idiopathic interstitial pneumonia and non-specific idiopathic interstitial pneumonia, which comprises using an anti-CCR4 antibody and an anti-CXCR3 antibody.

### Background of the Invention

Idiopathic interstitial pneumonia is a cryptogenic inflammatory lung disease which is found in relatively old people in the case of male, and its prevalence rate is to be approximately 3.4 per 100,000 of population. The chronic idiopathic interstitial pneumonia is classified into a usual case (idiopathic pulmonary fibrosis; IPF) and a non-specific case (non-specific interstitial pneumonia; NSIP), and their difference is based mainly on X-ray findings. Characteristics of the idiopathic interstitial pneumonia pathologically include accumulation of mononuclear cells into a lung tissue and subsequent deposition of collagen and the like and development of fibrosis, which cause destruction of the lung tissue and reduction of a respiratory function. The pathological characteristics are almost the same in both of the usual case and non-specific case. Since infiltration of mononuclear cells, particularly CD4-positive T cell, is observed in focal regions, it is considered that an immunological mechanism is concerned in the pathogenesis of the disease. However, details of the immunological mechanism are unclear, and the cause of idiopathic interstitial pneumonia, pathophysiological difference between the usual case and non-specific case and the like have not been found.

In addition, the interstitial pneumonia is known as the symptoms frequently observed in collagen diseases including erythematosus, rheumatoid arthritis (RA) and scleroderma. It is considered that the frequency is the highest in scleroderma with a ratio of 50 to 70% or more, and is 5 to 20% in the case of rheumatoid arthritis (*Outline of the Latest Internal Medicine, Immune and Allergic Diseases* 3 (1993)). Although the patients show symptoms such as shortness of breath, effective therapeutic methods for interstitial pneumonia in collagen diseases are not known, and they are generally resistant to the current therapeutic methods. The cause of lung lesions in collagen diseases has hardly been found.

Inflammation is frequently observed in autoimmunity, infection, allergy, graft rejection and the like, and accumulation of inflammatory cells including T cell is observed in common.

With the development of immunology in recent years, it has been found that a CD4-positive T cell plays a central role in various diseases. Depending on the kinds of cytokine produced thereby, the CD4-positive T cell is roughly classified into two types, namely Th1 cell and Th2 cell (*Annu*. *Rev*. *Immunol.,* 7, 145 (1989)). It is considered that the Th1 cell is mainly concerned in the cellular immunity by producing interferon-γ, interleukin-2 and the like. It is considered that the Th2 cell controls humoral immune reactions by secreting cytokines such as interleukin-4, -5 and -13. Accumulation of a T cell is observed in an inflammatory site, but each T cell subgroup is accumulated in each inflammatory region. For example, the Th1 cell is significantly accumulated in inflammatory region in rheumatoid arthritis, and the Th2 cell in allergic diseases such as bronchial asthma (*Immunology Today,* 21, 183 (2000)).

For example, the Th2 cell is present in the broncho-alveolar lavage fluid or airway mucosa of bronchial asthma patients (*N*. *Engl*. *J*. *Med.,* 326, 298 (1992), *Eur. J*. *Immunol*., 23, 1445 (1993)), and concern of the Th2 cell in the pathogenesis of asthma in an asthma model animal has also been shown (*Clin*. *Immunol*. *Immunopathol*., 75, 75 (1995), *J. Exp*. *Med.,* 186, 1737 (1997), *J*. *Immunol.,* 160, 1378 (1998), *J. Immunol.,* 161, 3128 (1998)). These findings are also a ground for a theory in which control of the Th2 cell or control of the activity of a cytokine produced by the Th2 is important for the treatment of asthma (*Nature*, 402, B31 (1999)). Actually, asthma treating agents under development based on the control of the Th2 cell as a concept include a humanized anti-interleukin-5 antibody (SB-240563: manufactured by Glaxo-Smith-Kline-Beecham, SCH-55700: manufactured by Schering-Plough/Celltech), a humanized anti-interleukin-4 antibody (US Patent No. 5,914,110), a soluble interleukin receptor (Nuvance: manufactured by Immunex), a humanized anti-interleukin-5 receptor antibody (WO97/10354), an anti-CCR8 antibody (WO99/25734), an anti-CCR4 antibody (WO01/64754) and the like.

It has been shown that various chemokine receptors are expressed on respective leukocyte surfaces such as of Th1 cell and Th2 cell, and the respectively expressed chemokine receptors are different from one another (*Immunol. Today,* 21, 418 (2000)). Chemokine is a general term of basic heparin-binding proteins having leukocyte migration and leukocyte activating actions, and many receptors have been identified (*Curr. Opi*. *Immunol.,* 11, 626 (1999)).

Human CCR4 is a chemokine receptor of which gene has been cloned from a human immature basophile cell line KU-812 (*J. Biol*. *Chem.,* 270, 19495 (1995)), and it has been found that its ligands are TARC (thymus and activation-regulated chemokine) and MDC (macrophage-derived chemokine) (*Curr*. *Opin*. *Immunol*., 11, 626 (1999)). In addition, based on the examinations in recent years, it has been found that CCR4 is selectively expressed in the Th2 cell (*J*. *Exp*. *Med.,* 187, 129 (1998), *J.* *Immunol*., 161, 5111 (1998), *Int*. *Immunol*., 11, 81 (1999), *J. Clin*. *Invest*., 108, 1331 (2001)), and it has been suggested that CCR4 and its ligands are concerned in inflammatory reactions via migration and activation of the Th2 cell. Actually, it has been found that the ratio of the CCR4-positive and CD4-positive cell in peripheral blood is high in patients of atopic dermatitis in which the Th2 cell is considered to play an important role in pathogenesis of the disease (*J*. *Leuk*. *Biol*., 68, 568 (2000)), and that CCR4-positive cells are accumulated in a skin lesion (*J*. *Allergy Clin*. *Immunol.,* 107, 353 (2001)). In addition, it has been also reported in atopic bronchial asthma that the ratio of CCR4-positive cells is reduced accompanied by its treatment with a steroid hormone preparation (*Am*. *J. Respir. Crit. Care Med*., 164, 754 (2001)), and that CCR4 is expressed in the CD4-positive T cell infiltrating into an airway region after antigen inhalation (*J. Clin*. *Invest*., 107, 1357 (2001)). In autoimmune diseases, it has been reported that CCR4-positive cells are increased in peripheral blood at the active stage in patients of systemic lupus erythematosus (*J*. *Leukocyte Biol*., 70, 749 (2001)), but a relationship between interstitial pneumonia and CCR4-positive cell and a relationship between interstitial pneumonia and Th2 cell have not been found.

In general, in order to provide a therapeutic method having high usefulness, deep understanding is necessary on pathogenesis of the disease and cause of the disease, but as described above, the cause and the like of interstitial pneumonia in idiopathic interstitial pneumonia and collagen disease have hardly been elucidated and its effective therapeutic method has not been established yet. Treatment of this disease is based on the use of steroids, and only immunosuppressants such as azathioprine, cyclophosphamide and cyclosporin are used as adjuvands. It is well known that steroids generate side effects such as osteoporosis, glaucoma and cataract by prolonged use thereof, in addition to problems such as resistance acquisition and the like. Also, the use of an immunosuppressants cannot necessarily be considered as an appropriate therapeutic method when a possibility of causing a serious infection or the like is taken into consideration, so that development of a more safe and effective therapeutic agent is required.

In addition, in the case of the idiopathic interstitial pneumonia, it is important in considering its treatment to understand pathological characteristics of inflammation of its usual case and non-specific case, but effective diagnostic methods have not been shown. Also in the case of the interstitial pneumonia caused by a collagen disease, basic analyses, such as whether or not the cause of a lung lesion varies depending on the respective collagen disease-induced diseases (dermatomyositis, chronic arthritis, rheumatism, *etc*.), have not been carried out. Concern has been directed toward the development of a more superior therapeutic agent to be used in the treatment of asthma too, from the viewpoint of efficacy and safety, and elucidation of pathogenesis of the disease is the possibility. That is, it is considered that the elucidation of morbid states is essential in developing a therapeutic method, and in the case of the interstitial pneumonia in idiopathic interstitial pneumonia and collagen disease, its elucidation and development of a diagnostic method are also required.

### Disclosure of the Invention

An object of the present invention is to provide a preventive agent, a diagnostic agent or a therapeutic agent for interstitial pneumonia, particularly a preventive agent, a diagnostic agent or a therapeutic agent for the interstitial pneumonia in usual case idiopathic interstitial pneumonia and collagen disease, and a diagnostic agent for discriminating between usual idiopathic interstitial pneumonia and non-specific idiopathic interstitial pneumonia.

Another object of the present invention is to provide a method for discriminating between usual idiopathic interstitial pneumonia and non-specific idiopathic interstitial pneumonia.

The present invention relates to the following (1) to (55).
(1) A preventive agent, a diagnostic agent or a therapeutic agent for interstitial pneumonia, which comprises an anti-CCR4 antibody and/or an anti-CXCR3 antibody as an active ingredient.
(2) The preventive agent, the diagnostic agent or the therapeutic agent according to the above (1), wherein the interstitial pneumonia is usual idiopathic interstitial pneumonia or non-specific idiopathic interstitial pneumonia.
(3) The preventive agent, the diagnostic agent or the therapeutic agent according to the above (1), wherein the interstitial pneumonia is interstitial pneumonia in collagen disease.
(4) A diagnostic agent for discriminating between usual idiopathic interstitial pneumonia and non-specific idiopathic interstitial pneumonia, which comprises an anti-CCR4 antibody and an anti-CXCR3 antibody as an active ingredient.
(5) The preventive agent, the diagnostic agent or the therapeutic agent according to any one of the above (1) to (4), wherein the anti-CCR4 antibody is an antibody which specifically binds to an extracellular region of CCR4.
(6) The preventive agent, the diagnostic agent or the therapeutic agent according to the above (5), wherein the extracellular region is an extracellular region selected from the group consisting of positions 1 to 39, 98 to 112, 176 to 206 and 271 to 284 in the amino acid sequence represented by SEQ ID NO:1.
(7) The preventive agent, the diagnostic agent or the therapeutic agent according to the above (5), wherein the extracellular region is an epitope present in positions 2 to 29 in the amino acid sequence represented by SEQ ID NO:1.
(8) The preventive agent, the diagnostic agent or the therapeutic agent according to the above (5), wherein the extracellular region is an epitope present in positions 13 to 29 in the amino acid sequence represented by SEQ ID NO:1.
(9) The preventive agent, the diagnostic agent or the therapeutic agent according to any one of the above (1) to (8), wherein the anti-CCR4 antibody is a polyclonal antibody, a monoclonal antibody or an antibody fragment thereof.
(10) The preventive agent, the diagnostic agent or the therapeutic agent according to any one of the above (1) to (8), wherein the anti-CCR4 antibody is a recombinant antibody or an antibody fragment thereof.
(11) The preventive agent, the diagnostic agent or the therapeutic agent according to the above (10), wherein the recombinant antibody is an antibody selected from the group consisting of a humanized antibody and an antibody fragment thereof.
(12) The preventive agent, the diagnostic agent or the therapeutic agent according to the above (11), wherein the humanized antibody or the antibody fragment thereof comprises:
   complementarity determining region (CDR) 1, CDR2 and CDR3 of an antibody heavy chain (H chain) variable region (V region) having the amino acid sequences represented by SEQ ID NOs:2, 3 and 4, respectively, and/or
   CDR1, CDR2 and CDR3 of an antibody light chain (L chain) V region having the amino acid sequences represented by SEQ ID NOs:5, 6 and 7, respectively.
(13) The preventive agent, the diagnostic agent or the therapeutic agent according to the above (11) or (12), wherein the humanized antibody is an antibody selected from the group consisting of a human chimeric antibody, a human complementarity determining region (CDR)-grafted antibody, and an antibody fragment thereof.
(14) The preventive agent, the diagnostic agent or the therapeutic agent according to the above (13), wherein the human chimeric antibody or the antibody fragment thereof comprises:
   a heavy chain (H chain) variable region (V region) and a light chain (L chain) V region of a monoclonal antibody against CCR4, and
   an H chain constant region (C region) and an L chain C region of a human antibody.
(15) The preventive agent, the diagnostic agent or the therapeutic agent according to the above (13) or (14), wherein the human chimeric antibody or the antibody fragment thereof comprises:
   CDR1, CDR2 and CDR3 of an antibody heavy chain (H chain) variable region (V region) having the amino acid sequences represented by SEQ ID NOs:2, 3 and 4, respectively, and/o the above r
   CDR1, CDR2 and CDR3 of an antibody light chain (L chain) V region having the amino acid sequences represented by SEQ ID NOs:5, 6 and 7, respectively.
(16) The preventive agent, the diagnostic agent or the therapeutic agent according to any one of the above (13) to (15), wherein the human chimeric antibody or the antibody fragment thereof comprises:
   a heavy chain (H chain) variable region (V region) of an antibody comprising the amino acid sequence represented by SEQ ID NO:8, and/or
   a light chain (L chain) V region of an antibody comprising the amino acid sequence represented by SEQ ID NO:9.
(17) The preventive agent, the diagnostic agent or the therapeutic agent according to any one of the above (13) to (16), wherein the human chimeric antibody or the antibody fragment thereof is an antibody KM2760 produced by a transformant KM2760 (FERM BP-7054).
(18) The preventive agent, the diagnostic agent or the therapeutic agent according to the above (13), wherein the human CDR-grafted antibody or the antibody fragment thereof comprises CDRs of a heavy chain (H chain) variable region (V region) and a light chain (L chain) V region of a monoclonal antibody against CCR4.
(19) The preventive agent, the diagnostic agent or the therapeutic agent according to the above (13), wherein the human CDR-grafted antibody or the antibody fragment thereof comprises:
   CDRs of a heavy chain (H chain) variable region (V region) and a light chain (L chain) V region of a monoclonal antibody against CCR4, and
   framework regions (FRs) of an H chain V region and an L chain V region of a human antibody.
(20) The preventive agent, the diagnostic agent or the therapeutic agent according to the above (13), wherein the human CDR-grafted antibody or the antibody fragment thereof comprises:
   CDRs of a heavy chain (H chain) variable region (V region) and a light chain (L chain) V region of a monoclonal antibody against CCR4,
   framework regions (FRs) of an H chain V region and an L chain V region of a human antibody, and
   an H chain constant region (C region) and an L chain C region of a human antibody.
(21) The preventive agent, the diagnostic agent or the therapeutic agent according to any one of the above (13) and (18) to (20), wherein the human CDR-grafted antibody or the antibody fragment thereof comprises:
   CDR1, CDR2 and CDR3 of an antibody heavy chain (H chain) variable region (V region) having the amino acid sequences represented by SEQ ID NOs:2, 3 and 4, respectively, and/or
   CDR1, CDR2 and CDR3 of an antibody light chain (L chain) V region having the amino acid sequences represented by SEQ ID NOs:5, 6 and 7, respectively.
(22) The preventive agent, the diagnostic agent or the therapeutic agent according to any one of the above (13) and (18) to (21), wherein the human CDR-grafted antibody or the antibody fragment thereof comprises:
   a heavy chain (H chain) variable region (V region) of an antibody comprising an amino acid sequence in which at least one amino acid residue selected from Ala at position 40, Gly at position 42, Lys at position 43, Gly at position 44, Lys at position 76 and Ala at position 97 in the amino acid sequence represented by SEQ ID NO:10 is substituted with other amino acid, and/or
   a light chain (L chain) variable region (V region) of an antibody comprising an amino acid sequence in which at least one amino acid residue selected from Ile at position 2, Val at position 3, Gln at position 50 and Val at position 88 in the amino acid sequence represented by SEQ ID NO:12 is substituted with other amino acid.
(23) The preventive agent, the diagnostic agent or the therapeutic agent according to any one of the above (13) and (18) to (21), wherein the human CDR-grafted antibody or the antibody fragment thereof comprises:
   a heavy chain (H chain) variable region (V region) of an antibody comprising an amino acid sequence in which at least one amino acid residue selected from Thr at position 28 and Ala at position 97 in the amino acid sequence represented by SEQ ID NO:11 is substituted with other amino acid, and/or
   a light chain (L chain) V region of an antibody comprising an amino acid sequence in which at least one amino acid residue selected from Ile at position 2, Val at position 3, Gln at position 50 and Val at position 88 in the amino acid sequence represented by SEQ ID NO:12 is substituted with other amino acid.
(24) The preventive agent, the diagnostic agent or the therapeutic agent according to any one of the above (13) and (18) to (21), wherein the human CDR-grafted antibody or the antibody fragment thereof comprises:
   a heavy chain (H chain) variable region (V region) of an antibody comprising the amino acid sequence selected from SEQ ID NOs:10, 11 and 13 to 18, and/or
   a light chain (L chain) V region of an antibody comprising the amino acid sequence selected from SEQ ID NOs:12 and 19 to 21.
(25) The preventive agent, the diagnostic agent or the therapeutic agent according to any one of the above (13) and (18) to (21), wherein the human CDR-grafted antibody or the antibody fragment thereof comprises:
   a heavy chain (H chain) variable region (V region) of an antibody comprising the amino acid sequence represented by SEQ ID NO:13, and/or
   a light chain (L chain) V region of an antibody comprising the amino acid sequence represented by SEQ ID NO:21.
(26) The preventive agent, the diagnostic agent or the therapeutic agent according to any one of the above (13) and (18) to (21), wherein the human CDR-grafted antibody or the antibody fragment thereof comprises:
   a heavy chain (H chain) variable region (V region) of an antibody comprising the amino acid sequence represented by SEQ ID NO:14, and/or
   a light chain (L chain) V region of an antibody comprising the amino acid sequence represented by SEQ ID NO:21.
(27) The preventive agent, the diagnostic agent or the therapeutic agent according to any one of the above (13) and (18) to (21), wherein the human CDR-grafted antibody or the antibody fragment thereof is a human CDR-grafted antibody produced by a transformant selected from the group consisting of FERM BP-8129 and FERM BP-8130 or an antibody fragment thereof.
(28) The preventive agent, the diagnostic agent or the therapeutic agent according to any one of the above (9) to (27), wherein the antibody fragment is an antibody fragment selected from Fab, Fab', F(ab')₂, a single chain antibody (scFv), a dimerized variable region (V region) fragment (Diabody), a disulfide-stabilized V region fragment (dsFv) and a peptide comprising CDR.
(29) The preventive agent, the diagnostic agent or the therapeutic agent according to any one of the above (9) to (28), wherein the antibody or the antibody fragment thereof according to any one of the above (9) to (28) is chemically or genetically bound to a radioisotope, a protein or an agent.
(30) A the above method for discriminating between usual idiopathic interstitial pneumonia and non-specific idiopathic interstitial pneumonia, which comprises detecting and/or determining a Th2 cell and a Th1 cell in a sample by using an anti-CCR4 antibody and an anti-CXCR3 antibody.
(31) The method according to the above (30), wherein the anti-CCR4 antibody is an antibody which specifically binds to an extracellular region of CCR4.
(32) The method according to the above (31), wherein the extracellular region is an extracellular region selected from the group consisting of positions 1 to 39, 98 to 112, 176 to 206 and 271 to 284 in the amino acid sequence represented by SEQ ID NO:1.
(33) The method according to the above (31), wherein the extracellular region is an epitope present in positions 2 to 29 in the amino acid sequence represented by SEQ ID NO:1.
(34) The method according to the above (31), wherein the extracellular region is an epitope present in positions 13 to 29 in the amino acid sequence represented by SEQ ID NO:1.
(35) The method according to any one of the above (31) to (34), wherein the anti-CCR4 antibody is a polyclonal antibody, a monoclonal antibody or an antibody fragment thereof.
(36) The method according to any one of the above (31) to (34), wherein the anti-CCR4 antibody is a recombinant antibody or an antibody fragment thereof.
(37) The method according to the above (36), wherein the recombinant antibody is an antibody selected from a humanized antibody and an antibody fragment thereof.
(38) The method according to the above (37), wherein the humanized antibody or the antibody fragment thereof comprises:
   complementarity determining region (CDR) 1, CDR2 and CDR3 of an antibody heavy chain (H chain) variable region (V region) having the amino acid sequences represented by SEQ ID NOs:2, 3 and 4, respectively, and/or
   CDR1, CDR2 and CDR3 of an antibody light chain (L chain) V region having the amino acid sequences represented by SEQ ID NOs:5, 6 and 7, respectively.
(39) The method according to the above (37) or (38), wherein the humanized antibody is an antibody selected from a human chimeric antibody, a human complementarity determining region (CDR)-grafted antibody, and an antibody fragment thereof.
(40) The method according to the above (39), wherein the human chimeric antibody or the antibody fragment thereof comprises:
   a heavy chain (H chain) variable region (V region) and a light chain (L chain) V region of a monoclonal antibody against CCR4, and
   an H chain constant region (C region) and an L chain C region of a human antibody.
(41) The method according to the above (39) or (40), wherein the human chimeric antibody or the antibody fragment thereof comprises:
   CDR1, CDR2 and CDR3 of an antibody heavy chain (H chain) variable region (V region) having the amino acid sequences represented by SEQ ID NOs:2, 3 and 4, respectively, and/or
   CDR1, CDR2 and CDR3 of an antibody light chain (L chain) V region having the amino acid sequences represented by SEQ ID NOs:5, 6 and 7, respectively.
(42) The method according to any one of the above (39) to (41), wherein the human chimeric antibody or the antibody fragment thereof comprises:
   a heavy chain (H chain) variable region (V region) of an antibody comprising the amino acid sequence represented by SEQ ID NO:8, and/or
   a light chain (L chain) V region of an antibody comprising the amino acid sequence represented by SEQ ID NO:9.
(43) The method according to any one of the above (39) to (42), wherein the human chimeric antibody or the antibody fragment thereof is an antibody KM2760 produced by a transformant KM2760 (FERM BP-7054).
(44) The method according to the above (39), wherein the human CDR-grafted antibody or the antibody fragment thereof comprises CDRs of a heavy chain (H chain) variable region (V region) and a light chain (L chain) V region of a monoclonal antibody against CCR4.
(45) The method according to the above (39), wherein the human CDR-grafted antibody or the antibody fragment thereof comprises:
   CDRs of a heavy chain (H chain) variable region (V region) and a light chain (L chain) V region of a monoclonal antibody against CCR4, and
   framework regions (FRs) of an H chain V region and an L chain V region of a human antibody.
(46) The method according to the above (39), wherein the human CDR-grafted antibody or the antibody fragment thereof comprises:
   CDRs of a heavy chain (H chain) variable region (V region) and a light chain (L chain) V region of a monoclonal antibody against CCR4,
   framework regions (FRs) of an H chain V region and an L chain V region of a human antibody, and
   an H chain constant region (C region) and an L chain C region of a human antibody.
(47) The method according to any one of the above (39) and (44) to (46), wherein the human CDR-grafted antibody or the antibody fragment thereof comprises:
   CDR1, CDR2 and CDR3 of an antibody heavy chain (H chain) variable region (V region) having the amino acid sequences represented by SEQ ID NOs:2, 3 and 4, respectively, and/or
   CDR1, CDR2 and CDR3 of an antibody light chain (L chain) V region having the amino acid sequences represented by SEQ ID NOs:5, 6 and 7, respectively.
(48) The method according to any one of the above (39) and (44) to (47), wherein the human CDR-grafted antibody or the antibody fragment thereof comprises:
   a heavy chain (H chain) variable region (V region) of an antibody comprising an amino acid sequence in which at least one amino acid residue selected from Ala at position 40, Gly at position 42, Lys at position 43, Gly at position 44, Lys at position 76 and Ala at position 97 in the amino acid sequence represented by SEQ ID NO:10 is substituted with other amino acid, and/or
   a light chain (L chain) variable region (V region) of an antibody comprising an amino acid sequence in which at least one amino acid residue selected from Ile at position 2, Val at position 3, Gln at position 50 and Val at position 88 in the amino acid sequence represented by SEQ ID NO:12 is substituted with other amino acid.
(49) The method according to any one of the above (39) and (44) to (47), wherein the human CDR-grafted antibody or the antibody fragment thereof comprises:
   a heavy chain (H chain) variable region (V region) of an antibody comprising an amino acid sequence in which at least one amino acid residue selected from Thr at position 28 and Ala at position 97 in the amino acid sequence represented by SEQ ID NO:11 is substituted with other amino acid, and/or
   a antibody light chain (L chain) variable region (V region) of an antibody comprising an amino acid sequence in which at least one amino acid residue selected from Ile at position 2, Val at position 3, Gln at position 50 and Val at position 88 in the amino acid sequence represented by SEQ ID NO:12 is substituted with other amino acid.
(50) The method according to any one of the above (39) and (44) to (47), wherein the human CDR-grafted antibody or the antibody fragment thereof comprises:
   a heavy chain (H chain) variable region (V region) of an antibody comprising the amino acid sequence selected from SEQ ID NOs:10, 11 and 13 to 18, and/or
   a light chain (L chain) V region of an antibody comprising the amino acid sequence selected from SEQ ID NOs:12 and 19 to 21.
(51) The method according to any one of the above (39) and (44) to (47), wherein the human CDR-grafted antibody or the antibody fragment thereof comprises:
   a heavy chain (H chain) variable region (V region) of an antibody comprising the amino acid sequence represented by SEQ ID NO:13, and/or
   a light chain (L chain) V region of an antibody comprising the amino acid sequence represented by SEQ ID NO:21.
(52) The method according to any one of the above (39) and (44) to (47), wherein the human CDR-grafted antibody or the antibody fragment thereof comprises:
   a heavy chain (H chain) variable region (V region) of an antibody comprising the amino acid sequence represented by SEQ ID NO:14, and/or
   a light chain (L chain) V region of an antibody comprising the amino acid sequence represented by SEQ ID NO:21.
(53) The method according to any one of the above (39) and (44) to (47), wherein the human CDR-grafted antibody or the antibody fragment thereof is a human CDR-grafted antibody produced by a transformant selected from the group consisting of FERM BP-8129 and FERM BP-8130 or an antibody fragment thereof.
(54) The method according to any one of the above (35) to (53), wherein the antibody fragment is an antibody fragment selected from Fab, Fab', F(ab')₂, a single chain antibody (scFv), a dimerized variable region (V region) fragment (Diabody), a disulfide-stabilized V region fragment (dsFv) and a peptide comprising CDR.
(55) The method according to any one of the above (35) to (54), wherein the antibody or the antibody fragment thereof according to any one of the above (35) to (54) is chemically or genetically bound to a radioisotope, a protein or an agent.

The present invention is explained below in detail.

It is considered that onset of the interstitial pneumonia is a) due to a collagen disease, b) due to an infectious disease, c) caused by a radioactive ray, d) originated from an occupation or environment, e) caused by a drug or f) induced by sarcoidosis, eosinophilic granuloma, AIDS or the like, and an interstitial pneumonia whose onset cannot be specified is called idiopathic interstitial pneumonia.

Among the interstitial pneumonia, the idiopathic interstitial pneumonia includes non-specific idiopathic interstitial pneumonia and usual idiopathic interstitial pneumonia.

The collagen disease includes dermatomyositis, polymyositis, systemic sclerosis (scleroderma), rheumatoid arthritis and the like.

The anti-CCR4 antibody used in the present invention may be any antibody, so long as it specifically binds to CCR4.

The anti-CCR4 antibody used as a preventive agent or therapeutic agent may be any antibody, so long as it can regulate function of CCR4-positive cells by specifically binding to CCR4 or can exclude CCR4-positive cells from the living body by the effector function of the antibody. Examples include an antibody, an antibody fragment thereof and the like which can deplete CCR4-expressing cells by the antibody-dependent cell-mediated cytotoxic activity (ADCC activity) through their binding to the extracellular region of CCR4.

Also, the anti-CXCR3 antibody used in the present invention may be any antibody, so long as it specifically binds to CXCR3.

The anti-CXCR3 antibody used as a preventive agent or therapeutic agent may be any antibody, so long as it can regulate function of CXCR3-positive cells by specifically binding to CXCR3 or can exclude CXCR3-positive cells from the living body by the effector function of the antibody. Examples include an antibody, an antibody fragment thereof and the like which can injure CXCR3-expressing cells by the antibody-dependent cellular cytotoxic activity (ADCC activity) through their binding to the extracellular region of CXCR3.

The antibody used in the present invention includes any of a polyclonal antibody, a monoclonal antibody, a recombinant antibody, an antibody fragment thereof and the like.

The recombinant antibody includes humanized antibodies such as a human chimeric antibody and a human CDR-grafted antibody. The antibody fragment includes Fab (abbreviation of fragment of antigen binding), F(ab')₂, Fab', a single chain antibody (single chain Fv; hereinafter referred to as "scFv"), a dimerized V region fragment (Diabody), a disulfide-stabilized V region fragment antibody (disulfide stabilized Fv; hereinafter referred to as "dsFv"), a peptide comprising CDR, and the like.

It is preferred that the anti-CXCR3 antibody specifically reacts with the extracellular region of human CXCR3. The anti-CXCR3 antibody can be manufactured by a known method (Harlow E. and Lane D., *Antibodies: A Laboratory Manual*, Cold Spring Harbor Laboratory (1988), hereinafter referred to as *"Antibodies: A Laboratory Manual").* Alternatively, a commercially available anti-CXCR3 antibody (manufactured by Pharmingen) or an antibody disclosed in WO98/11218 can be used.

It is preferred that the anti-CCR4 antibody specifically reacts with the extracellular region of human CCR4. The anti-CCR4 antibody include an antibody which specifically reacts with the region comprising positions 1 to 39, 98 to 112, 176 to 206 or 271 to 284 in the amino acid sequence represented by SEQ ID NO:1, and is preferably an antibody which reacts with positions 2 to 29 (SEQ ID NO:22) in the amino acid sequence represented by SEQ ID NO:1, and positions 12 to 29 (SEQ ID NO:23) in the amino acid sequence represented by SEQ ID NO:1.

The anti-CCR4 antibody can be prepared by a known method (*Antibodies: A Laboratory Manual*).

As the anti-CCR4 antibody, any of a polyclonal antibody and a monoclonal antibody can be used, and the monoclonal antibody is preferably used.

The monoclonal antibody used in the present invention includes an antibody produced by a hybridoma, a humanized antibody and an antibody fragment thereof.

The anti-CCR4 monoclonal antibody produced by a hybridoma can be specifically prepared by the following method.

That is, a cell which expresses the CCR4 protein or a synthetic peptide based on a partial sequence of CCR4 is prepared as an antigen, and a plasma cell having an antigen specificity is induced from an animal immunized by the antigen. Then, the plasma cell is fused with a myeloma cell to prepare a hybridoma, the hybridoma is cultured or the hybridoma cells are administered into the animal to cause ascites tumor in the animal, and an antibody which specifically binds to CCR4 is separated and purified from the culture or ascites. The thus obtained anti-CCR4 monoclonal antibody includes a monoclonal antibody KM2160 produced by a hybridoma KM2160 belonging to the mouse IgG1 subclass disclosed in WO01/64754 (*Int*. *Immunol.,* 11, 81 (1999)).

Furthermore, the anti-CCR4 antibody includes a recombinant antibody such as a humanized antibody, and an antibody fragment thereof.

The humanized antibody used in the present invention includes a recombinant antibody obtained by modifying the above anti-CCR4 monoclonal antibody using genetic recombinant techniques and an antibody fragment thereof. In the antibody, one having low antigenicity and a prolonged half-time in blood is preferred as a preventive agent or a therapeutic agent.

The humanized antibody is preferably a humanized antibody or the antibody fragment thereof which comprises:
complementarity determining region (hereinafter referred to as "CDR") 1, CDR2 and CDR3 of an antibody heavy chain (H chain) variable region (V region) having the amino acid sequences represented by SEQ ID NOs:2, 3 and 4, respectively, and/or
CDR1, CDR2 and CDR3 of an antibody light chain (L chain) V region having the amino acid sequences represented by SEQ ID NOs:5, 6 and 7, respectively (WO01/64754).

Furthermore, the humanized antibody includes a human chimeric antibody, a human CDR-grafted antibody, and an antibody fragment thereof

A human chimeric antibody is an antibody comprising a heavy chain variable region (hereinafter the heavy chain, the variable region and the H chain V region are referred to as "H chain", "V region", and "VH", respectively) and a light chain V region (hereinafter the light chain and the L chain V region are referred to as "L chain" and "VL", respectively) of an antibody derived from a non-human animal, an H chain constant region (hereinafter the constant region and the H chain C region are referred to as "C region" and "CH", respectively) of a human antibody, and an L chain C region (hereinafter also referred to as "CL") of a human antibody. The non-human animal may be any of mouse, rat, hamster, rabbit and the like, so long as a hybridoma can be prepared therefrom.

The human chimeric antibody used in the present invention can be produced by obtaining cDNAs encoding VH and VL from a hybridoma capable of producing an anti-CCR4 antibody, inserting each of the cDNAs into an animal cell expression vector having DNAs encoding CH and CL of a human antibody to construct a human chimeric antibody expression vector, and introducing the vector into an animal cell to express the antibody.

Any CH of a human chimeric antibody may be used, so long as it belongs to human immunoglobulin (hIg), but those of hIgG class are preferred, and any one of subclasses further belonging to hIgG such as γ 1, γ2, γ3 and γ4 can be used. Also, any CL of a human chimeric antibody may be used, so long as it belongs to hIg, and those of κ class or λ class can be used.

The human chimeric antibody which specifically binds to CCR4 (hereinafter, referred to as "anti-CCR4 chimeric antibody") is preferably a human chimeric antibody or the antibody fragment thereof which comprises:
CDR1, CDR2 and CDR3 of VH having the amino acid sequences represented by SEQ ID NOs:2, 3 and 4, respectively, and/or
CDR1, CDR2 and CDR3 of VL having the amino acid sequences represented by SEQ ID NOs:5, 6 and 7, respectively, and
is preferably a human chimeric antibody or the antibody fragment thereof which comprises:
VH comprising the amino acid sequence represented by SEQ ID NO:8, and/or
VL comprising the amino acid sequence represented by SEQ ID NO:9 (WO01/64754).

Specifically, it includes a human chimeric antibody KM2760 and the antibody fragment thereof disclosed in WO01/64754 wherein VH and CH of the antibody comprise the amino acid sequence of SEQ ID NO:8 and the amino acid sequence of human γ1 subclass, respectively, and VL and CL of the antibody comprise the amino acid sequence of SEQ ID NO:9 and the amino acid sequence of human κ class, respectively.

Transformant KM2760 which is capable of producing a human chimeric antibody KM2760 has been internationally deposited as FERM BP-7054 on February 24, 2000, in National Institute of Bioscience and Human Technology, Agency of Industrial Science and Technology, the Ministry of International Trade and Industry (present name: International Patent Organism Depositary, National Institute of Advanced Industrial Science and Technology) (Higashi 1-1-3, Tsukuba-shi, Ibaraki-ken, Japan (present address: Tsukuba Central 6, 1-1, Higashi 1-Chome Tsukuba-shi, Ibaraki-ken, Japan)) (WO01/64754).

The human CDR-grafted antibody is a human antibody in which CDRs of VH and VL of an antibody derived from a non-human animal are respectively substituted by CDR sequences of an antibody derived from a non-human animal.

The human CDR-grafted antibody used in the present invention can be produced by constructing cDNAs encoding V regions in which CDR sequences of VH and VL in a human antibody are substituted by CDR sequences of VH and VL in an antibody derived from a non-human animal, inserting them respectively into an expression vector for animal cell having gene encoding CH of a human antibody and CL of a human antibody to construct a human CDR-grafted antibody expression vector, and then introducing it into an animal cell to express the human CDR-grafted antibody.

Any CH in the human CDR-grafted antibody can be used, so long as it belongs to hIg. Preferably, an hIgG class, and any one of γ1, γ2, γ3 and γ4 subclasses belonging to the hIgG class can be used. Also, any CL in the human CDR-grafted antibody can be used, so long as it belongs to the hIg, and those of κ class or λ class can be used.

The human CDR-grafted antibody which specifically binds to CCR4 (hereinafter referred to as "anti-CCR4 CDR-grafted antibody") is preferably a human CDR-grafted antibody or the antibody fragment thereof which comprises:
CDR1, CDR2 and CDR3 of VH having the amino acid sequences represented by SEQ ID NOs:2, 3 and 4, respectively, and/or
CDR1, CDR2 and CDR3 of VL having the amino acid sequences represented by SEQ ID NOs:5, 6 and 7, respectively,
more preferably a human CDR-grafted antibody or the antibody fragment thereof which comprises:
VH comprising the amino acid sequence of SEQ ID NO:10 or 11, and/or
VL comprising the amino acid sequence of SEQ ID NO:12, and
still more preferably a human CDR-grafted antibody or the antibody fragment thereof which comprises:
VH comprising an amino acid sequence in which at least one amino acid residue selected from Ala at position 40, Gly at position 42, Lys at position 43, Gly at position 44, Lys at position 76 and Ala at position 97 in the amino acid sequence represented by SEQ ID NO:10 is substituted with an other amino acid, and/or
VL comprising an amino acid sequence in which at least one amino acid residue selected from Ile at position 2, Val at position 3, Gln at position 50 and Val at position 88 in the amino acid sequence represented by SEQ ID NO:12 is substituted with an other amino acid, or
a human CDR-grafted antibody or the antibody fragment thereof which comprises:
VH comprising an amino acid sequence in which at least one amino acid residue selected from Thr at position 28 and Ala at position 97 in the amino acid sequence represented by SEQ ID NO:11 is substituted with an other amino acid, and/or
VL of an antibody comprising an amino acid sequence in which at least one amino acid residue selected from Ile at position 2, Val at position 3, Gln at position 50 and Val at position 88 in the amino acid sequence represented by SEQ ID NO:12 is substituted with an other amino acid.

Specifically, it includes a human CDR-grafted antibody or the antibody fragment thereof which comprises:
VH comprising the amino acid sequence selected from SEQ ID NOs:13 to 18, and/or
VL comprising the amino acid sequence selected from SEQ ID NOs:19 to 21.

More specifically, it includes a human CDR-grafted antibody or the antibody fragment thereof which comprises:
VH comprising the amino acid sequence represented by SEQ ID NO:13, and/or
VL comprising the amino acid sequence represented by SEQ ID NO:21; and
a human CDR-grafted antibody or the antibody fragment thereof comprises:
VH of an antibody comprising the amino acid sequence represented by SEQ ID NO:14, and/or
VL of an antibody comprising the amino acid sequence represented by SEQ ID NO:21.

Transformant KM8759 which is capable of producing a human CDR-grafted antibody KM8759 which reacts with CCR4 and comprises VH comprising the amino acid sequence represented by SEQ ID NO:13 and VL comprising the amino acid sequence represented by SEQ ID NO:21 has been internationally deposited as FERM BP-8129 on July 30, 2002, in International Patent Organism Depositary, National Institute of Advanced Industrial Science and Technology (Tsukuba Central 6, 1-1, Higashi 1-Chome Tsukuba-shi, Ibaraki-ken, Japan).

Transformant KM8760 which is capable of producing a human CDR-grafted antibody KM8760 which reacts with CCR4 and comprises VH comprising the amino acid sequence represented by SEQ ID NO:14 and VL comprising the amino acid sequence represented by SEQ ID NO:21 has been internationally deposited as FERM BP-8130 on July 30, 2002, in International Patent Organism Depositary, National Institute of Advanced Industrial Science and Technology (Tsukuba Central 6, 1-1, Higashi 1-Chome Tsukuba-shi, Ibaraki-ken, Japan).

The anti-CCR4 antibody used in the present invention includes an antibody which comprises the amino acid sequence in which one or more amino acid residues are deleted, substituted, inserted or added in the amino acid sequence defining the anti-CCR4 antibody and specifically react with CCR4, and an antibody fragments thereof.

In the present invention, one or more amino acid deletion, substitution, insertion or addition in the amino acid sequence means that one or more amino acids are deleted, substituted, inserted or added to at one or plural positions in the amino acid sequence. The deletion, substitution, insertion or addition can be caused in the same amino acid sequence simultaneously. Also, the amino acid residue substituted, inserted or added can be natural or non-natural. The natural amino acid residue includes L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, L-cysteine and the like.

Thereinafter, preferred examples of amino acid residues which are substituted with each other are shown. The amino acid residues in the same group can be substituted with each other.
Group A:
leucine, isoleucine, norleucine, valine, norvaline, alanine, 2-aminobutanoic acid, methionine, O-methylserine, t-butylglycine, t-butylalanine, cyclohexylalanine;
Group B:
aspartic acid, glutamic acid, isoaspartic acid, isoglutamic acid, 2-aminoadipic acid, 2-aminosuberic acid;
Group C:
asparagine, glutamine;
Group D:
lysine, arginine, ornithine, 2,4-diaminobutanoic acid, 2,3-diaminopropionic acid;
Group E:
proline, 3-hydroxyproline, 4-hydroxyproline;
Group F:
serine, threonine, homoserine;
Group G:
phenylalanine, tyrosine.

The anti-CCR4 antibody used in the present invention includes an antibody fragment. The antibody fragment includes antibody fragments which specifically bind to CCR4, such as Fab, F(ab')₂, Fab', scFv, Diabody, dsFv, and a peptide comprising CDR.

An Fab is an antibody fragment having a molecular weight of about 50,000 and antigen binding activity, in which about a half of the N-terminal side of H chain and the entire L chain, among fragments obtained by treating IgG with a protease, papain (cut at an amino acid residue at position 224 of the H chain), are bound together through a disulfide bond (S-S bond).

The Fab of the present invention can be obtained by treating the anti-CCR4 antibody with a protease, papain. Also, the Fab can be produced by inserting DNA encoding Fab of the antibody into an expression vector for prokaryote or an expression vector for eukaryote, and introducing the vector into a prokaryote or eukaryote to express the Fab.

An F(ab')₂ is an antibody fragment having a molecular weight of about 100,000 and antigen binding activity, which is slightly larger than the Fab bound via a S-S bond of the hinge region, among fragments obtained by treating IgG with a protease, pepsin.

The F(ab')₂ of the present invention can be obtained by treating the anti-CCR4 antibody with a protease, pepsin. Also, the F(ab')₂ can be produced by binding Fab' described below via a thioether bond or a S-S bond.

An Fab' is an antibody fragment having a molecular weight of about 50,000 and antigen binding activity, which is obtained by cutting a disulfide bond of the hinge region of the above F(ab')₂.

The Fab' of the present invention can be obtained by treating the above F(ab')₂ with a reducing agent, dithiothreitol. Also, the Fab' of the present invention can be produced by inserting DNA encoding an Fab' of the anti-CCR4 antibody into an expression vector for prokaryote or an expression vector for eukaryote, and introducing the vector into a prokaryote or eukaryote to express the Fab'.

An scFv is a VH-P-VL or VL-P-VH polypeptide in which one chain VH and one chain VL are linked by using an appropriate peptide linker (P) of 12 or more residues and which has an antigen-binding activity.

The scFv of the present invention can be produced by obtaining cDNAs encoding VH and VL of the anti-CCR4 antibody, constructing DNA encoding scFv, inserting the DNA into an expression vector for prokaryote or an expression vector for eukaryote, and then introducing the expression vector into a prokaryote or eukaryote to express the scFv.

A diabody is an antibody fragment in which scFv's having the same or different antigen binding specificity forms a dimer, and has an divalent antigen binding activity to the same antigen or two specific antigen binding activity to different antigens.

The diabody of the present invention, for example, a divalent diabody which specifically reacts with CCR4, can be produced by obtaining cDNAs encoding VH and VL of the anti-CCR4 antibody, constructing DNA encoding scFv having a polypeptide linker of 3 to 10 residues, inserting the DNA into an expression vector for prokaryote or an expression vector for eukaryote, and then introducing the expression vector into a prokaryote or eukaryote to express the diabody.

A dsFV is an antibody fragment which is obtained by binding polypeptides in which one amino acid residue of each of VH and VL is substituted with a cysteine residue and those cysteine residues are bound via a disulfide bond between the cysteine residues. The amino acid residue which is substituted with a cysteine residue can be selected based on a three-dimensional structure estimation of the antibody in accordance with the method shown by Reiter *et al*. (*Protein Engineering,* 7, 697 (1994)).

The dsFv of the present invention can be produced by obtaining cDNAs encoding VH and VL of the CCR4-antibody antibody, constructing DNA encoding dsFv, inserting the DNA into an expression vector for prokaryote or an expression vector for eukaryote, and then introducing the expression vector into a prokaryote or eukaryote to express the dsFv.

A peptide comprising CDR is an antibody fragment comprising at least one region of CDRs of VH and VL. The peptide comprising plural CDRs can be produced by binding directly to or via an appropriate peptide linker.

The peptide comprising CDR of the present invention can be produced by obtaining cDNA encoding CDR of VH and VL of the CCR4-antibody antibody, inserting the DNA into an expression vector for prokaryote or an expression vector for eukaryote, and then by introducing the expression vector into a prokaryote or eukaryote to express the peptide. Also, the peptide comprising CDR can also be produced by a chemical synthesis method such as an Fmoc method (fluorenylmethoxycarbonyl method), a tBoc method (t-butyloxycarbonyl method), or the like.

The anti-CCR4 antibody of the present invention includes derivatives of an antibody in which a radioisotope, a protein, an agent or the like is chemically or genetically bound to the anti-CCR4 antibody of the present invention.

The derivatives of the anti CCR4-antibody of the present invention can be produced by chemically conjugating a radioisotope, a protein, agent or the like to the N-terminal side or C-terminal side of an H chain or an L chain of the anti-CCR4 antibody or the antibody fragment thereof to an appropriate substituent group or side chain of the antibody or antibody fragment or to a sugar chain in the antibody or antibody fragment (*Antibody Engineering Handbook,* edited by Osamu Kanemitsu, published by Chijin Shokan (1994)).

Also, it can be genetically produced by linking a DNA encoding the anti-CCR4 antibody or the antibody fragment thereof to other DNA encoding a protein to be bound, inserting the DNA into an expression vector, and introducing the expression vector into a host cell.

The radioisotope includes ¹³¹I, ¹²⁵I and the like, and it can be conjugated to the antibody by, e.g., a chloramine T method.

The agent is preferably a low molecular weight compound. Examples include anticancer agents such as alkylating agents (e.g., nitrogen mustard, cyclophosphamide), metabolic antagonists (e.g., 5-fluorouracil, methotrexate), antibiotics (e.g., daunomycin, bleomycin, mitomycin C, daunorubicin, doxorubicin), plant alkaloids (e.g., vincristine, vinblastine, vindesine), hormone drugs (e.g., tamoxifen, dexamethasone), and the like (*Clinical Oncology*, edited by Japanese Society of Clinical Oncology, published by Cancer and Chemotherapy (1996)); anti-inflammatory agents such as steroid agents (e.g., hydrocortisone, prednisone), non-steroidal drugs (e.g., aspirin, indometacin), immunomodulators (e.g., aurothiomalate, penicillamine), immunosuppressing agents (e.g., cyclophosphamide, azathioprine) and antihistaminic agents (e.g., chlorpheniramine maleate, clemastine) (*Inflammation and Antiinflammatory Therapy,* Ishiyaku Shuppan (1982)); and the like. The method for conjugating daunomycin to an antibody includes a method in which daunomycin and an amino group of an antibody are conjugated via glutaraldehyde, a method in which an amino group of daunomycin and a carboxyl group of an antibody are conjugated via a water-soluble carbodiimide, and the like.

The protein is preferably cytokine which activates immune cells. Examples include human interleukin 2 (hereinafter referred to as "hIL-2"), human granulocyte macrophage colony-stimulating factor (hereinafter referred to as "hGM-CSF"), human macrophage colony-stimulating factor (hereinafter referred to as "hM-CSF"), human interleukin 12 (hereinafter referred to as "hIL-12"), and the like. Also, in order to inhibit cancer cells directly, a toxin such as ricin, diphtheria toxin and the like, can be used. For example, a fusion antibody with a protein can be produced by linking a cDNA encoding an antibody or antibody fragment to other cDNA encoding the protein, constructing DNA encoding the fusion antibody, inserting the DNA into an expression vector for prokaryote or an expression vector for eukaryote, and then introducing it into a prokaryote or eukaryote to express the fusion antibody.

Specific production methods and activity evaluation methods of the anti-CCR4 antibody used in the present invention, a preventive agent, a diagnostic agent or a therapeutic agent for interstitial pneumonia comprising the anti-CCR4 antibody, and a discrimination method of idiopathic interstitial pneumonia are explained below.

### 1. Preparation method of anti-CCR4 antibody (polyclonal antibody, monoclonal antibody)

### (1) Preparation of antigen

The antigen necessary for preparing an anti-CCR4 antibody includes a cell which expresses CCR4 or a cell fraction thereof, CCR4, a partial fragment of CCR4, a peptide having a partial sequence of the amino acid sequence of CCR4, and the like.

The CCR4 and the partial fragment of CCR4 can be produced intracellularly or on the surface of the cell as such or as fusion proteins, by constructing a recombinant vector in which a complete cDNA encoding CCR4 or a partial fragment thereof (*J. Biol*. *Chem*., 270, 19495 (1995)) is inserted into the promoter downstream of an appropriate vector, introducing this into a host cell, and then culturing the thus obtained CCR4-expressing cell in an appropriate medium. In addition, the peptide having a partial sequence of CCR4 can be prepared by using an amino acid synthesizer.

The complete cDNA encoding CCR4 or a partial fragment thereof can be prepared by a polymerase chain reaction (hereinafter referred to as "PCR"; Sambrook J. *et al*., *Molecular Cloning,* 3rd edition, Cold Spring Harbor Laboratory (2001) (hereinafter referred to as *"Molecular Cloning,* 3rd edition"), Ausubel F.M. *et al*., *Current Protocols in Molecular Biology,* John Wiley & Sons (1987-2001) (hereinafter referred to as "*Current Protocols in Molecular Biology*")) by using a cDNA prepared from a CCR4-expressing cell in human peripheral blood or the like as the template.

Any host can be used, so long as it can express the gene of interest, such as bacteria, yeast, animal cells and insect cells. The bacteria include bacteria belonging to the genus *Escherichia*, the genus *Bacillus* and the like such as *Escherichia coli* and *Bacillus subtilis.* The yeast includes *Saccharomyces cerevisiae, Schizosaccharomyces pombe* and the like. The animal cells include human cell Namalwa cell, monkey cell COS cell, Chinese hamster cell CHO cell and the like. The insect cells include Sf9, Sf21 (manufactured by Pharmingen), High Five (manufactured by Invitrogen) and the like.

As the vector to be introduced with the complete cDNA encoding CCR4 or a partial fragment thereof, any vector can be used, so long as the DNA can be inserted therein and be expressed in a host cell.

When bacteria such as *Escherichia coli* are used as the host, the expression vector preferably comprises a promoter, a ribosome binding sequence, a complete cDNA encoding CCR4 or a partial fragment thereof, a transcription termination sequence and, as occasion demands, a promoter regulatory sequence, and examples include commercially available pGEX-2T (manufactured by Amersham Biosciences), pET17b (manufactured by Novagen) and the like.

As the method for introducing a recombinant vector into a bacterium, any method can be used, so long as it is a method in which DNA can be introduced into bacteria, and examples include a method which uses a calcium ion (Cohen S.N. *et al*., *Proc. Natl*. *Acad. Sci. USA*, 69, 2110-2114 (1972)), a protoplast method (Japanese Published Unexamined Patent Application No. 248394/88) and the like.

When yeast are used as a host cell, the expression vector includes Yep13 (ATCC 37115), Yep24 (ATCC 37051), YCp50 (ATCC 37419) and the like.

As the method for introducing a recombinant vector into a yeast, any method can be used, so long as it is a method in which DNA can be introduce into yeast, and examples include electroporation (Becker D.M. and Guarente L., *Methods*. *Enzymol*., 194, 182-187 (1991)), a spheroplast method (Hinnen A. *et al*., *Proc. Natl*. *Acad. Sci*. *USA*, 84, 1929-1933 (1978)), a lithium acetate method (Ito H. *et al*., *J*. *Bacteriol.,* 153, 163-168 (1983)) and the like.

When animal cells are used as the host, the expression vector includes pAGE107 (Japanese Published Unexamined Patent Application No. 22979/91; Miyaji H. *et al*., *Cytotechnology,* 3, 133-140 (1990)), pAGE103 (Mizukami T. and Itoh S., *J*. *Biochem.,* 101, 1307-1310 (1987)) and the like.

Any promoter can be used, so long as it can be expressed in animal cells, and examples include the promoter of cytomegalovirus (CMV) IE (immediate early) gene, the promoter of SV40 or metalothionein and the like. In addition, the enhancer of human CMV IE gene can be used together with the promoter.

As the method for introducing a recombinant vector into an animal cell, any method can be used, so long as it is a method for introducing DNA into an animal cell, and examples includes electroporation (Miyaji H. *et al*., *Cytotechnology,* 3, 133-140 (1990)), a calcium phosphate method (Japanese Published Unexamined Patent Application No. 227075/90), a lipofection method (Felgner P.L. *et al*., *Proc. Natl. Acad*. *Sic. USA*, 84, 7413-7417 (1987)) and the like.

When insect cells are used as the host, a protein can be expressed, for example, by the method described in *Current Protocols in Molecular Biology,* O'Reilly *et al*., *Baculovirus Expression Vectors: A Laboratory Manual,* Oxford University Press (1994) or the like. That is, a protein expressing insect cell is obtained by preparing a recombinant virus in an insect cell culture supernatant through co-transfer of the recombinant gene transfer vector and baculovirus described below into an insect cell, and further infecting the insect cell with the recombinant virus.

The gene transfer vector includes pVL1392, pVL1393 (both manufactured by Pharmingen), pBlueBac4.5 (manufactured by Invitrogen) and the like.

The baculovirus includes a virus which infects *Barathra* insects, *Autographa californica* nuclear polyhedrosis virus and the like.

The method for co-transferring the above gene transfer vector and the above baculovirus into an insect cell for the preparation of a recombinant virus includes the calcium phosphate method (Japanese Published Unexamined Patent Application No. 227075/90), the lipofection method (Felgner P.L. *et al*., *Proc. Natl*. *Acad*. *Sic. USA,* 84, 7413-7417 (1987)) and the like.

Also, CCR4 can be produced by preparing a recombinant baculovirus using BaculoGold Starter Kit manufactured by Pharmingen or the like, and then infecting an insect cell such as the above Sf9, Sf21 or High Five with the recombinant virus (*Bio*/*Technology*, 6, 47 (1988)).

A full length CCR4 or a partial fragment thereof can be produced as such or as a fusion protein, by culturing the transformant obtained in the above in a medium and recovering CCR4 from the culture.

The method for culturing a transformant in a medium is carried out in accordance with a conventional method used in the culturing of hosts.

The medium used in the culturing of a transformant obtained by using a microorganism such as *Escherichia coli* or yeast as the host may be any of a natural medium or a synthetic medium, so long as it contains a carbon source, a nitrogen source, inorganic salts and the like which can be assimilated by the microorganism and can efficiently culture the transformant (*Molecular Cloning*, 3rd edition). The culturing is carried out at 15 to 40°C for 16 to 96 hours generally under aerobic conditions such as shaking culture or submerged aeration agitation culturing. During the culturing, pH is maintained at 3.0 to 9.0. The pH is adjusted by using an inorganic or organic acid, an alkali solution, urea, calcium carbonate, ammonia or the like. If necessary, antibiotics such as ampicillin and tetracycline can be added to the medium during the culturing.

The medium for culturing a transformant obtained by using animal cells as the host includes generally used RPMI 1640 medium, Eagle's MEM medium, media prepared by adding fetal bovine serum to these media and the like. The culturing is carried out at 35 to 37°C for 3 to 7 days generally in the presence of 5% CO₂, and antibiotics such as kanamycin and penicillin can be added to the medium during the culturing, if necessary.

The medium for culturing a transformant obtained by using insect cells as the host includes generally used TNM-FH medium (manufactured by Pharmingen), Sf900IISFM (manufactured by Invitrogen), Excell400, Excell405 (both manufactured by JRH Biosciences) and the like. The culturing is carried out at 25 to 30°C for 1 to 4 days. If necessary, antibiotics such as gentamicin can be added to the medium during the culturing.

In the above culturing of an animal cell or insect cell, if possible, a serum-free medium is preferably used in order to facilitate purification of a partial fragment of CCR4 as such or as a fusion protein thereof.

When a full length CCR4 or a partial fragment thereof is accumulated in the host cell as such or as a fusion protein thereof, the cells after completion of the culturing are centrifuged and suspended in an aqueous buffer, and the resulting cells are disrupted by an ultrasonication method, a French press method or the like to recover the protein in the centrifugation supernatant.

In addition, when an insoluble body is formed inside the cell, three-dimensional structure of its protein can be formed by solubilizing the insoluble body with a protein denaturing agent and diluting with or dialyzing against a solution which does not contain the protein denaturing agent or contains the protein denaturing agent but in such a thin concentration that the protein is not denatured.

When the CCR4 or a partial fragment thereof or a fusion protein thereof is secreted extracellularly, the expressed protein can be recovered in the culture supernatant.

Isolation and purification can be carried out by using isolation operations such as solvent extraction, fractional precipitation with an organic solvent, salting out, dialysis, centrifugation, ultrafiltration, ion exchange chromatography, gel filtration chromatography, hydrophobic chromatography, affinity chromatography, reverse phase chromatography, crystallization and electrophoresis, alone or in combination.

The peptide having a partial sequence of the amino acid sequence of CCR4 can be produced by a chemical synthesis method such as an Fmoc method (fluorenylmethoxycarbonyl method) or a tBoc method (t-butoxycarbonyl method). It can also be produced by a peptide synthesizer manufactured by Advanced ChemTech, Applied Biosystems, Protein Technologies, Shimadzu Corp. or the like.

### (2) Immunization of animal and preparation of antibody-producing cell

An animal is immunized with the protein obtained in the above as the antigen. As the immunization method, the antigen may be directly administered to an animal subcutaneously, intravenously or intraperitoneally, but it is preferred to administer the antigen which is bound to a carrier protein having high antigenicity, or administer the antigen together with an appropriate adjuvant.

The carrier protein includes keyhole limpet hemocyanin, bovine serum albumin, bovine thyroglobulin and the like, and the adjuvant includes complete Freund's adjuvant, aluminum hydroxide gel with pertussis vaccine and the like.

The animal to be immunized includes non-human animals such as rabbit, goat, mouse, rat and hamster.

The antigen is administered 3 to 10 times at an interval of 1 to 2 weeks after the first administration. Dose of the antigen is preferably 50 to 100 µg per animal. After each administration, blood is taken from the immunized animal from the venous plexus of the fundus of the eye or from a caudal vein, and specific affinity of the serum for the antigen is confirmed by enzyme-linked immunosorbent assay shown below (ELISA; *Enzyme-Linked Immunosorbent Assay,* 3rd edition, published by Igaku Shoin (1987); *Antibodies: A Laboratory Manual,* Chapter 14, Goding J.W., *Monoclonal Antibodies: Principles and Practice,* Academic Press (1996)) and the like.

The enzyme-linked immunosorbent assay can be carried out as follows.

An antigen protein or cells expressing the antigen protein are coated on a plate and allowed to react with a serum collected from the immunized animal as a primary antibody. After the reaction with the primary antibody, the plate is washed and a secondary antibody is added thereto. After the reaction, the antibody titer is measured by carrying out a detection reaction of the secondary antibody corresponding to the labeled substance.

The secondary antibody is an antibody which can recognize the primary antibody further labeled with an enzyme such as peroxidase or a substance such as biotin. Specifically, when a mouse is used as the animal to be immunized, an antibody capable of recognizing mouse immunoglobulin is used as the secondary antibody.

Thereafter, a non-human mammal of which serum shows a sufficient antibody titer is used as the supply source of antibody-producing cells.

Three to 7 days after the final administration of the antigen, lymphocytes are excised from the immunized animal in accordance with a known method (*Antibodies: A Laboratory Manual*), and the lymphocytes are fused with myeloma cells.

A polyclonal antibody can be prepared by separating and purifying the serum.

A monoclonal antibody can be prepared by fusing the antibody-producing cell with a non-human mammal-derived myeloma cell to prepare a hybridoma, culturing the hybridoma or administering it to an animal to cause ascites tumor of the cell, and separating and purifying the culture medium or ascitic fluid.

The antibody-producing cell is collected from the spleen, lymph node, peripheral blood or the like of the antigen-administered non-human mammal.

### (3) Preparation of myeloma cell

As the myeloma cell, any myeloma cell can be used, so long as it can proliferate *in vitro*, and examples include the 8-azaguanine-resistant mouse (BALB/c-derived) myeloma cell lines P3-X63Ag8-U1 (Kohler G. and Milstein C., *Eur*. *J. Immunol*., 6, 511-519 (1976)), SP2/0-Ag14 (Shulman M, *et al*., *Nature*, 276, 269-270 (1978)), P3-X63-Ag8653 (Kearney J.F. *et al*., *J*. *Immunol*., 123, 1548-1550 (1979)), P3-X63-Ag8 (Kohler G. and Milstein C., *Nature,* 256, 495-497 (1975)) and the like. The culturing and sub-culturing of these cell lines can be carried out to according to a known method (*Antibodies: A Laboratory Manual*) to thereby obtain a cell number of 2×10⁷ cells or more until the time of cell fusion.

### (4) Cell fusion and selection of monoclonal antibody

The antibody-producing cell and myeloma cell obtained in the above are washed, fused by adding cell-aggregating medium such as polyethylene glycol-1000 (PEG-1000) and then suspended in a medium. MEM medium, PBS (disodium hydrogenphosphate 1.83 g, potassium dihydrogenphosphate 0.21 g, sodium chloride 7.65 g, distilled water 1 liter, pH 7.2) or the like is used for washing the cells. In addition, in order to selectively obtain the fused cells of interest alone, HAT medium [prepared by adding 10⁻⁴ mol/l hypoxanthine, 1.5×10⁻⁵ mol/l thymidine and 4×10⁻⁷ mol/l aminopterin to the normal medium (RPMI 1640 medium supplemented with 1.5 mmol/l glutamine, 5×10⁻⁵ mol/l 2-mercaptoethanol, 10 g/ml gentamicin and 10% fetal bovine serum)] is used as the medium for suspending the fused cells.

After the culturing, a part of the culture supernatants is subjected to the following enzyme-linked immunosorbent assay to select samples which react with the antigen protein but do not react with non-antigen proteins. Subsequently, their cloning is carried out by limiting dilution analysis, and a sample which shows a stable and high antibody titer by enzyme-linked immunosorbent assay is selected as a hybridoma strain capable of producing a monoclonal antibody which specifically binds to CCR4.

The enzyme-linked immunosorbent assay is carried out in the same manner as described in the item 1(2), except that a hybridoma culture supernatant or a purified antibody obtained by a method which is described later is used as the primary antibody.

Specific binding between the monoclonal antibody and CCR4 can also be evaluated by the surface plasmon resolution (Karlsson R. *et al*., *J*. *Immunol*. *Methods*, 145, 229-240 (1991)).

The anti-CCR4 monoclonal antibody includes a monoclonal antibody KM 2160 produced by the above hybridoma KM 2160.

### (5) Preparation of monoclonal antibody

The monoclonal antibody can be prepared by separating and purifying it from a culture medium obtained by culturing a hybridoma cell or from an ascitic fluid obtained by inducing ascites through the intraperitoneal administration of a monoclonal antibody producing hybridoma cells to 8 to 10-week-old mice or nude mice treated with pristane [intraperitoneal administration of 0.5 ml of pristane (2,6,10,14-tetramethylpentadecane), followed by feeding for 2 weeks].

The method for separating and purifying the monoclonal antibody includes centrifugation, salting out with 40 to 50% saturation ammonium sulfate, a caprylic acid precipitation method, chromatography which uses a DEAE-Sepharose column, an anion exchange column, a protein A or G-column or a gel filtration column and the like, which may be used alone or in combination. According to this method, an IgG or IgM fraction is recovered to give a purified monoclonal antibody.

The subclass of the purified monoclonal antibody can be determined by using a monoclonal antibody typing kit or the like. The amount of the protein can be calculated by the Lowry method or from the absorbance at 280 nm.

The subclass of antibody means an isotype in the class, and includes IgG1, IgG2a, IgG2b and IgG3 in the case of mouse, and IgG1, IgG2, IgG3 and IgG4 in the case of human. Particularly, mouse IgG1 and IgG2a and human IgG1 types are useful in applying to medical treatments since they have complement-dependent cytotoxic activity (hereinafter referred to as "CDC activity") and ADCC activity.

Specifically, the anti-CCR4 antibody can be prepared according to the method described in WO01/64754.

### 2. Preparation method of anti-CCR4 humanized antibody

### (1) Construction of vector for expression of humanized antibody

A vector for expression of humanized antibody is constructed to prepare a humanized antibody from an antibody derived from a non-human animal. The vector for expression of humanized antibody is an expression vector for animal cell into which genes encoding CH and CL of C region of a human antibody have been inserted, and is constructed by cloning each of CH and CL of a human antibody into an expression vector for animal cell.

The C region of a human antibody can be CH and CL of any human antibody. Examples include CH of γ1 subclass, CH of γ4 subclass and CL of κ class of a human antibody, and the like. As the DNA encoding CH and CL of a human antibody, a chromosome DNA which comprises exon and intron can be used. Also, cDNA can be used. As the expression vector for animal cell, any expression vector can be used, so long as a C region of a human antibody can be inserted and expressed.

Examples include pAGE107 (Japanese Published Unexamined Patent Application No. 22979/91; Miyaji H. *et al*., *Cytotechnology,* 3, 133-140 (1990)), pAGE103 (M. Mizukami T. and Itoh S., *J. Biochem.,* 101, 1307-1310 (1987)), pHSG274 (Brady G. *et al*., *Gene*, 27, 223-232 (1984)), pKCR (O'Hare K. *et al*., *Proc*. *Natl*. *Acad*. *Sci. USA*, 78, 1527-1531 (1981)), pSGIβd2-4 (Miyaji H. *et al*., *Cytotechnology,* 4, 173-180 (1990)) and the like. A promoter and enhancer used for an expression vector for animal cell includes an SV40 early promoter and enhancer (Mizukami T. and Itoh S., *J*. *Biochem.,* 101, 1307-1310 (1987)), a Moloney mouse leukemia virus LTR promoter and enhancer (Kuwana Y. *et al*., *Biochem. Biophys. Res. Comun*., 149, 960-968 (1987)), an immunoglobulin H chain promoter (Mason J.O. *et al*., *Cell,* 41, 479-487 (1985)) and enhancer (Gillies S. D. *et al*., *Cell,* 33, 717-728 (1983)), and the like.

The vector for expression of the humanized antibody can be either of a type in which a gene encoding an antibody H chain and a gene encoding an antibody L chain exist on separate vectors or of a type in which both genes exist on the same vector (tandem type). In respect of easiness of construction of a vector for expression of humanized antibody, easiness of introduction into animal cells, and balance between the expression amounts of antibody H and L chains in animal cells, a tandem type of the vector for expression of humanized antibody is more preferred (Shitara *K. et al*., *J*. *Immunol*. *Methods*, 167, 271-278 (1994)). The tandem type of the vector for expression of humanized antibody includes pKANTEX93 (WO97/10354), pEE18 (Bentely K.J. *et al*., Hybridoma, 17, 559-567 (1998)) and the like.

The constructed vector for expression of humanized antibody can be used for expression of a human chimeric antibody or a human CDR-grafted antibody in animal cells.

### (2) Obtaining cDNA encoding VH and VL of anti-CCR4 antibody derived from non-human animal

The cDNA encoding VH and VL of an anti-CCR4 antibody derived from a non-human animal, e.g., a mouse anti-CCR4 monoclonal antibody, is prepared in the following manner.

cDNAs are synthesized by extracting mRNA from a cell capable of producing a mouse anti-CCR4 monoclonal antibody, e.g., a mouse anti-CCR4 antibody producing hybridoma or the like. A cDNA library is prepared by inserting the thus synthesized cDNAs into a vector such as a phage or a plasmid. Using the C region or V region of a mouse antibody as a probe, a recombinant phage or recombinant plasmid having a cDNA encoding VH and a recombinant phage or recombinant plasmid having a cDNA encoding VL are respectively isolated from the library. By determining complete VH and VL nucleotide sequences on the recombinant phage or recombinant plasmid, full amino acid sequences of VH and VL are deduced from the nucleotide sequences.

As the non-human animal, any one of mouse, rat, hamster, rabbit and the like can be used, so long as a hybridoma can be prepared therefrom.

The method for preparing total RNA from a hybridoma includes the guanidine thiocyanate-cesium trifluoroacetate method (Okayama H. *et al*., *Methods Enzymol*., 154, 3-28 (1987)), and the method for preparing mRNA from the total RNA includes the oligo(dT) immobilized cellulose column method (*Molecular Cloning,* 3rd edition). In addition, the kit for preparing mRNA from a hybridoma includes FastTrack mRNA isolation kit (manufactured by Invitrogen), QuickPrep mRNA isolation kit (manufactured by Amersham Biosciences) and the like.

The methods for synthesizing a cDNA and preparing a cDNA library includes conventional methods (*Molecular Cloning,* 3rd edition; *Current Protocols in Molecular Biology*) or a method which uses a commercially available kit such as SuperScript Choice System for cDNA Synthesis (manufactured by Invitrogen), Zap-cDNA synthesis kit (manufactured by Stratagene) or TimeSaver cDNA synthesis kit (manufactured by Amersham Biosciences).

As the vector into which a cDNA synthesized by using a mRNA extracted from a hybridoma as the template is integrated in preparing a cDNA library, any vector can be used, so long as the cDNA can be introduced therein. Examples include phage or plasmid vectors such as ZAP Express (manufactured by Stratagene), pBluescript II SK (+) (manufactured by Stratagene), λZAPII (manufactured by Stratagene), λgt10 (manufactured by Stratagene), λgt11 (manufactured by Stratagene), Lambda BlueMid (manufactured by Clontech), λExCell (manufactured by Amersham Biosciences), pcD2 (Okayama H. and Berg P., *Mol*. *Cell. Biol*., 3, 280-289 (1983)) and pUC18 (Yanisch-Perron C. *et al*., *Gene*, 33, 103-119 (1985)).

As the *Escherichia coli* into which a cDNA library constructed by a phage or plasmid vector is introduced, any *Escherichia coli* can be used, so long as the cDNA library is introduced, expressed and kept. Examples include XL1-Blue MRF' (manufactured by Stratagene), C600 (Appleyard R.K., *Genetics,* 39, 440-452 (1954)), Y1088 (Young R.A. and Davis R., *Science,* 222, 778-782 (1983)), Y1090 (Young R.A. and Davis R., *Science,* 222, 778-782 (1983)), NM522 (Gough J.A. and Murray N.E., *J*. *Mol*. *Biol*., 166, 1-19 (1983)), K802 (Wood W.B., J. Mol. Biol., 16, 118-133 (1966)), JM105 (Yanisch-Perron C. *et al*., *Gene*, 33, 103-119 (1985)) and the like.

As the method for selecting a cDNA clone encoding VH and VL of an anti-CCR4 antibody derived from a non-human animal from a cDNA library, it can be selected by a colony hybridization method or plaque hybridization method (*Molecular Cloning,* 3rd edition) which uses an isotope- or fluorescence-labeled probe. In addition, a cDNA encoding VH and VL can also be prepared by PCR by preparing primers and using a cDNA synthesized from mRNA or a cDNA library as the template.

Nucleotide sequence of the cDNA selected by the above method can be determined by carrying out a reaction based on the dideoxy method (Sanger F. *et al*., *Proc. Natl. Acad*. *Sci. USA*, 74, 5463-5467 (1977)) using the cDNA cloned into an appropriate vector, and analyzing the product by using a nucleotide sequencer such as ABI377 (manufactured by Applied Biosystems).

### (3) Analysis of amino acid sequences of VH and VL of anti-CCR4 antibody derived from non-human animal and identification of amino acid sequence of CDR

By deducing full amino acid sequences of VH and VL encoded by the cDNA obtained in the item 2(2) from the nucleotide sequences of the cDNA determined therein, and comparing the results with total amino acid sequences of the VH and VL of already known antibodies (*Sequences of Proteins of Immunological Interest,* US Dept. Health and Human Services (1991), hereinafter referred to as "*Sequences of Proteins of* *Immunological Interest*"), whether the obtained cDNA is encoding full amino acid sequences of VH and VL of the antibody including a secretion signal sequence can be verified. Length of the secretion signal sequence and the N-terminal amino acid sequences can be deduced and the subgroup to which they belongs can also be known, by comparing the full amino acid sequences of VH and VL of the antibody including a secretion signal sequence with total amino acid sequences of the VH and VL of already known antibodies (*Sequences of Proteins of Immunological Interest*).

In addition, novelty of the sequences can be evaluated by carrying out homology retrieval of the thus obtained full amino acid sequences of the obtained VH and VL for any data base such as SWISS-PROT or PIR-Protein by using program for a homology retrieving program such as BLAST (Altschul S.F. *et al*., *J*. *Mol. Biol*., 215, 403-410 (1990)).

The VH and VL which form the antigen binding region of antibody comprise 4 framework regions (hereinafter referred to as "FRs") having relatively preserved sequences and 3 CDRs (CDR1, CDR2 and CDR3) having various sequence which connect them alternately (*Sequences of Proteins of Immunological Interest*). The amino acid sequence of each CDR of the VH and VL can be identified by comparing it with the amino acid sequences of the V regions of already known antibodies (*Sequences of Proteins of Immunological Interest*).

### (4) Construction of anti-CCR4 chimeric antibody expression vector

An anti-CCR4 chimeric antibody expression vector can be constructed by inserting a cDNA encoding the VH and VL of anti-CCR4 antibody derived from a non-human animal into upstream of a gene encoding human antibody CH and CL of the vector for expression of humanized antibody which is constructed in the item 2(1). For example, a plasmid having a DNA encoding the amino acid sequences of VH and VL of an anti-CCR4 antibody is obtained by amplifying the VH and VL of the antibody by PCR using a plasmid having a cDNA encoding the VH and VL of anti-CCR4 antibody derived from a non-human animal as the template and using 5'-terminal side and 3'-terminal side primers comprising nucleotide sequences encoding appropriate restriction enzyme recognizing sequences and V regions, and cloning respective amplified products in a plasmid vector such as pBluescript II SK (-) (manufactured by Stratagene) and determining their nucleotide sequences by the method described in the item 2(2). An anti-CCR4 chimeric antibody expression vector can be constructed by isolating the cDNA encoding the amino acid sequences of VH and VL of anti-CCR4 antibody from the thus obtained plasmid and cloning it into upstream of a gene encoding human antibody CH and CL of the vector for humanized antibody expression use constructed in the item 2 (1), in such a manner that they are expressed in an appropriate form.

### (5) Construction of cDNA encoding V region of anti-CCR4 CDR-grafted antibody

cDNAs encoding VH and VL of an anti-CCR4 CDR-grafted antibody can be obtained as follows. First, amino acid sequences of FRs in VH and VL of a human antibody to which amino acid sequences of CDRs in VH and VL of an anti-CCR4 antibody derived from a non-human animal antibody are grafted are selected. Any amino acid sequences of FRs in VH and VL of a human antibody can be used, so long as they are derived from human antibody. Examples include amino acid sequences of FRs in VH and VL of human antibodies registered in database such as Protein Data Bank, and amino acid sequences common to subgroups of FRs in VH and VL of human antibodies (*Sequences of Proteins of Immunological Interest*) and the like. In order to produce a human CDR-grafted antibody having potent activity, amino acid sequences having high homology, preferably homology of 60% or more, with amino acid sequence of FRs in VH and VL of a target antibody derived from a non-human animal is preferably selected.

Then, amino acid sequences of CDRs in VH and VL of the antibody derived from a non-human animal are grafted to the selected amino acid sequences of FRs in VH and VL of a human antibody to design amino acid sequences of VH and VL of an anti-CCR4-CDR -grafted antibody. The designed amino acid sequences are converted to nucleotide sequences by considering the frequency of codon usage found in nucleotide sequences of genes of antibodies (*Sequence of Proteins of Immunological Interest*), and the nucleotide sequences encoding the amino acid sequences of VH and VL of an anti-CCR4 CDR-grafted antibody are designed. Several synthetic DNAs having a length of about 100 nucleotides are synthesized, and PCR is carried out by using them. In this case, it is preferred in each of VH and VL that 6 synthetic DNAs are designed in view of the reaction efficiency of PCR and the lengths of DNAs which can be synthesized. Furthermore, they can be easily cloned into the humanized antibody expression vector constructed in the item 2(1) by introducing the recognition sequence of an appropriate restriction enzyme to the 5' end of the synthetic DNAs present on the both ends. After the PCR, an amplified product is cloned into a plasmid such as pBluescript SK (-) (manufactured by Stratagene), and the nucleotide sequences are determined according to the method described in the item 2(2) to obtain a plasmid having nucleotide sequences encoding VH and VL of the anti-CCR4 CDR-grafted antibody of interest.

### (6) Modification of amino acid sequences of VH and VL of human CDR-grafted antibody

It is known that when a human CDR-grafted antibody is produced by simply grafting CDRs in VH and VL of an antibody derived from a non-human animal into FRs in VH and VL of a human antibody, its antigen-binding activity is lower than that of the original antibody derived from a non-human animal (Tempest P.R. *et al*., *Bio*/*technology*, 9, 266-271 (1991)). As the reason, it is considered that several amino acid residues in not only CDRs but also FRs directly or indirectly relate to antigen-binding activity in VH and VL of the original antibody derived from a non-human animal, and that they are changed to different amino acid residues of different FRs in VH and VL of a human antibody. In order to solve the problem, in human CDR-grafted antibodies, among the amino acid sequences of FRs in VH and VL of a human antibody, an amino acid residue which directly relates to binding to an antigen, or an amino acid residue which indirectly relates to binding to an antigen by interacting with an amino acid residue in CDR or by maintaining the three-dimensional structure of an antibody is identified and modified to an amino acid residue which is found in the original non-human animal antibody to thereby increase the antigen binding activity which has been decreased (Tempest P.R., *et al*., *Bio*/*technology*, 9, 266-271 (1991)). In the production of a human CDR-grafted antibody, how to efficiently identify the amino acid residues relating to the antigen binding activity in FR is most important, so that the three-dimensional structure of an antibody is constructed and analyzed by X-ray crystallography (Bernstein F.C. *et al*., *J*. *Mol*. *Biol*., 112, 535-542 (1977)), computer-modeling (Tempest P.R. *et al*., *Protein Engineering,* 7, 1501-1507 (1994)) or the like. Although the information of the three-dimensional structure of antibodies has been useful in the production of a human CDR-grafted antibody, no method for producing a human CDR-grafted antibody which can be applied to any antibodies has been established yet. Therefore, various attempts must be currently be necessary, for example, several modified antibodies of each antibody are produced and the relationship between each of the modified antibodies and its antibody binding activity is examined.

Modification of amino acid residues of FR of VH and VL of a human antibody can be achieved by PCR using synthetic DNA fragments for modification use as primers. A vector containing a cDNA into which a mutation of interest was introduced (hereinafter referred to as "amino acid sequence modification vector") is obtained by determining nucleotide sequence of the amplified product after PCR based on the method described in item 2(2) to thereby confirm that the modification of interest has been carried out.

In addition, in the case of a modification of a narrow range of amino avid sequence, it can be carried out by a PCR mutation introducing method using mutation introducing primers each comprising 20 to 35 bases. Specifically, a sense mutation primer and an antisense mutation primer, each comprising 20 to 35 bases and containing a nucleotide sequence encoding the amino acid residues after modification, are synthesized, and two steps of PCR are carried out by using a plasmid containing cDNA encoding the amino acid sequences of VH and VL to be modified as the template. By subcloning the finally amplified fragment into an appropriate vector and determining its nucleotide sequence, an amino acid sequence modification vector containing a cDNA into which the mutation of interest was introduced is obtained.

### (7) Construction of anti-CCR4- CDR-grafted antibody expression vector

An anti-CCR4 CDR-grafted antibody expression vector can be constructed by cloning cDNAs encoding VH and VL of the anti-CCR4 CDR-grafted antibody constructed in the items 2(5) and 2(6) into upstream of the DNAs encoding CH and CL of the human antibody in the vector for expression of humanized antibody as described in the item 2(1). For example, when recognition sites for an appropriate restriction enzymes are introduced to the 5'-terminal of synthetic DNAs positioned at both ends among synthetic DNAs used in the construction of VH and VL of the anti-CCR4 CDR-grafted antibody in the items 2(5) and 2(6), cloning can be carried out so that they are expressed in an appropriate form in upstream of DNAs encoding CH and CL of the human antibody in the vector for expression of humanized antibody as described in the item 2(1).

### (8) Transient expression and activity evaluation of humanized antibody

In order to efficiently evaluate the antigen binding activity of various humanized antibodies produced, the humanized antibodies can be expressed transiently by using the anti-CCR4 chimeric antibody described in the item 2(4), the anti-CCR4 CDR-grafted antibody expression vector as described in the item 2(7) or the modified expression vector thereof. Any cell can be used as a host cell, so long as the host cell can express a humanized antibody. Generally, COS-7 cell (ATCC CRL1651) is used in view of its high expression amount (Warr G.W. *et al*, *Methods in Nucleic Acids Res.,* CRC Press, 283 (1990)). The method for introducing the expression vector into COS-7 cell includes a DEAE-dextran method (Warr G.W. *et al*., *Methods in Nucleic Acids Res*., CRC Press, 283 (1990)), a lipofection method (Felgner P.L. *et al*., *Proc. Natl*. *Acad. Sci*. *USA*, 84, 7413-7417 (1987)), and the like.

After introduction of the expression vector, the expression amount and antigen binding activity of the humanized antibody in the culture supernatant can be determined by the enzyme-linked immunosorbent assay (ELISA) described in the item 1(2) using the culture supernatant as the primary antibody and a labeled anti-human immunoglobulin antibody as the secondary antibody.

### (9) Stable expression and activity evaluation of humanized antibody

A transformant which produces a humanized antibody stably can be obtained by introducing the anti-CCR4 chimeric antibody expression vector described in the item 2(4) or the anti-CCR4 CDR-grafted antibody expression vector described in the item 2(7) into an appropriate host cell.

The method for introducing the expression vector into a host cell includes electroporation (Japanese Published Unexamined Patent Application No. 257891/90; Miyaji H. *et al*., *Cytotechnology,* 3, 133-140 (1990)) and the like.

Any cell can be used as the host cell into which the anti-CCR4 chimeric antibody expression vector or the anti-CCR4 CDR-grafted antibody expression vector is to be introduced, so long as it can express a humanized antibody. Examples include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is detective (Urlaub G. and Chasin L.A., *Proc. Natl*. *Acad. Sci. U*.*S*.*A*., 77, 4216-4220 (1980)), rat YB2/3HL.P2.G11.16Ag.20 cell (ATCC No: CRL1662, hereinafter referred to as "YB2/0 cell") and the like.

After introduction of the expression vector, transformants which express a humanized antibody stably are selected by culturing in a medium for animal cell culture containing an agent such as G418 (G418 sulfate; manufactured by Sigma Aldrich) (Shitara K. *et al*., *J*. *Immuol*. *Methods,* 167, 271-278 (1994)). The medium for animal cell culture includes RPMI1640 medium (manufactured by Nissui Pharmaceutical), GIT medium (manufactured by Nissui Pharmaceutical), EX-CELL302 medium (manufactured by JRH Biosciences), IMDM medium (manufactured by Invitrogen), Hybridoma-SFM medium (manufactured by Invitrogen), media obtained by adding various additives such as FBS to these media, and the like. The humanized antibody can be produced and accumulated in a culture medium by culturing the selected transformants in a medium. The expression amount and antigen binding activity of the humanized antibody in the culture supernatant can be measured by ELISA or the like. Also, in the transformant, the expression amount of the humanized antibody can be increased by using a DHFR gene amplification system or the like (*J. Immuol*. *Methods,* 167, 271-278 (1994)).

The humanized antibody can be purified from the culture supernatant of the transformant by using a protein A column (*Antibodies: A Laboratory Manual,* Chapter 8, Goding J.W., *Monoclonal Antibodies: Principles and Practice,* Academic Press Limited (1996)). Any other conventional methods for protein purification can be used. For example, the humanized antibody can be purified by a combination of gel filtration, ionexchange chromatography, ultrafiltration and the like. The molecular weight of the H chain or the L chain of the purified humanized antibody or the antibody molecule as a whole can be determined by polyacrylamide gel electrophoresis (SDS-PAGE; laemmli U.K., *Nature*, 227, 680-685 (1970)), Western blotting (*Antibodies: A Laboratory Manual,* Chapter 12; Goding J.W., *Monoclonal Antibodies: Principles and Practice,* Academic Press Limited (1996)) and the like.

Antigen binding activity of the purified humanized antibody can be measured by the above enzyme-linked immunosorbent assay described in the item 1(2) which uses a purified humanized antibody as the primary antibody, and a labeled anti-human immunoglobulin antibody as the secondary antibody, and an immunofluorescent method (*Cancer Immunol*. *Immunother.,* 36, 373 (1993)), a surface plasmon resonance which uses BIAcore™ or the like (Karlsson R. *et al*., *J. Immunol*. *Methods,* 145, 229-240 (1991)) and the like. The cytotoxicity upon antigen-positive cultured cells can be evaluated by measuring CDC activity, ADCC activity and the like (*Cancer Immunol*. *Immunother.,* 36, 373 (1993)). Change in the amount of produced cytokine can be measured by the ELISA, immunofluorescent method and the like.

Specifically, the anti-CCR4 chimeric antibody can be prepared in accordance with the method described in WO01/64754. In addition, the anti-CCR4 CDR-grafted antibody can be prepared in accordance with the method described below.

### 3. Preparation of antibody fragment

The antibody fragment can be prepared based on the anti-CCR4 monoclonal antibody and the anti-CCR4 humanized antibody described in the items 1 and 2 by genetic engineering techniques or protein chemical techniques. The antibody fragment includes Fab, F(ab')₂, Fab', scFv, Diabody, dsFv, a peptide comprising CDR, and the like.

### (1) Preparation of Fab

Fab can be prepared by treating an anti-CCR4 antibody with a proteolytic enzyme, papain. After the papain treatment, when the original antibody is an IgG subclass having a protein A binding activity, uniform Fab can be recovered by separating it from IgG molecules and Fc fragments by passing through a protein A column (Goding J.W., *Monoclonal Antibodies: Principles and Practice,* Academic Press (1996)). When the original antibody is an antibody of IgG subclass having no protein A binding activity, Fab can be recovered by ion exchange chromatography in a fraction eluted at a low salt concentration (Goding J.W., *Monoclonal Antibodies: Principles and Practice,* Academic Press (1996)). In addition, Fab can also be prepared by genetic engineering techniques using *Escherichia coli.* For example, an Fab expression vector can be prepared by cloning the DNA encoding the antibody V region described in the items 2(2), 2(5) and 2(6) into a vector for Fab expression. As the vector for Fab expression, any vector can be used, so long as a DNA for Fab can be inserted and expressed. Examples include pIT106 (Betler M. et al., *Science,* 240, 1041-1043 (1988)) and the like. Fab can be formed and accumulated in an inclusion body or periplasm by introducing the Fab expression vector into an appropriate *Escherichia coli.* Active Fab can be obtained from the inclusion body by a refolding method generally used for protein, and when it is expressed in the periplasmic space, active Fab is leaked in the culture supernatant. Uniform Fab can be purified after the refolding or from the culture supernatant using an antibody-linked column (Boreebeck K., *Antibody Engineering, A Practical Guide,* Oxford University Press (1991)).

### (2) Preparation of F(ab')₂

F(ab')₂ can be prepared by treating an anti-CCR4 antibody with a proteolytic enzyme, pepsin. After the pepsin treatment, it can be recovered as uniform F(ab')₂ by a purification procedure similar to the case of Fab (Goding J. W., *Monoclonal Antibodies: Principles and Practice,* Academic Press (1996)). In addition, it can also be prepared by the method described in the item 3(3) in which Fab' is treated with maleimide such as N,N'-o-phenylenedimaleimide or bismaleimide hexane to form a thioether bond, or a method in which it is treated with 5,5'-dithiobis(2-nitrobenzoic acid) (DTNB) to form a disulfide bond (MaCafferty J. *et al*., *Antibody Engineering, A Practical Approach,* IRL PRESS (1996)).

### (3) Preparation of Fab'

Fab' can be obtained by treating the F(ab')₂ described in the item 3(2) with a reducing agent such as dithiothreitol. Also, Fab' can be prepared by genetic engineering techniques using *Escherichia coli.* For example, an Fab' expression vector can be constructed by cloning the DNA encoding the antibody V region described in the items 2(2), 2(5) and 2(6) into a vector for expression of Fab'. As the vector for expression of Fab', any vector can be used, so long as a DNA encoding the V region of the antibody described in the items 2(2), 2(5) and 2(6) can be inserted and expressed. Examples include pAK19 (Carter P. *et al*., *Bio*/*technology*, 10, 163-167 (1992)) and the like. Fab' can be formed and accumulated in an inclusion body or periplasmic space by introducing the Fab' expression vector into an appropriate *Escherichia coli.* Active Fab' can be obtained from the inclusion body by a refolding method generally used for protein, and when it is expressed in the periplasmic space, it can be recovered into extracellular moiety by disrupting the cells with a treatment such as lysozyme partial digestion, osmotic pressure shock, sonication or the like. Uniform Fab' can be purified after the refolding or from the disrupted cell suspension using a protein G column or the like (MaCafferty J. *et al*., *Antibody Engineering, A Practical Approach,* IRL PRESS (1996)).

### (4) Preparation of scFv

scFv can be prepared using a phage or *Escherichia coli* by genetic engineering techniques. For example, a DNA encoding scFv is produced by ligating DNAs encoding the antibody VH and VL described in the items 2(2), 2(5) and 2(6) via a DNA encoding a polypeptide linker comprising an amino acid sequence of 12 residues or more. It is important that the polypeptide linker is optimized so that its addition does not inhibit the binding of VH and VL to the antigen. For example, the polypeptide linker shown by Pantoliano *et al*. (Pantorliano M.W. *et al*, *Biochemistry*, 30, 10117-10125 (1991)) and the modified one can be used.

An scFv expression vector can be constructed by cloning the resulting DNA into a vector for scFv expression. As the vector for scFv expression, any vector can be used, so long as a DNA for scFv can be inserted and expressed. Examples include pCANTAB5E (manufactured by Amasham Biosciences), Phfa (Lah M. *et al*., *Hum. Antibody Hybridoma*, 5, 48-56 (1994)) and the like. The scFv expression vector was introduced into an appropriate *Escherichia coli* and infected with a helper phage to thereby obtain a phage which expresses scFv on the phage surface in a fused form with the phage surface protein. Also, scFv can be formed and accumulated in the inclusion body or periplasmic space of *Escherichia coli* into which scFv expression vector is introduced. Active scFv can be obtained from the inclusion body by a refolding method generally used for protein, and when it is expressed in the periplasmic space, it can be recovered extracellularly by disrupting the cells with a treatment such as lysozyme partial digestion, osmotic pressure shock, sonication or the like. Uniform scFv can be purified after the refolding or from the disrupted cell suspension by cation exchange chromatography or the like (McCafferty J. *et al*., *Antibody Engineering, A Practical Approach,* IRL PRESS (1996)).

### (5) Preparation of diabody

Diabody can be prepared by changing the size of the polypeptide linker for preparing scFv to about 3 to 10 residues. A divalent diabody can be prepared when VH and VL of one antibody species is used, and a diabody having two different specificity can be prepared when VH and VL of two antibody species are used (Le Gall F. *et al*., *FEBS Letters,* 453, 164-168 (1999), Courage C. *et al*., *Int. J*. *Cancer*, 77, 763-768 (1998)).

### (6) Preparation of dsFv

dsFv can be prepared using *Escherichia coli* by genetic engineering techniques. First, DNAs in which an encoded amino acid residue is replaced with a cysteine residue are produced by introducing mutation into appropriate positions of the DNAs encoding the antibody VH and VL described in the items 2(2), 2(5) and 2(6). The modification of the amino acid residue to a cystein residue can be carried out by the mutation introduction method using PCR of the item 2(6). VH and VL expression vectors can be produced by cloning each of the resulting DNAs into a vector for dsFv expression. As the vector for dsFv expression, any vector can be used, so long as a DNA for dsFv can be inserted and expressed. Examples include pULI9 (Reiter Y. *et al*., *Protein Engineering,* 7, 697-704 (1994)) and the like. The VH and VL expression vectors are introduced into an appropriate *Escherichia coli* to thereby form and accumulate the VH and VL in the inclusion body or periplasmic space. The VH and VL are obtained from the inclusion body or periplasmic space and mixed, and active dsFv can be obtained by a refolding method generally used for protein. After the refolding, it can be further purified by ion exchange chromatography and gel filtration or the like (Reiter Y. *et al*., *Protein Engineering,* 7, 697-704 (1994)).

### (7) Preparation of peptide comprising CDR

A peptide comprising CDR can be prepared by a chemical synthesis method such as Fmoc, tBoc or the like. Also, a DNA encoding a peptide comprising CDR is prepared, and the resulting DNA is cloned into an appropriate vector for expression to thereby prepare the peptide comprising CDR. As the vector for expression, any vector can be used, so long as a DNA encoding a peptide comprising CDR is inserted and expressed. Examples include pLEX (manufactured by Invitrogen), pAX4a+ (manufactured by MoBiTec) and the like. The expression vector is introduced into an appropriate *Escherichia coli* so that the peptide comprising CDR can be formed and accumulated in the inclusion body or periplasmic space. The peptide comprising CDR can be obtained from the inclusion body or periplasmic space, and it can be purified by ion exchange chromatography and gel filtration or the like (Reiter Y. *et al*., *Protein Engineering,* 7, 697 (1994)).

### (8) Evaluation of activity

The antigen binding activity of the above antibody fragments can be measured by the enzyme-linked immunosorbent assay described in the item 1(2) which uses an antibody fragment as the primary antibody, the surface plasmon resonance (Karlsson R. *et al*., *J*. *Immunol*. *Methods*, 145, 229-240 (1991)) and the like.

### 4. The diagnostic agent, preventive agent and therapeutic agent of the present invention

The anti-CCR4 antibody binds to the CCR4 protein distributing on the cell membrane surface of a CCR4 expressing cell infiltrated in peripheral blood or an inflammatory site. The anti-CCR4 antibody inhibits activation and migration of CCR4-positive cells by inhibiting functions of CCR4. Also, a CCR4 expressing cell is injured by the CDC activity or ADCC activity of the anti-CCR4 antibody linked to the cell surface. The CCR4-positive cells include a group of cells which have been found to play an important role in the formation of an allergic inflammation, also called Th2 cells.

Also, the anti-CXCR3 antibody binds to the CXCR3 protein distributing on the cell membrane surface of a CXCR3 expressing cell infiltrated in peripheral blood or an inflammatory site. The anti-CXCR3 antibody inhibits activation and migration of CXCR3-positive cells by inhibiting functions of CXCR3. Also, a CXCR3 expressing cell is injured by the CDC activity or ADCC activity of the anti-CXCR3 antibody linked to the cell surface. The CXCR3-positive cells include a group of cells which have been found to play an important role in the formation of an allergic inflammation, also called Th1 cells.

Accordingly, a medicament comprising an anti-CCR4 antibody and/or an anti-CXCR3 antibody can be used in the diagnosis, prevention and treatment of interstitial pneumonia which is one of inflammatory diseases.

It is considered that, in the case of an inflammatory disease, morbid state of the disease progresses when ratio of the Th2 cells and Th1 cells infiltrating into the inflammatory part becomes markedly unbalanced. Accordingly, inflammation of the interstitial pneumonia, pulmonary fibrosis induced after the inflammatory reaction and reduction of respiratory function can be diagnosed, prevented and treated by grasping ratio of the Th2 cells and Th1 cells using the discrimination method which is described later in 5, and injuring excess infiltrated cells.

As the subjects to be diagnosed, prevented or treated by the anti-CCR4 antibody, it is effective in diseases in which infiltration of the Th2 cells is excessively found in the inflammatory region in comparison with that of the Th1 cells, such as usual idiopathic interstitial pneumonia

As the subjects to be diagnosed, prevented or treated by the anti-CXCR3 antibody, it is effective in diseases in which infiltration of the Th1 cells is excessively found in the inflammatory region in comparison with that of the Th2 cells, such as non-specific idiopathic interstitial pneumonia, interstitial pneumonia in collagen diseases such as rheumatoid arthritis, dermatomyositis and polymyositis, and the like.

In addition, in the case that the morbid state of the disease progresses even when the ratio of the Th2 cells and Th1 cells infiltrating into the inflammatory region is balanced, inflammation of the interstitial pneumonia, pulmonary fibrosis induced after the inflammatory reaction and reduction of respiratory function can be diagnosed, prevented and treated by injuring the infiltrating T cells.

Also, the medicament "comprising an anti-CCR4 antibody and an anti-CXCR3 antibody as active ingredients" may have any form, and examples include a medical mixture, a preparation in which the anti-CCR4 antibody and anti-CXCR3 antibody are linked to each other, and a kit in which the anti-CCR4 antibody and anti-CXCR3 antibody are separately contained.

Since most part of amino acids consisted by a humanized antibody is derived from the amino acid sequence of a human antibody in comparison with the case of a monoclonal antibody derived from a non-human animal, it is expected to show high efficacy in the human body with low immunogenicity, and the effects are continued for a long time and therefore preferred as a preventive agent and a therapeutic agent.

The medicament comprising the anti-CCR4 antibody and/or the anti-CXCR3 antibody can be administered as a preventive agent or a therapeutic agent alone, but it is generally preferred to provide it in the form of a pharmaceutical formulation produced by mixing it with at least one pharmaceutically acceptable carrier in accordance with a method well known in the technical field of pharmaceutics.

It is preferred to select a route of administration which is the most effective in carrying out the intended treatment such as oral administration or parenteral administration, e.g., intraoral administration, tracheal administration, rectal administration, subcutaneous injection, intramuscular injection, intraarticular injection, intravenous injection, and the like. Intraarticular injection and intravenous injection are preferred in case of an antibody or peptide formulation.

The dosage form includes sprays, capsules, tablets, granules, syrups, emulsions, suppositories, injections, ointments, tapes, and the like.

Formulations suitable for oral administration include emulsions, syrups, capsules, tablets, powders, granules, and the like.

Liquid preparations such as emulsions and syrups, can be produced using additives such as water; saccharides, e.g., sucrose, sorbitol, fructose; glycols, e.g., polyethylene glycol, propylene glycol; oils, e.g., sesame oil, olive oil, soybean oil; antiseptics, e.g., p-hydroxybenzoate; and flavors, e.g., strawberry flavor, peppermint.

Capsules, tablets, powders, granules and the like can be produced using additives such as fillers, e.g., lactose, glucose, sucrose, mannitol; disintegrating agents, e.g., starch, sodium alginate; lubricants, e.g., magnesium stearate; talc, binders, e.g., polyvinyl alcohol, hydroxypropylcellulose, gelatin; surfactants, e.g., fatty acid esters; and plasticizers, e.g., glycerine.

Formulations suitable for parenteral administration include injections, suppositories, sprays, and the like.

Injections can be prepared using a carrier such as a salt solution, glucose solution or a mixture thereof, or the like.

Suppositories can be prepared using a carrier such as cacao butter, hydrogenated fat, a carboxylic acid, or the like.

Also, sprays can be prepared from the antibody itself or using a carrier or the like which does not stimulate oral and airway mucous membranes of patients and can facilitate absorption of the antibody or antibody fragment thereof by dispersing it as minute particles.

The carrier includes lactose, glycerine, and the like. Depending on the properties of the antibody or peptide and the carrier to be used, aerosols, dry powders and the like can be produced. The additives exemplified in the oral preparations can also be added to the parenteral preparations.

The dose and frequency of administration vary depending on intended therapeutic effect, administration method, treating period, age, body weight and the like, but the dose is generally from 10µg/kg to 20 mg/kg per day per adult.

As the dosage forms and route of administration in administering the anti-CCR4 antibody to a model animal, they can be optionally selected according to the properties and seriousness of the model animal to be tested. For example, the antibody can be administered to the model animal orally or parenterally (intraperitoneal, intravenous, intraarticular, intramuscular, subcutaneous administration and the like), as such or together with other pharmaceutically acceptable carrier, filler, diluent and the like.

The mixing amount and dose of the anti-CCR4 antibody are not particularly limited but decided depending on each administration method of the pharmaceutical preparation, administration form, purpose for use, specific symptoms of each model animal, body weight of the model animal and the like, and a range of approximately from 1 µg/kg to 100 mg/kg per day is possible as the dose and approximately once a day is possible as the administration interval, but it can also be administered by dividing the daily dose into 2 to 4 doses per day or more. In addition, it is also possible to administer continuously for example by drip infusion or the like. When administered to a topical part such as a joint, a dose of roughly 1 pg to 100 mg is administered per one region.

### 5. Method for discriminating between usual idiopathic interstitial pneumonia and non-specific idiopathic interstitial pneumonia, and diagnostic agent for the discrimination

Since the anti-CCR4 antibody specifically binds to CCR4, a CCR4-positive cell can be detected and determined. Also, since the anti-CXCR3 antibody specifically binds to CXCR3, a CXCR3-positive cell can be detected and determined.

Among patients of idiopathic interstitial pneumonia, the number of CCR4-positive cells is increased in the lung tissues of patients of usual idiopathic interstitial pneumonia in comparison with the number of CXCR3-positive cells. On the other hand, the number of CXCR3-positive cells is increased in the lung tissues of patients of non-specific idiopathic interstitial pneumonia in comparison with the number of CCR4-positive cells. Thus, usual idiopathic interstitial pneumonia and non-specific idiopathic interstitial pneumonia can be discriminated by allowing the anti-CCR4 antibody and anti-CXCR3 antibody to react with biological samples of patients of idiopathic interstitial pneumonia.

In addition, since the diagnostic agent of the present invention can discriminate between CCR4-positive cells and CXCR3-positive cells, therapeutic agent of the above item 4 can be selected by discriminating between patients having significant infiltration of CCR4-positive cells and patients having significant infiltration of CXCR3-positive cells, among patients of interstitial pneumonia.

As the biological samples, a lung tissue section collected from a person to be tested by biopsy or the like, a cell extract prepared from the cell or tissue, blood and the like can be cited.

As the anti-CCR4 antibody to be used in the diagnostic agent of the present invention, any antibody can be used, so long as it specifically binds to CCR4, and any one of a polyclonal antibody, a monoclonal antibody, a gene recombinant antibody, an antibody fragment thereof and the like is included in the antibody to be used in the present invention. The gene recombinant antibody includes humanized antibodies such as a human chimeric antibody and a human CDR-grafted antibody. The antibody fragment includes Fab, F(ab')₂, Fab', scFv, Diabody, dsFv, a CDR-containing peptide and the like.

The diagnostic agent which comprises an anti-CCR4 antibody and/or an anti-CXCR3 antibody may contain a reagent for carrying out an antigen-antibody reaction and a reagent for detecting the reaction, according to the diagnostic method shown below. The reagent for carrying out an antigen-antibody reaction includes a buffer agent, a salt and the like. The reagent for detection includes reagents which are used in general immunological detection methods such as a labeled secondary antibody capable of recognizing an anti-CCR4 antibody or an anti-CXCR3 antibody, and a label-corresponding substrate.

As the method for immunologically detecting and/or determining CCR4-positive cells or CXCR3-positive cells infiltrating into a biological sample using an anti-CCR4 antibody or an anti-CXCR3 antibody, an immunofluorescent method, enzyme-linked immunosorbent assay (ELISA), radioimmunoassay (RIA), tissue immunostaining, cell immunostaining, Western blotting, immunoprecipitation, sandwich ELISA (Sakuji Toyama and Tamie Ando, *Monoclonal Antibody Experimentation Manual,* Kodan-sha Scientific (1987); The Japanese Biochemical Society, *Second Series Biochemical Experimentation Course,* 5, Methods for Studying Immuno-Biochemistry, Tokyo Kagaku Dojin (1986)) and the like can be used.

The immunofluorescent method can be carried out by using the methods described in references (Goding J.W., *Monoclonal Antibodies: Principles and Practice,* Academic Press (1996); Sakuji Toyama and Tamie Ando, *Monoclonal Antibody Experimentation Manual,* Kodan-sha Scientific (1987)) and the like. Specifically, this method is a method in which an anti-CCR4 antibody is allowed to react with an isolated cell, tissue or the like and then allowed to react with an anti-immunoglobulin antibody labeled with a fluorescent material such as fluorescein isothiocyanate (FITC) or phycoerythrin, and then the fluorescence dye is measured by using a flow cytometer.

The enzyme-linked immunosorbent assay (ELISA) is a method in which an anti-CCR4 antibody is allowed to react with an isolated cell, tissue or the like and then allowed to react with an anti-immunoglobulin antibody labeled with an enzyme such as peroxidase or alkaline phosphatase, and then a substrate which develops a color by the enzyme reaction is added to react and the color-developed rye is measured by using a spectrophotometer.

The radioimmunoassay (RIA) is a method in which an anti-CCR4 antibody is allowed to react with an isolated cell, tissue or the like and then allowed to react with an anti-immunoglobulin antibody labeled with a radioisotope, and then the radioactivity is measured by using a scintillation counter or the like.

The cell immunostaining or tissue immunostaining is a method in which an anti-CCR4 antibody is allowed to react with an isolated tissue or the like and then allowed to react with an anti-immunoglobulin antibody labeled with a fluorescent material such as FITC or an enzyme such as peroxidase or alkaline phosphatase, and then, in the case of an enzyme labeling, a substrate which develops a color by the enzyme reaction is added to react and the sample is observed under a microscope, and this method can be carried out by using the methods described in references (Goding J.W., *Monoclonal Antibodies: Principles and Practice,* Academic Press (1996); S. Toyama and T. Ando, *Monoclonal Antibody Experimentation Manual,* Kodan-sha Scientific (1987)) and the like.

Western blotting is a method in which an isolated cell or tissue is dissolved in an SDS-containing buffer for sample and subjected to SDS-polyacrylamide gel electrophoresis, and the gel is transferred on a poly(vinylidene difluoride) (PVDF) membrane, allowed to react with an anti-CCR4 antibody and further allowed to react with an anti-immunoglobulin antibody labeled with an enzyme such as peroxidase or alkaline phosphatase, and then a substrate which develops a color by the enzyme reaction or chemiluminescence is added to proceed the reaction and the substance of interest is detected as a band.

The immunoprecipitation is a method in which a disrupted suspension of an isolated cell or tissue and an anti-CCR4 antibody immobilized on beads or the like are allowed to react with each other, the beads are isolated by centrifugation or the like and then treated with an SDS-containing buffer for sample use, and the thus dissolved CCR4 is detected by Western blotting or the like.

The sandwich ELISA is an enzyme-linked immunosorbent assay which uses two anti-CCR4 antibodies having different epitopes. One of the anti-CCR4 antibodies is immobilized on a plate and allowed to react with a disrupted suspension of an isolated cell or tissue, and then the other anti-CCR4 antibody is further allowed to react with the CCR4 bound to the anti-CCR4 antibody on the plate. After reaction with an anti-immunoglobulin antibody labeled with an enzyme such as peroxidase or alkaline phosphatase, a substrate which develops a color by the enzyme reaction is added to reaction and then the color-developed rye is measured by using a spectrophotometer.

The present invention is described in the following based on the Examples and Reference Examples, but the present invention is not limited thereto.

### Brief Description of the Drawings

Fig. 1 shows construction steps of a plasmid pKM2160Gal0.
Fig. 2 shows construction steps of a plasmid pKM2160Gal1. The symbol * shows the position of mutation genetic codon modifying an amino acid residue.
Fig. 3 shows construction steps of a plasmid pKM2160Gal3. The symbol * shows the position of mutation genetic codon modifying an amino acid residue.
Fig. 4 shows construction steps of a plasmid pKM2160LV0.
Fig. 5 shows construction steps of a plasmid pKM2160LV 1. The symbol * shows the position of mutation genetic codon modifying an amino acid residue.
Fig. 6 shows construction steps of a plasmid pKANTEX2160LV0 and plasmid pKANTEX2160Gal0LV0.
Fig. 7 shows reactivity of a culture supernatant obtained by transiently expressing an expression vector of each anti-CCR4 CDR-grafted antibody in COS-7 cell, with a CCR4 partial peptide according to ELISA.
Fig. 8 shows reactivity of a culture supernatant obtained by transiently expressing an expression vector of each anti-CCR4 CDR-grafted antibody prepared by using other FRs in COS-7 cell, with CCR4 partial peptide according to ELISA.
Fig. 9 shows reactivity of a purified anti-CCR4 CDR-grafted antibody with a CCR4 partial peptide.
Fig. 10 shows reactivity of a purified anti-CCR4 CDR-grafted antibody with a CCR4 high expression cell (CCR4/EL-4).
Fig. 11 shows affinity of a purified anti-CCR4 CDR-grafted antibody with a CCR4 partial peptide measured using a surface plasmon resonance sensor.

### Best Mode for Carrying Out the Invention

### Example 1

### Staining of lung tissues of patients of idiopathic interstitial pneumonia using anti-CCR4 antibody:

T cells are accumulated in inflammatory regions. Accordingly, T cells accumulated in lung tissues of patients of idiopathic interstitial pneumonia were stained by using an antibody which reacts with CXCR3 expressing on the cell surface of Th1 cells and an antibody which reacts with CCR4 expressing on the cell surface of Th2 cells.

Paraffin sections were prepared from lung tissues collected from idiopathic interstitial pneumonia patients (14 cases) in accordance with the method described in a reference (*Histotechnology*: A self-instruction test. ASCP Press (1990)).

Immunostaining of the lung tissue paraffin sections was carried out using 6.32 µg/ml of a mouse anti-CCR4 antibody KM2160, 6.32 µg/ml of an anti-CXCR3 antibody (manufactured by Pharmingen) and a commercially available tissue immunostaining kit (SS MultiLink Detection Kit, manufactured by BioGenex). The method was carried out in accordance with the instructions attached to the kit.

The number of CXCR3- or CCR4-positive cells was calculated by selecting 20 fields of view at random in the case of fibrous region (400 magnifications) and counting the number of antibody-positive mononuclear cells in each field of view. The results are shown in Table 1.

**Table 1**

| Distribution of CXCR3- and CCR4-positive cells in idiopathic interstitial pneumonia | | | | | |
|---|---|---|---|---|---|
| Patients | Age | Diagnosis | The number of positive cells | | |
| | | | CXCR3 | CCR4 | CXCR3:CCR4 ratio |
| 1 | 74 | NSIP | 313 | 2 | 156.5 |
| 2 | 66 | NSIP | 395 | 12 | 32.9 |
| 3 | 60 | NSIP | 173 | 2 | 86.5 |
| 4 | 74 | NSIP | 195 | 12 | 16.3 |
| 5 | 52 | NSIP | 347 | 16 | 21.7 |
| 6 | 67 | NSIP | 321 | 9 | 35.7 |
| 7 | 67 | NSIP | 545 | 7 | 77.9 |
| 8 | 69 | NSIP | 274 | 3 | 91.3 |
| 9 | 73 | NSIP | 43 | 2 | 21.5 |
| 10 | 74 | NSIP | 220 | 31 | 7.1 |
| 11 | 62 | NSIP | 285 | 12 | 23.8 |
| 12 | 76 | NSIP | 22 | 6 | 3.7 |
| 13 | 73 | UIP | 43 | 94 | 0.46 |
| 14 | 61 | UIP | 45 | 66 | 0.68 |
| UIP: usual interstitial pneumonia | | | | | |
| NSIP: non-specific interstitial pneumonia | | | | | |

In two cases of usual idiopathic interstitial pneumonia (patients 13 and 14), predominant infiltration of CCR4-positive cells, namely Th2 cells, was observed. Contrary to this, predominant infiltration of CXCR3-positive cells, namely Th1 cells, was observed in 12 cases of non-specific idiopathic interstitial pneumonia (patients 1 to 12).

Accordingly, it is possible to diagnose idiopathic interstitial pneumonia by using the anti-CCR4 antibody and anti-CXCR3 antibody. Also, it is possible to discriminate between usual idiopathic interstitial pneumonia and non-specific idiopathic interstitial pneumonia by examining the ratio of infiltrated Th1 cells and Th2 cells by using the anti-CCR4 antibody and anti-CXCR3 antibody by examining the ratio of Th1 cells and Th2 cells.

In addition, it was suggested that the usual idiopathic interstitial pneumonia can be treated by the use of the anti-CCR4 antibody, and that the non-specific idiopathic interstitial pneumonia can be treated by the use of the anti-CXCR3 antibody.

### Example 2

### Staining of lung tissues of patients of collagen diseases using anti-CCR4 antibody:

Patients of collagen diseases are suffering from interstitial pneumonia in many cases. Accordingly, T cells accumulated in lung tissues of patients suffering from interstitial pneumonia due to collagen diseases such as dermatornyositis, polymyositis, systemic sclerosis (scleroderma) and rheumatoid arthritis were stained in the same manner as in Example 1 by using antibodies which react respectively with CXCR3 expressing on the cell surface of Th1 cells and with CCR4 expressing on the cell surface of Th2 cells.

Paraffin sections were prepared from lung tissues of interstitial pneumonia of collagen disease patients (CVD-IP) in accordance with the method described in the reference (*Histotechnology*: A self-instruction test. ASCP Press (1990)). Immunostaining of the tissues was carried out using an anti-CCR4 antibody KM2160, a commercially available anti-CXCR3 antibody (manufactured by Pharmingen) and a commercially available tissue immunostaining kit (SS MultiLink Detection Kit, manufactured by BioGenex) in accordance with the instructions attached to the kit. The number of CXCR3- or CCR4-positive cells was calculated by selecting 5 fields of view at random in the case of fibrous region (400 magnifications) and counting the number of antibody-positive mononuclear cells in each field of view. For the lymph follicle region, the counting was carried out in the same manner also by selecting 5 fields of view. The results are shown in Table 2.

**Table 2**

| Distribution of CXCR3- and CCR4-positive cells in CVD-IP | | | | | |
|---|---|---|---|---|---|
| Patients | Age | Diagnosis | The number of positive cells | | |
| | | | CXCR3 | CCR4 | CXCR3:CCR4 ratio |
| 1 | 64 | RA | 10 | 4 | 2.5 |
| 2 | 68 | RA | 110 | 10 | 11.0 |
| 3 | 89 | RA | 92 | 6 | 15.3 |
| 4 | 59 | RA | 60 | 5 | 12 |
| 5 | 71 | RA | 208 | 60 | 77.6 |
| 6 | 73 | RA | 309 | 15 | 20.6 |
| 7 | 57 | RA | 48 | 8 | 6 |
| 8 | 56 | RA | 159 | 41 | 3.9 |
| 9 | 64 | RA | 140 | 25 | 5.6 |
| 10 | 62 | RA | 280 | 10 | 28 |
| 11 | 72 | RA | 298 | 2 | 149 |
| 12 | 44 | DM | 80 | 1 | 80 |
| 13 | 69 | DM | 30 | 3 | 10 |
| 14 | 45 | DM | 38 | 6 | 6.3 |
| 15 | 56 | PM | 30 | 10 | 3 |
| 16 | 61 | PM | 51 | 20 | 2.6 |
| 17 | 65 | PM | 41 | 25 | 1.6 |
| 18 | 58 | PM | 138 | 58 | 2.4 |
| 19 | 57 | DM | 19 | 9 | 2.1 |
| 20 | 50 | SSc | 21 | 1 | 21.0 |
| 21 | 58 | SSc | 20 | 72 | 0.28 |
| 22 | 56 | SSc | 17 | 44 | 0.39 |
| 23 | 38 | SSc | 231 | 46 | 5.0 |
| 24 | 50 | SSc | 23 | 34 | 0.68 |
| 25 | 41 | SSc | 41 | 4 | 10.0 |
| 26 | 67 | SSc | 20 | 20 | 1 |
| 27 | 60 | SSc | 96 | 43 | 2.2 |
| DM: dermatomyositis | | | | | |
| PM: polymyositis | | | | | |
| SSc: systemic sclerosis (scleroderma) | | | | | |
| RA: rheumatoid arthritis | | | | | |

In the lung tissues collected from patients of systemic sclerosis (patients 20 to 27), a case showing predominant infiltration of CXCR3-positive cells (patients 20, 23, 25 and 27) and a case showing predominant infiltration of CCR4-positive cells (patients 21, 22 and 24) were observed.

Thus, it was found that, among patients of systemic sclerosis, the anti-CCR4 antibody is useful as a therapeutic agent for a case showing predominant infiltration of CCR4-positive cells, and the anti-CXCR3 antibody is useful as a therapeutic agent for a case showing predominant infiltration of CXCR3-positive cells.

The above results show that an appropriate therapeutic method can be selected by carrying out staining of lung lesion tissues of collagen disease patients and carrying out diagnosis of morbid states of the patients.

### Reference Example 1

### Preparation of human CDR-grafted antibody against CCR4 (anti-CCR4 CDR-grafted antibody):

### 1. Designing of cDNA encoding VH and VL of anti-CCR4 CDR-grafted antibody

### (1) Designing of amino acid sequence of VH of anti-CCR4 CDR-grafted antibody

First, an amino acid sequence of the VH of an anti-CCR4 CDR-grafted antibody was designed as follows. An amino acid sequence of FR of VH of a human antibody was selected for grafting amino acid sequences of CDR1, 2 and 3 of VH represented by SEQ ID NOs:2, 3 and 4 using the anti-CCR4 mouse antibody KM2160 produced by according to the method described in Example 1 of WO01/64754. Human antibodies having high homology with KM2160 were retrieved from amino acid sequence data bases of existing proteins by BLASTP method (*Nucleic Acid Res.,* 25, 3389 (1997)) using GCG Package (manufactured by Genetics Computer Group) as a sequence analyzing system. When the homology of the actual amino acid sequence was compared with the homology scores, SWISSPROT data base accession number P01781, Ig Heavy chain V-III region Gal (*Hoppe. Seylers*. *Z. Physiol. Chem*., 354, 1505-1509 (1973); hereinafter referred to as "Gal") was a human antibody showing the highest homology of 82.5%, so that the FR amino acid sequence of the antibody was selected. However, positions where the amino acid residues cannot be determined uniquely (positions 28 and 30 from the N-terminal of a secretory antibody) and an amino acid residue which has low generation frequency in sequences of human antibodies (Thr as the final residue of V region) were found in the FR amino acid sequence of Gal on the data base. Accordingly, Ile and Ser as residues found in the mouse KM2160 were selected as positions 28 and 30, and Thr as the final residue of V region was substituted with Ser. Since the amino acid residues are found at high frequencies in sequences of any human antibodies (*Sequences of Proteins of Immunological Interest*), they do not deviate from human antibody sequences.

The VH amino acid sequence GalO of anti-CCR4 CDR-grafted antibody represented by SEQ ID NO:10 was designed by grafting amino acid sequences of CDR1, 2 and 3 of VH of the anti-CCR4 mouse antibody KM2160 represented by SEQ ID NOs:2, 3 and 4, respectively, to appropriate positions in the determined human antibody FR amino acid sequence. A nucleotide sequence encoding the amino acid sequence of SEQ ID NO:10 is represented by SEQ ID NO:24.

Also, an amino acid sequence of VH of the anti-CCR4 CDR-grafted antibody was designed based on the common sequences classified by Kabat *et al*.

Kabat *et al*. have classified the VH of various already known human antibodies into three subgroups (HSG I to III) based on the homology of their amino acid sequences and reported common sequences in each subgroup (*Sequences of Proteins of Immunological Interest*). There is a possibility that immunogenicity of the common sequences will be reduced in human. Accordingly, in order to prepare an anti-CCR4 CDR-grafted antibody having high activity, among FR amino acid sequences of the common sequences of three subgroups of the human antibody VH, an FR amino acid sequence having the highest homology with the FR amino acid sequence of VH of KM2160 was selected in the designing. Table 3 shows a result of the retrieval of homology between the FR amino acid sequences of the common sequences of each subgroup of the human antibody VH and the FR amino acid sequence of VH of KM2160. As shown in Table 3, the FR amino acid sequence of the VH region of KM2160 showed the highest homology with the subgroup III.

**Table 3**

| Homology between the amino acid sequence of FR in the common sequence of subgroups of VH of human antibody and the amino acid sequence of FR in VH of KM2160 | | |
|---|---|---|
| HSG I | HSG II | HSG III |
| 57.47% | 50.58% | 77.01% |

Based on the above results, the VH amino acid sequence HV0 of anti-CCR4 CDR-grafted antibody represented by SEQ ID NO:11 was designed by grafting amino acid sequence of CDR of VH of the anti-CCR4 mouse antibody KM2160 to an appropriate position of the amino acid sequence of FR of the common sequence of subgroup III of the human antibody VH. A nucleotide sequence encoding the amino acid sequence of SEQ ID NO:11 is represented by SEQ ID NO:25.

### (2) Designing of amino acid sequence of VL of human CDR-grafted antibody against CCR4

Next, an amino acid sequence of VL of an anti-CCR4 CDR-grafted antibody was designed as follows. An amino acid sequence of FR of VL of a human antibody was selected for grafting amino acid sequences of CDR1, 2 and 3 of VL of anti-CCR4 mouse antibody KM2160 represented by SEQ ID NOs:5, 6 and 7, respectively. Kabat *et al*. have classified the VL of various already known human antibodies into four subgroups (HSG I to IV) based on the homology of their amino acid sequences and reported common sequences in each subgroup (*Sequences of Proteins of Immunological Interest*). Accordingly, among FR amino acid sequences of the common sequences of four subgroups of the human antibody VL, an FR amino acid sequence having the highest homology with the FR amino acid sequence of VL of KM2160 was selected. Table 4 shows a result of the retrieval of homology. As shown in Table 4, the FR amino acid sequence of VL of KM2160 showed the highest homology with the subgroup II.

**Table 4**

| Homology between the amino acid sequence of FR in the common sequence of subgroups of VL of human antibody and the amino acid sequence of FR in VL of KM2160 | | | |
|---|---|---|---|
| HSG I | HSG II | HSG III | HSG IV |
| 65.00% | 82.50% | 65.00% | 72.50% |

Based on the above results, the VL amino acid sequence LV0 of anti-CCR4 CDR-grafted antibody represented by SEQ ID NO:12 was designed by grafting the amino acid sequences of CDR1, 2 and 3 of VL of anti-CCR4 mouse antibody KM2160 represented by SEQ ID NOs: 5, 6 and 7, respectively, to appropriate positions in the amino acid sequence of FR of the common sequence of subgroup II of the human antibody VL. A nucleotide sequence encoding the amino acid sequence of SEQ ID NO:12 is represented by SEQ ID NO:26.

### (3) Modification of VH and VL of human CDR-grafted antibody against CCR4

The VH amino acid sequences Gal0 and HV0, and VL amino acid sequence LV0 of anti-CCR4 CDR-grafted antibody designed in the above are antibodies in which the CDR amino acid sequence of the anti-CCR4 mouse antibody KM2160 alone is grafted to the selected FR amino acid sequences of human antibody. However, when grafting with only CDR amino acid sequence of a mouse antibody is carried out, the activity of a human CDR-grafted antibody is frequently decreased so that, in order to avoid the decrease, certain amino acid residues among the FR amino acid residues different between a human antibody and a mouse antibody, which are considered to have influences on the activity, are generally grafted together with the CDR amino acid sequence. Accordingly, in this Reference Example, an examination was carried out to identify the FR amino acid residues considered to have influences on the activity.

First, three-dimensional structures of antibody V regions (Gal0LV0 and HV0LV0) comprising amino acid sequences Gal0 and HV0 of VH and amino acid sequence LV0 of VL in the anti-CCR4 CDR-grafted antibody designed in the above were constructed by using computer modeling techniques. The three-dimensional structure coordinates were prepared by using a software AbM (manufactured by Oxford Molecular), and display of the three-dimensional structures by using a software Pro-Explore (manufactured by Oxford Molecular) or RasMol (manufactured by Glaxo) according to the respective attached manufacture's instructions. Also, computer models of the three-dimensional structures of V regions of anti-CCR4 mouse antibody KM2160 were constructed in the same manner. In addition, three-dimensional structure models comprising modified amino acid sequences were constructed in the same manner, in which certain residues of the FR amino acid sequences of VH and VL of Gal0LV0 or HV0LV0, different from the anti-CCR4 mouse antibody KM2160, were substituted with other residues found at corresponding positions in the anti-CCR4 mouse antibody KM2160, and the three-dimensional structures of V regions of the anti-CCR4 mouse antibody KM2160, Gal0LV0 or HV0LV0 and the modified product were compared.

As a result, the three-dimensional structure of the antigen binding region was changed so that Ala at position 40, Gly at position 42, Lys at position 43, Gly at position 44, Lys at position 76 and Ala at position 97 in Gal0, Thr at position 28 and Ala at position 97 for HV0 and Ile at position 2, Val at position 3, Gln at position 50 and Val at position 88 in LV0 were selected as residues considered to have influence on the activity of antibody among the FR amino acid residues of Gal0LV0 or HV0LV0. Among these selected amino acid residues, at least one amino acid is modified into an amino acid residue(s) found in the mouse antibody KM2160 so that VH and VL of human CDR-grafted antibody having various modifications were designed.

First, regarding the VH, for example, Gal1 represented by SEQ ID NO:13 in which Ala at position 97 of Gal0 was modified, Gal2 represented by SEQ ID NO:14 in which Gly at position 42 and Gly at position 44 of Gal0 were modified, Gal3 represented by SEQ ID NO:15 in which Ala at position 97, Gly at position 42 and Gly at position 44 of Gal0 were modified, HV1 represented by SEQ ID NO:16 in which Thr at position 28 of HV0 was modified, HV2 represented by SEQ ID NO:17 in which Ala at position 97 of HV0 was modified, and HV3 represented by SEQ ID NO:18 in which Thr at position 28 and Ala at position 97 of HV0 were modified were designed. Furthermore, regarding the VL, for example, LV1 represented by SEQ ID NO:19 in which Ile at position 2 was modified, LV2 represented by SEQ ID NO:20 in which Val at position 3 was modified, and LV3 represented by SEQ ID NO:21 in which Ile at position 2 and Val at position 3 were modified were designed. Nucleotide sequences encoding the amino acid sequences represented by SEQ ID NOs:13 to 21 are represented by SEQ ID NOs:27 to 35, respectively.

### 2. Construction of cDNA encoding anti-CCR4 CDR-grafted antibody

### (1) Construction of cDNA encoding VH of anti-CCR4 CDR-grafted antibody

cDNA encoding the amino acid sequence GalO of VH of the anti-CCR4 CDR-grafted antibody designed in the item 1(1) of Reference Example 1 was constructed by PCR as follows.

First, a full length antibody amino acid sequence was prepared by ligating the designed amino acid sequence with the H chain secretory signal sequence of anti-CCR4 mouse antibody KM2160 represented by SEQ ID NO:36 (WO01/67754). Next, the amino acid sequence was converted into genetic codons. When two or more genetic codons are present for one amino acid residue, a corresponding genetic codon was determined by taking into consideration the codon usage found in nucleotide sequences of antibody genes (*Sequences of Proteins of Immunological Interest*). A nucleotide sequence of cDNA encoding the amino acid sequence of complete antibody V region was designed by ligating the determined genetic codons, and binding nucleotide sequences of primers for PCR amplification (including restriction enzyme recognition sequences for cloning into a vector for humanized antibody expression) were added to its 5'-terminal and 3'-terminal. The designed nucleotide sequence was divided into a total of 6 nucleotide sequences each having about 100 nucleotides counting from the 5'-terminal (adjoining nucleotide sequences are designed such that they have a complementary sequence of about 20 nucleotides on their terminal), and 6 synthetic oligonucleotides of SEQ ID NOs:37 to 42 were synthesized in reciprocal orders of a sense chain and an antisense chain (manufactured by GENSET).

PCR was carried out by adding each oligonucleotide to a reaction solution containing 0.2 mM dNTPs and 1 mM magnesium chloride to give a final concentration of 0.1 µM, and adjusting the total volume to 50 µl by using 0.4 µM M13 primer RV (manufactured by Takara Shuzo), 0.4 µM M13 primer M3 (manufactured by GENSET) and 2.5 units of KOD polymerase (manufactured by TOYOBO). The reaction was carried out by 30 cycles, each cycle consisting of 94°C for 30 seconds, 55°C for 30 seconds and 74°C for 60 seconds, and then 1 cycle at 74°C for 10 minutes. The reaction solution was purified by using QIA quick PCR purification kit (manufactured by QIAGEN) and finally dissolved in sterile water. The reaction solution was allowed to react at 37°C for 1 hour by using 10 units of a restriction enzyme *Apa*I (manufactured by Takara Shuzo) and 10 units of a restriction enzyme *Not*I (manufactured by Takara Shuzo). The reaction solution was fractionated by agarose gel electrophoresis, and an *Apa*I-*Not*I fragment of about 0.47 kb was recovered.

Next, 3 µg of plasmid pBluescript II SK(-) (manufactured by Stratagene) was allowed to react with the fragment by using 10 units of a restriction enzyme *Apa*I (manufactured by Takara Shuzo) and 10 units of a restriction enzyme *Not*I (manufactured by Takara Shuzo) at 37°C for 1 hour. the reaction solution was fractionated by agarose gel electrophoresis, and an *Apa*I-*Not*I fragment of about 2.95 kb was recovered.

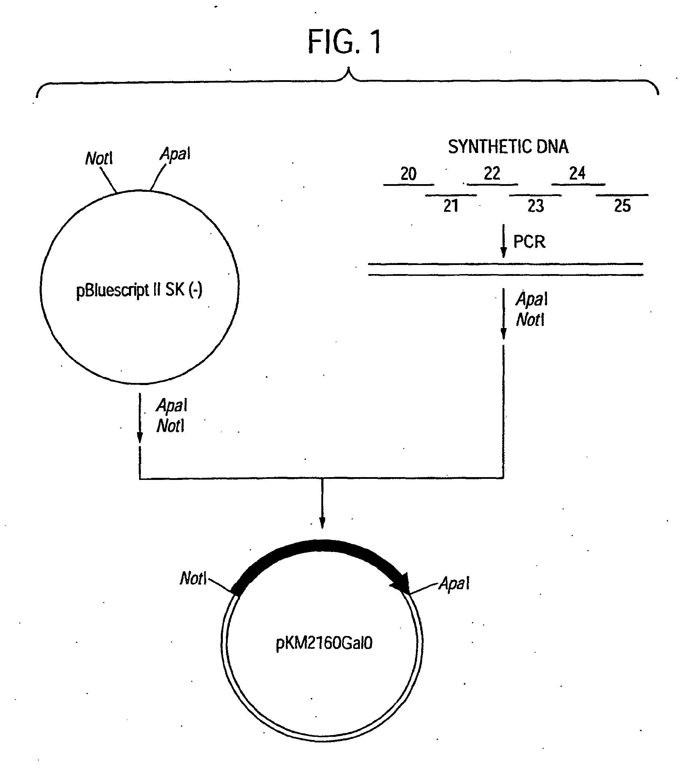

Next, the resulting *Apa*I-*Not*I fragment of the PCR product of VH of the anti-CCR4 CDR-grafted antibody and the *Apa*I-*Not*I fragment of plasmid pBluescript II SK(-) were ligated by using Solution I of DNA Ligation Kit Ver. 2 (manufactured by Takara Shuzo) according to the manufacture's instructions. *Escherichia coli* DH5α (manufactured by TOYOBO) was transformed with the recombinant plasmid DNA solution obtained in this manner, each plasmid DNA was prepared from the transformant clones and the nucleotide sequences were analyzed by using Big Dye Terminator Kit ver. 2 (manufactured by Applied Biosystems). As a result of the nucleotide sequence analysis, a plasmid pKM2160Gal0 shown in Fig. 1 having the objective nucleotide sequence was obtained. *Escherichia coli* transformed with pKM2160Gal0, *Escherichia coli* DH5α/pKM2160Gal0, has been deposited on August 22, 2001, as FERM BP-7709 in International Patent Organism Depositary, National Institute of Advanced Industrial Science and Technology (Tsukuba Central 6, 1-1, Higashi 1-Chome Tsukuba-shi, Ibaraki-ken, Japan).

Next, the FR amino acid residues designed in the item 1 (3) of Reference Example 1 was modified as follows. The genetic codons for amino acid residues after the modification were modified to have genetic codons found in the mouse antibody KM2160.

In modification of Ala at position 97 to Gly, the PCR was carried out by using 25 ng of the plasmid pKM2160Ga10 prepared in this item as the template, first heating at 94°C for 2 minutes and then carrying out 35 cycles of the reaction, each cycle consisting of 94°C for 15 seconds, 55°C for 30 seconds and 68°C for 40 seconds, in 50 µl of a reaction system prepared by adding each of the synthetic DNAs for mutagenesis comprising the nucleotide sequences represented by SEQ IDs:43 and 44 (manufactured by GENSET) as primers to give a final concentration of 0.4 µM and using 2.5 units of KOD plus polymerase (manufactured by TOYOBO) according to the manufacture's instructions. The reaction solution was purified by using QIA quick PCR purification kit (manufactured by QIAGEN) and finally dissolved in sterile water. The total volume was allowed to react for 1 hour at 37°C by using 10 units of a restriction enzyme *Pst*I (manufactured by Takara Shuzo) and then allowed to react for 1 hour at 37°C by using 10 units of a restriction enzyme *Dra*III (manufactured by New England Biolabs). The reaction solution was fractionated by agarose gel electrophoresis, and a *Pst*I-*Dra*III fragment of about 0.58 kb was recovered.

Next, 3 µg of the plasmid pKM2160Gal0 was allowed to react at 37°C for 1 hour by using 10 units of a restriction enzyme *Pst*I (manufactured by Takara Shuzo) and then to react at 37°C for 1 hour by using 10 units of a restriction enzyme *Dra*III (manufactured by New England Biolabs). The reaction solution was fractionated by agarose gel electrophoresis, and a *Pst*I-*Dra*III fragment of about 2.7 kb was recovered.

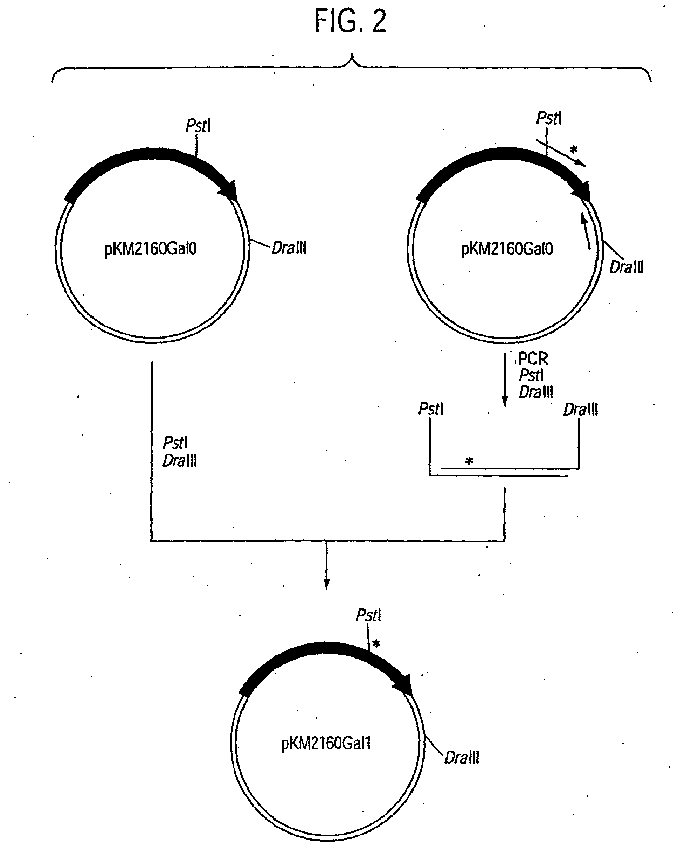

Next, the thus obtained *Pst*I-*Dra*III fragment derived from the PCR product and the *Pst*I-*Dra*III fragment derived from the plasmid pKM2160Gal0 were ligated by using Solution I of DNA Ligation Kit Ver. 2 (manufactured by Takara Shuzo) according to the manufacture's instructions. *Escherichia coli* DH5α (manufactured by TOYOBO) was transformed with the recombinant plasmid DNA solution obtained in this manner, each plasmid DNA was prepared from the transformant clones and the nucleotide sequences were analyzed by using Big Dye Terminator Kit ver. 2 (manufactured by Applied Biosystems). As a result of the nucleotide sequence analysis, a plasmid pKM2160Gal1 shown in Fig. 2 having the objective nucleotide sequence was obtained. *Escherichia coli* transformed with the plasmid pKM2160Gal1, *Escherichia coli* DH5α/pKM2160Gal1, has been deposited on August 22, 2001, as FERM BP-7710 in International Patent Organism Depositary, National Institute of Advanced Industrial Science and Technology (Tsukuba Central 6, 1-1, Higashi 1-Chome Tsukuba-shi, Ibaraki-ken, Japan).

In modifications of Gly at position 42 to Asp and Gly at position 44 into Arg, a plasmid pKM2160Gal2 was obtained by carrying out the method basically similar to the above, except that the synthetic DNA for mutagenesis comprising the nucleotide sequence represented by SEQ ID NO:45 (manufactured by GENSET) and M13 primer RV (manufactured by Takara Shuzo) were used as the PCR primers. *Escherichia coli* transformed with the plasmid pKM2160Gal2, *Escherichia coli* DH5α/pKM2160Gal2, has been deposited on August 22, 2001, as FERM BP-7711 in International Patent Organism Depositary, National Institute of Advanced Industrial Science and Technology (Tsukuba Central 6, 1-1, Higashi 1-Chome Tsukuba-shi, Ibaraki-ken, Japan).

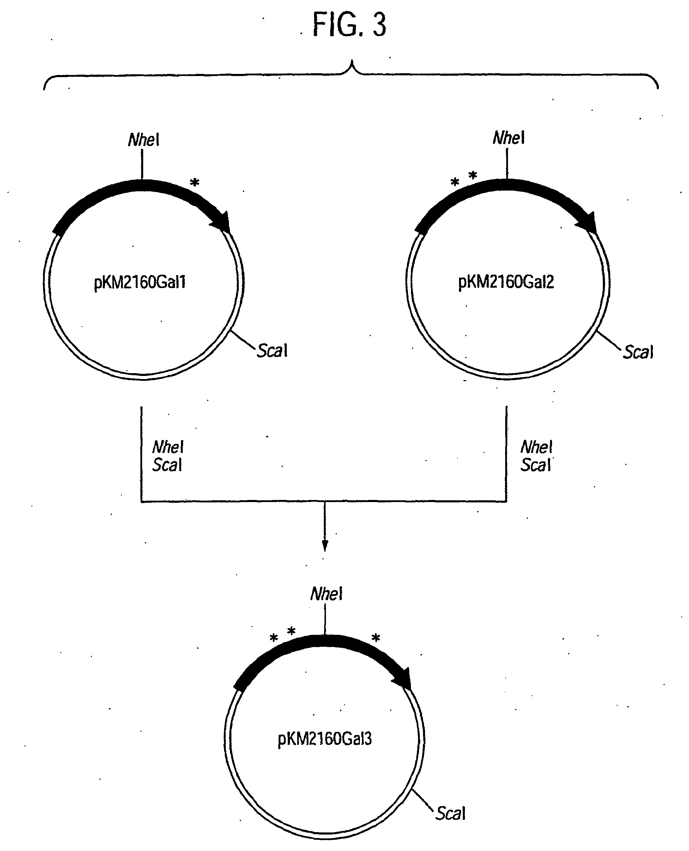

Also, modification of all of the above three residues was constructed as follows. About 0.5 µg of each of the pKM2160Gal1 and pKM2160Gal2 obtained in the above was allowed to react at 37°C for 1 hour by using 10 units of *Nhe*I (manufactured by Takara Shuzo) and then allowed to react at 37°C for 1 hour by using *Sca*I (manufactured by Takara Shuzo). The reaction solution was fractionated by agarose gel electrophoresis, and a *Nhe*I-*Sca*I fragment of about 1.3 kb derived from pKM2160Gal1 and a fragment of about 2.0 kb derived from pKM2160Gal2 were recovered. The resulting two fragments were ligated by using Solution I of DNA Ligation Kit Ver. 2 (manufactured by Takara Shuzo) according to the manufacture's instructions. *Escherichia coli* DH5α (manufactured by TOYOBO) was transformed with the recombinant plasmid DNA solution obtained in this manner, each plasmid DNA was prepared from the transformant clones and nucleotide sequences were analyzed by using Big Dye Terminator Kit ver. 2 (manufactured by Applied Biosystems). As a result of the nucleotide sequence analysis, a plasmid pKM2160Gal3 shown in Fig. 3 having the objective nucleotide sequence was obtained. *Escherichia coli* transformed with the plasmid pKM2160Gal3, *Escherichia coli* DH5α/pKM2160Gal3, has been deposited on August 22, 2001, as FERM BP-7712 in International Patent Organism Depositary, National Institute of Advanced Industrial Science and Technology (Tsukuba Central 6, 1-1, Higashi 1-Chome Tsukuba-shi, Ibaraki-ken, Japan).

Next, a cDNA encoding the amino acid sequence HV0 of VH of the anti-CCR4 CDR-grafted antibody designed in the item 1(1) of Reference Example 1 was constructed by PCR as follows.

First, a complete antibody amino acid sequence was prepared by ligating the designed amino acid sequence with the H chain secretory signal sequence of anti-CCR4 mouse antibody KM2160 represented by SEQ ID NO:36. Next, the amino acid sequence was converted into genetic codons. When two or more genetic codons are present for one amino acid residue, a corresponding genetic codon was determined by taking into consideration the codon usage found in nucleotide sequences of antibody genes (*Sequences of Proteins of Immunological Interest*). The determined genetic codons were ligated so that a nucleotide sequence of cDNA encoding the amino acid sequence of complete antibody V region was designed, and binding nucleotide sequences of primers for PCR amplification (including restriction enzyme recognition sequences for cloning into a vector for humanized antibody expression use) were added to its 5'-terminal and 3'-terminal. The designed nucleotide sequence was divided into a total of 6 nucleotide sequences each having about 100 nucleotides counting from the 5'-terminal (adjoining nucleotide sequences are designed such that they have a duplication sequence of about 20 nucleotides on their termini) and 6 synthetic oligonucleotides of SEQ ID NOs:37, 46 to 49 and 42 were synthesized in reciprocal orders of a sense chain and an antisense chain (manufactured by GENSET), and then pKM2160HV0 was obtained by the method similar to pKM2160Gal0 described in this item. *Escherichia coli* transformed with the plasmid pKM2160HV0, *Escherichia coli* DH5α/pKM2160HV0, has been deposited on August 27, 2001, as FERM BP-7718 in International Patent Organism Depositary, National Institute of Advanced Industrial Science and Technology (Tsukuba Central 6, 1-1, Higashi 1-Chome Tsukuba-shi, Ibaraki-ken, Japan).

In modification of Thr at position 28 to Ile, pKM2160HV1 having the objective nucleotide sequence was obtained by carrying out the reaction similar to the construction of the above plasmid pKM2160HV0, using an oligonucleotide having the nucleotide sequence represented by SEQ ID NO:50 instead of the oligonucleotide having the nucleotide sequence represented by SEQ ID NO:46, and using an oligonucleotide having the nucleotide sequence represented by SEQ ID NO:51 instead of the oligonucleotide having the nucleotide sequence represented by SEQ ID NO:47. *Escherichia coli* transformed with the plasmid pKM2160HV1, *Escherichia coli* DH5α/pKM2160HV1, has been deposited on August 27, 2001, as FERM BP-7719 in International Patent Organism Depositary, National Institute of Advanced Industrial Science and Technology (Tsukuba Central 6, 1-1, Higashi 1-Chome Tsukuba-shi, Ibaraki-ken, Japan).

In modification of Thr at position 28 to Ile and Ala at position 97 to Gly, pKM2160HV3 having the objective nucleotide sequence was obtained by the reaction similar to the construction of the above plasmid pKM2160HV0, using an oligonucleotide having the nucleotide sequence represented by SEQ ID NO:50 instead of the oligonucleotide having the nucleotide sequence represented by SEQ ID NO:46, using an oligonucleotide having the nucleotide sequence represented by SEQ ID NO:51 instead of the oligonucleotide having the nucleotide sequence represented by SEQ ID NO:47 and using an oligonucleotide having the nucleotide sequence represented by SEQ ID NO:52 instead of the oligonucleotide having the nucleotide sequence represented by SEQ ID NO:49. *Escherichia coli* transformed with the plasmid pKM2160HV3, *Escherichia coli* DH5α/pKM2160HV3, has been deposited on August 27, 2001, as FERM BP-7721 in International Patent Organism Depositary, National Institute of Advanced Industrial Science and Technology (Tsukuba Central 6, 1-1, Higashi 1-Chome Tsukuba-shi, Ibaraki-ken, Japan).

In modification of Ala at position 97 to Gly, the plasmid was constructed as follows. About 0.5 µg of each of the obtained pKM2160HV0 and pKM2160HV3 was allowed to react at 37°C for 1 hour by using 10 units of a restriction enzyme *Nhe*I (manufactured by Takara Shuzo) and then allowed to react at 37°C for 1 hour by using *Sca*I (manufactured by Takara Shuzo). The reaction solution was fractionated by agarose gel electrophoresis, and a *Nhe*I-*Sca*I fragment of about 1.3 kb derived from pKM2160HV3 and a fragment of about 2.0 kb derived from pKM2160HV0 were recovered. The resulting two fragments were ligated by using Solution I of DNA Ligation Kit Ver. 2 (manufactured by Takara Shuzo) according to the manufacture's instructions. *Escherichia coli* DH5α (manufactured by TOYOBO) was transformed with the recombinant plasmid DNA solution obtained in this manner, each plasmid DNA was prepared from the transformant clones and nucleotide sequences were analyzed by using Big Dye Terminator Kit ver. 2 (manufactured by Applied Biosystems). As a result of the nucleotide sequence analysis, a plasmid pKM2160HV2 having the objective nucleotide sequence was obtained. *Escherichia coli* transformed with the plasmid pKM2160HV2, *Escherichia coli* DH5α/pKM2160HV2, has been deposited on August 27, 2001, as FERM BP-7720 in International Patent Organism Depositary, National Institute of Advanced Industrial Science and Technology (Tsukuba Central 6, 1-1, Higashi 1-Chome Tsukuba-shi, Ibaraki-ken, Japan).

### (2) Construction of cDNA encoding VL of anti-CCR4 CDR-grafted antibody

A cDNA encoding the amino acid sequence LV0 of VL of the anti-CCR4 CDR-grafted antibody designed in the item 1(2) of Reference Example 1 was constructed using PCR similar to the case of VH as follows. In this case, the L chain sequence of anti-CCR4 mouse antibody KM2160 having the amino acid sequence represented by SEQ ID NO:25 was used as the secretory signal sequence (WO01/64754).

First, 6 synthetic oligonucleotides having the nucleotide sequences described in SEQ ID NOs:54 to 59 were synthesized (manufactured by GENSET). PCR was carried out by adding each oligonucleotide to 50 µl of a reaction solution to give a final concentration of 0.1 µM, and using 0.4 µM of M13 primer RV (manufactured by Takara Shuzo) and 0.4 µM of M13 primer M4 (manufactured by Takara Shuzo) or M13 primer M3 (manufactured by GENSET) represented by SEQ ID NO:60 and 2.5 units of KOD polymerase (manufactured by TOYOBO). The reaction was carried out by 30 cycles, each cycle consisting of 94°C for 30 seconds, 55°C for 30 seconds and 74°C for 60 seconds, and subsequent 1 cycle at 72°C for 10 minutes. The reaction solution was purified by using QIA quick PCR purification kit (manufactured by QIAGEN) and finally dissolved in sterile water. The reaction solution was allowed to react by using 10 units of a restriction enzyme EcoRI (manufactured by Takara Shuzo) and 10 units of a restriction enzyme *Xho*I (manufactured by Takara Shuzo) at 37°C for 1 hour. The reaction solution was fractionated by agarose gel electrophoresis, and an *Eco*RI-*Xho*I fragment of about 0.44 kb was recovered.

Next, 3 µg of the plasmid pBluescript II SK(-) (manufactured by Stratagene) was allowed to react by using 15 units of a restriction enzyme *Eco*RI (manufactured by Takara Shuzo) and 15 units of a restriction enzyme *Xho*I (manufactured by Takara Shuzo) at 37°C for 1 hour. The reaction solution was fractionated by agarose gel electrophoresis, and an *Eco*RI*-Xho*I fragment of about 2.95 kb was recovered.

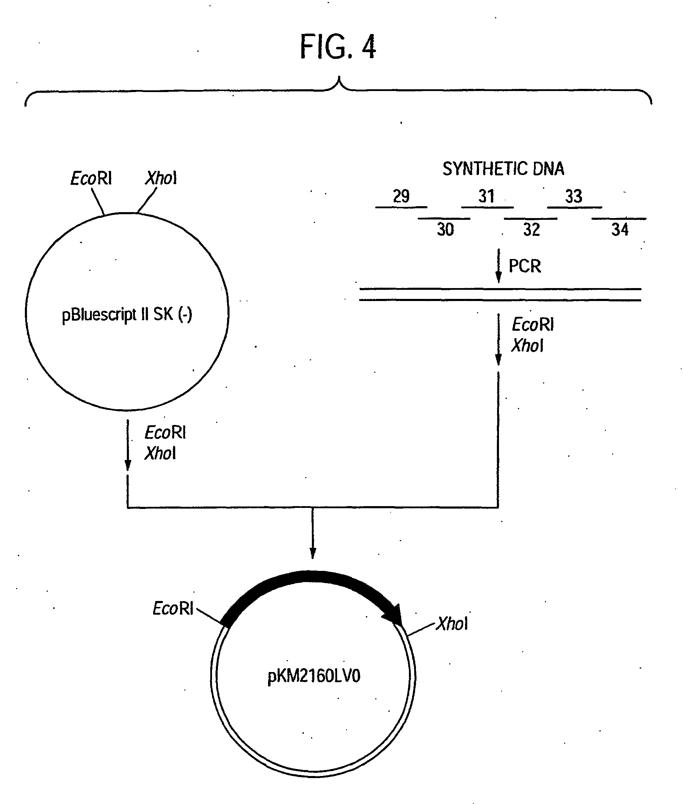

Next, the resulting *Eco*RI-*Xho*I fragment of the PCR product of VL of the anti-CCR4 CDR-grafted antibody and the *Eco*RI-*Xho*I fragment of plasmid pBluescript II SK(-) were ligated by using Solution I of DNA Ligation Kit Ver. 2 (manufactured by Takara Shuzo) according to the manufacture's instructions. *Escherichia coli* DH5α (manufactured by TOYOBO) was transformed with the recombinant plasmid DNA solution obtained in this manner, each plasmid DNA was prepared from the transformant clones and nucleotide sequences were analyzed by using Big Dye Terminator Kit ver. 2 (manufactured by Applied Biosystems). As a result of the nucleotide sequence analysis, a plasmid pKM2160LV0 shown in Fig. 4 having the objective nucleotide sequence was obtained. *Escherichia coli* transformed with plasmid pKM2160LV0, *Escherichia coli* DH5α/pKM2160LV0, has been deposited on August 22, 2001, as FERM BP-7713 in International Patent Organism Depositary, National Institute of Advanced Industrial Science and Technology (Tsukuba Central 6, 1-1, Higashi 1-Chome Tsukuba-shi, Ibaraki-ken, Japan).

Next, the FR amino acid residues designed in the item 1(3) of Reference Example 1 were modified as follows. The genetic codons for amino acid residues after the modification were modified to have genetic codons found in the mouse antibody KM2160.

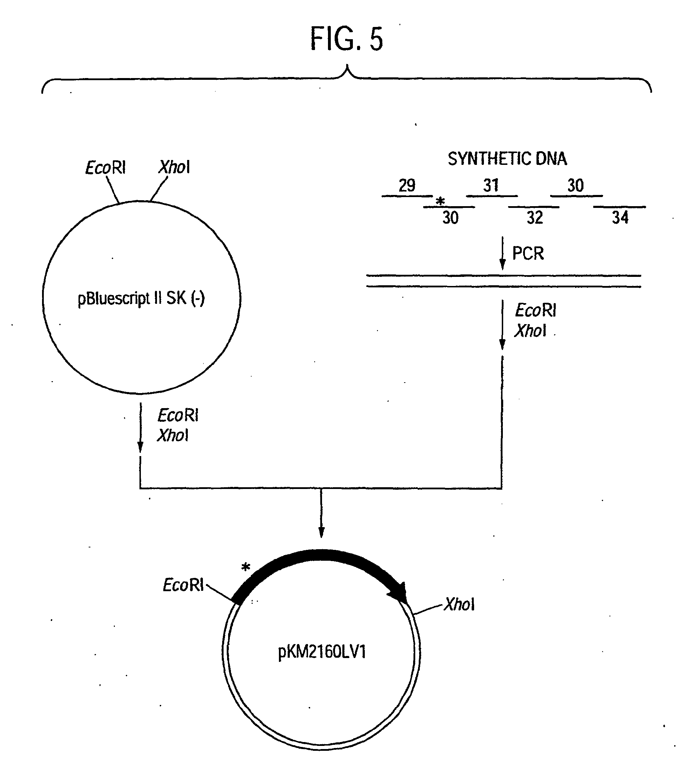

In modification of Ile at position 2 to Val, a plasmid pKM2160LV1 shown in Fig. 5 having the objective nucleotide sequence was obtained by carrying out the reaction similar to the construction of the above plasmid pKM2160LV0, using an oligonucleotide having the nucleotide sequence represented by SEQ ID NO:61 instead of the oligonucleotide having the nucleotide sequence represented by SEQ ID NO:50. *Escherichia coli* transformed with the plasmid pKM2160LV1, *Escherichia coli* DH5α/pKM2160LV1, has been deposited on August 22, 2001, as FERM BP-7714 in International Patent Organism Depositary, National Institute of Advanced Industrial Science and Technology (Tsukuba Central 6, 1-1, Higashi 1-Chome Tsukuba-shi, Ibaraki-ken, Japan).

In the same manner, each of the objective plasmids pKM2160LV2 and pKM2160LV3 was obtained by using an oligonucleotide having the nucleotide sequence represented by SEQ ID NO:62 instead of the oligonucleotide having the nucleotide sequence represented by SEQ ID NO:55 when Val at position 3 was modified into Leu, and using an oligonucleotide having the nucleotide sequence represented by SEQ ID NO:63 instead of the oligonucleotide having the nucleotide sequence represented by SEQ ID NO:56 when both of the above two residues were modified. *Escherichia coli* transformed with the plasmid pKM2160LV2, *Escherichia coli* DH5α/pKM2160LV2, and *Escherichia coli* transformed with the plasmid pKM2160LV3, *Escherichia coli* DH5α/pKM2160LV3, have been deposited on August 22, 2001, as FERM BP-7715 and FERM BP-7716, respectively, in International Patent Organism Depositary, National Institute of Advanced Industrial Science and Technology (Tsukuba Central 6, 1-1, Higashi 1-Chome Tsukuba-shi, Ibaraki-ken, Japan).

### (3) Construction of anti-CCR4 CDR-grafted antibody expression vector

An anti-CCR4 CDR-grafted antibody expression vector pKANTEX2160Gal0LV0 was constructed by using a vector for humanized antibody expression pKANTEX93 (*Mol. Immunol*., 37, 1035 (2000)) and the plasmids pKM2160Ga10 and pKM2160LV0 obtained in the items 2(1) and (2) of Reference Example 1 as follows.

The plasmid pKM2160LV0 (3 µg) obtained in the item 2(2) of Reference Example 1 was allowed to react with 10 units of a restriction enzyme *Bsi*WI (manufactured by New England Biolabs) at 55°C for 1 hour and then with 10 units of a restriction enzyme *Eco*RI (manufactured by Takara Shuzo) at 37°C for 1 hour. The reaction solution was fractionated by agarose gel electrophoresis, and a *Bsi*WI-*Eco*RI fragment of about 0.44 kb was recovered.

Next, 3 µg of the vector for humanized antibody expression pKANTEX93 was allowed to react with 10 units of a restriction enzyme *Bsi*WI (manufactured by New England Biolabs) at 55°C for 1 hour and then with 10 units of a restriction enzyme *Eco*RI (manufactured by Takara Shuzo) at 37°C for 1 hour. The reaction solution was fractionated by agarose gel electrophoresis, and a *Bsi*WI-*Eco*RI fragment of about 12.75 kb was recovered.

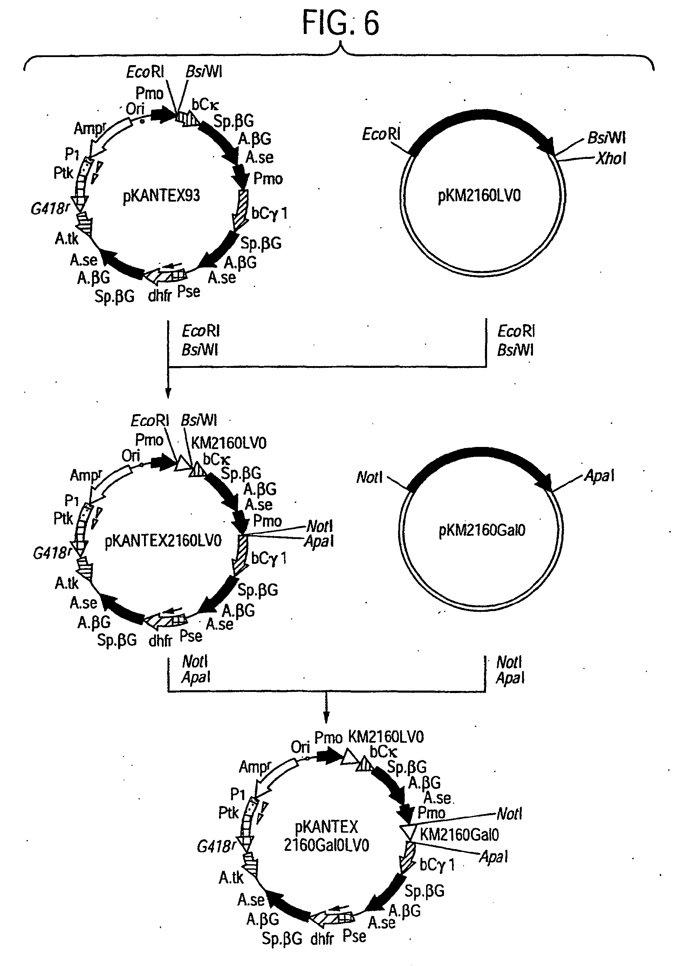

Next, the resulting *Bsi*WI-*Eco*RI fragment derived from pKM2160LV0 and the *Bsi*WI-*Eco*RI fragment derived from pKANTEX93 were ligated by using Solution I of DNA Ligation Kit Ver. 2 (manufactured by Takara Shuzo) according to the manufacture's instructions. *Escherichia coli* DH5α (manufactured by TOYOBO) was transformed with the recombinant plasmid DNA solution obtained in this manner to thereby obtain a plasmid pKANTEX2160LV0 shown in Fig. 6.

Next, 3 µg of the plasmid pKM2160Gal0 obtained in the item 2(1) of Reference Example 1 was allowed to react with 10 units of a restriction enzyme *Apa*I (manufactured by Takara Shuzo) at 37°C for 1 hour and then with 10 units of a restriction enzyme *Not*I (manufactured by Takara Shuzo) at 37°C for 1 hour. The reaction solution was fractionated by agarose gel electrophoresis, and an *Apa*I-*Not*I fragment of about 0.47 kb was recovered.

Next, 3 µg of the plasmid pKANTEX2160LV0 obtained in the above was allowed to react with 10 units of a restriction enzyme *Apa*I (manufactured by Takara Shuzo) at 37°C for 1 hour and then with 10 units of a restriction enzyme *Not*I (manufactured by Takara Shuzo) at 37°C for 1 hour. The reaction solution was fractionated by agarose gel electrophoresis, and an *Apa*I-*Not*I fragment of about 0.45 kb was recovered.

Next, the resulting *Apa*I-*Not*I fragment derived from the pKM2160Ga10 and the *Apa*I-*Not*I fragment derived from the plasmid pKANTEX2160LV0 were ligated by using Solution I of DNA Ligation Kit Ver. 2 (manufactured by Takara Shuzo) according to the manufacture's instructions. *Escherichia coli* DH5α (manufactured by TOYOBO) was transformed with the recombinant plasmid DNA solution obtained in this manner, and each plasmid DNA was prepared from the transformant clones.

As a result that the nucleotide sequences of the thus obtained plasmids were analyzed by using Big Dye Terminator Kit ver. 2 (manufactured by Applied Biosystems), it was confirmed that a plasmid shown in Fig. 6 into which the objective DNA had been cloned was obtained.

In addition, expression vectors were prepared by using the same method on the VH and VL in which amino acid residues of other FR were modified, including HV0.

Specifically, 22 expression vectors, Gal0LV0, Gal0LV1, Gal0LV2, Gal0LV3, Gal1LV1, Gal1LV3, Gal2LV1, Gal2LV3, Gal3LV1, Gal3LV3, HV0LV0, HV0LV1, HV0LV2, HV0LV3, HV1LV0, HV1LV1, HV1LV2, HV1LV3, HV2LV0, HV2LV3, HV3LV0 and HV3LV3 were constructed by respectively combining Gal0, Gall, Gal2, Gal3, HV0, HV1, HV2 and HV3 constructed in the item 2(1) of Reference Example 1 with LV0, LV1, LV2 and LV3 constructed in the item 2(2) of Reference Example 1.

### Reference Example 2

### Expression of anti-CCR4 CDR-grafted antibody in animal cells:

### 1. Transient expression of anti-CCR4 CDR-grafted antibody using COS-7 cell (ATCC CRL 1651)

### (1) Transient expression at COS-7 cell

Into a 6 well plate (manufactured by Iwaki Glass), 1×10⁵ cells/ml of COS-7 cell was dispensed at 2 ml/well by using DMEM medium (manufactured by Bibco) containing 10% FCS and cultured overnight at 37°C. Per 100 µl of OPTI-MEM medium (manufactured by Bibco), 3 µl of Fu-GENETM 6 Transfer Reagent (manufactured by Roche) was added and 1 µg of anti-CCR4 CDR-grafted antibody expression vectors obtained in the item 2(3) of Reference Example 1 was further added thereto, and the mixture was allowed to stand at room temperature for 15 minutes to form a DNA-liposome complex. Each of the reaction solutions was added dropwise to the above COS-7 cell and thoroughly mixed, followed by culturing at 37°C. After the culturing for 72 hours, the culture supernatants were recovered and the activity of the anti-CCR4 CDR-grafted antibody activity in the culture supernatants was evaluated.

### (2) Reactivity evaluation of anti-CCR4 CDR-grafted antibody for human CCR4

The activity of the resulting culture supernatants was evaluated as follows.

Compound 1 (SEQ ID NO:23) was selected as a human CCR4 extracellular region peptide which can react with the anti-CCR4 chimeric antibody KM2760 produced by the transformant KM2760 (FERM BP-7054) prepared in Example 2 in WO01/64754. In order to use Compound 1 in the activity measurement by ELISA, its conjugate with BSA (bovine serum albumin) (manufactured by Nakalai Tesque) was prepared and used as the antigen. That is, 100 ml of 25 mg/ml SMCC (4-(N-maleimidomethyl)cyclohexane-1-carboxylic acid N-hydroxysuccinimide ester) (manufactured by Sigma)-DMSO solution was added dropwise to 900 ml of PBS solution containing 10 mg BSA under vortex and gently stirred for 30 minutes. To a gel filtration column, such as NAP-10 column, equilibrated with 25 ml of PBS, 1 ml of the reaction solution was applied, and the eluate eluted with 1.5 ml of PBS was used as a BSA-SMCC solution (BSA concentration was calculated by A₂₈₀ measurement). Next, 250 ml of PBS was added to 0.5 mg of Compound 1 which was then completely dissolved by adding 250 ml of DMF, and then the above BSA-SMCC solution (BSA content: 1.25 mg) was added under vortex and gently stirred for 3 hours. The reaction solution was dialyzed overnight at 4°C against PBS, sodium azide was added thereto to give a final concentration of 0.05% and then the resulting mixture was filtered by using a 0.22 mm filter to give a BSA-Compound 1 solution.

Into a 96 well ELISA plate (manufactured by Greiner), 0.05 µg/ml of the prepared conjugate was dispensed in at 50 µl/well and allowed to stand at 4°C overnight for adsorption. After washing with PBS, PBS containing 1% BSA (hereinafter referred to as "1% BSA-PBS") was added thereto at 100 µl/well and allowed to react at room temperature for 1 hour to block the remaining active groups. After washing each well with PBS containing 0.05% Tween 20 (hereinafter referred to as "Tween-PBS"), a culture supernatant of the transformant was added at 50 µl/well and allowed to react at room temperature for 1 hour. After the reaction and subsequent washing of each well with Tween-PBS, a peroxidase-labeled goat anti-human IgG(γ) antibody solution (manufactured by American Qualex) diluted 6,000 folds with 1% BSA-PBS was added as a secondary antibody solution at 50 µl/well and allowed to react at room temperature for 1 hour. After the reaction and subsequent washing with Tween-PBS, an ABTS substrate solution (a solution prepared by dissolving 0.55 g of 2,2'-azino-bis(3-ethylbenzothiazoline-6-sulfonic acid) ammonium in 1 liter of 0.1 M citrate buffer (pH 4.2) and adding 1 µl/ml hydrogen peroxide just before use) was added at 50 µl/well to develop color, and the reaction was stopped 20 minutes thereafter by adding 5% SDS solution at 50 µl/well. Thereafter, absorbance at 415 nm was measured.

Also, in order to compare concentrations of a produced human IgG antibody in the culture supernatants, a goat anti-human IgG(γ) antibody (manufactured by American Qualex) diluted 2,000 folds with PBS was used as the antigen.

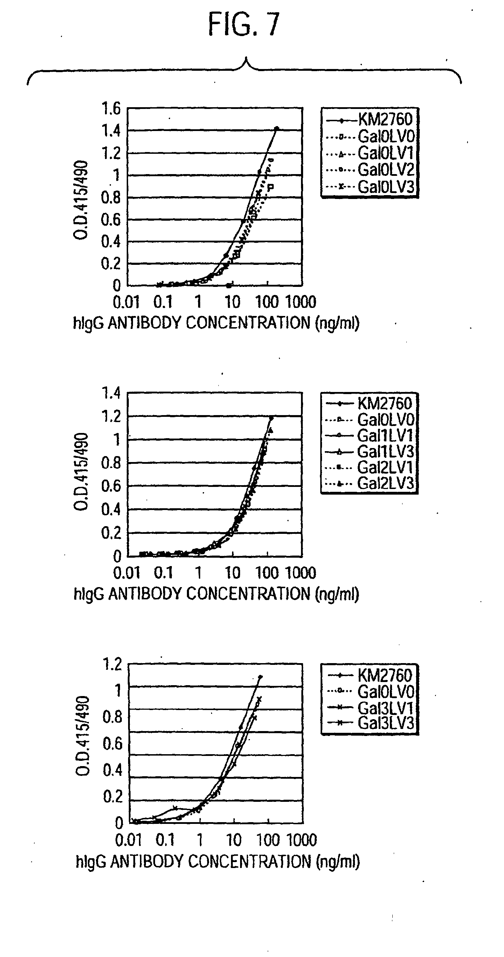

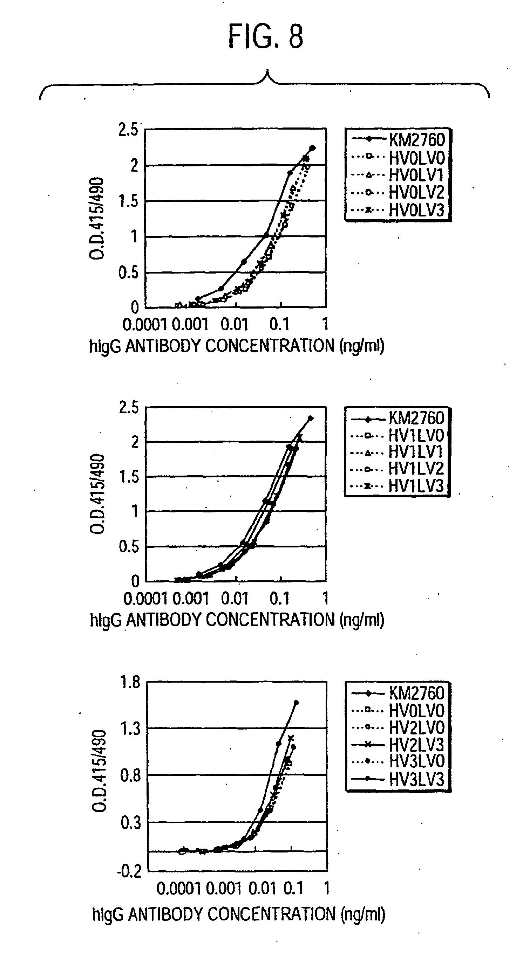

The results are shown in Fig. 7 and Fig. 8. Each human CCR4 CDR-grafted antibody showed almost the same activity of the human chimeric antibody KM2760.

### 2. Stable expression of anti-CCR4 CDR-grafted antibody using animal cells

An anti-CCR4 CDR-grafted antibody was expressed in animal cells by using the anti-CCR4 CDR-grafted antibody expression vector obtained in the item 2(3) of Reference Example 1 as follows.

### (1) Stable expression in rat myeloma cell line YB2/0 cell (ATCC CRL 1581)

Each humanized antibody expression plasmid was made into a linear state by digesting it with a restriction enzyme *Aat*II (manufactured by TOYOBO), 10 µg of the digested product was introduced into 4×10⁶ cells of the rat myeloma cell line YB2/0 cell (ATCC CRL 1581) by electroporation (*Cytotechnology,* 3, 133 (1990)), and then the cells were suspended in 40 ml of H-SFM (manufactured by GIBCO-BRL) medium (supplemented with 5% of fetal bovine serum (FBS)). After culturing at 37°C for 1 to 3 days in a 5% CO₂ incubator, G418 (manufactured by Nakalai Tesque) was added thereto to give a concentration of 1 mg/ml and the culturing was continued for 1 to 2 weeks to obtain G418-resistant transformants.

### (2) Purification of anti-CCR4 CDR-grafted antibody from culture supernatant

When a transformant showing G418 resistance appeared and became confluent, the medium was changed to 300 to 1,100 ml of H-SFM medium containing Daigo's GF21 (manufactured by Wako Pure Chemical Industries) at a concentration of 5%, followed by culturing for 3 to 5 days. When it became confluent, the culture supernatant was recovered. A purified protein was obtained by purifying the anti-CCR4 CDR-grafted antibody from about 300 to 1,100 ml of the culture supernatant by using Prosep-A column (manufactured by Millipore) in accordance with the attached instructions.

### 3. Activity evaluation of purified anti-CCR4 CDR-grafted antibody

### (1) Measurement of binding activity of anti-CCR4 CDR-grafted antibody for human CCR4 (ELISA)

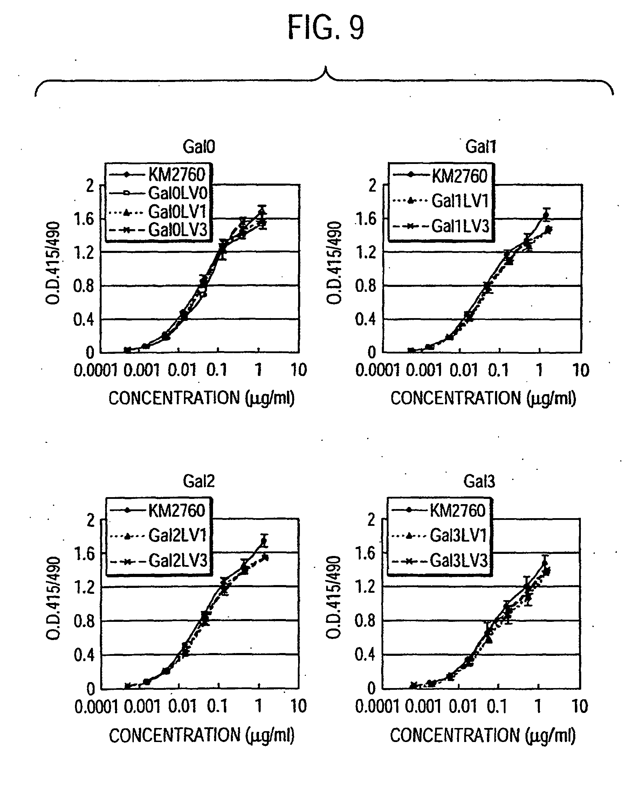

The measurement was carried out in the same manner as the method described in the item 1(2) of Reference Example 2. The results are shown in Fig. 9. Each anti-CCR4 CDR-grafted antibody showed almost the same activity of that of the human chimeric antibody KM2760.

### (2) Reactivity of anti-CCR4 CDR-grafted antibody with human CCR4-high-expressing cell (immunofluorescent method)

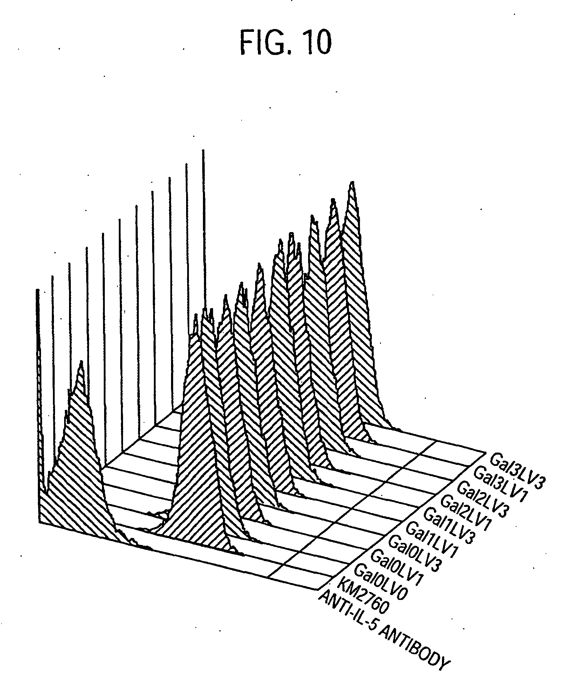

Into a 96 well plate, 2×10⁵ or more of CCR4/EL-4 cells, CCR4-high-expressing cells obtained in Reference Example 1, were dispensed. An antibody solution was prepared by diluting each of the purified antibodies and a human immunoglobulin (manufactured by Welfide) for preventing nonspecific staining to give concentrations of 10 µg/ml and 3.75 mg/ml, respectively, with a buffer for FACS (1% BSA-PBS, 0.02% EDTA, 0.05% NaN₃), and the antibody solution was added at 100 µl/well and allowed to react for 30 minutes in ice. As a negative control, an anti-human IL-5 receptor α chain antibody (WO97/10354) was reacted with the same concetration. After washing twice with 200 µl/well of the buffer for FACS, a PE-labeled anti-human IgG antibody (manufactured by Coulter) diluted 100 times was added at 50 µl/well. After the reaction in ice under shading and subsequent washing three times with 200 µl/well of the buffer for FACS, the reaction product was suspended in 500 µl of the buffer for FACS and the fluorescence intensity was measured by using a flow cytometer. The results are shown in Fig. 10. All of the anti-CCR4 CDR-grafted antibodies showed almost the same activity as the human chimeric antibody KM2760.

### (3) Measurement of activity of anti-CCR4 CDR-grafted antibody to bind to human CCR4 (BIAcore method)

In order to measure the binding activity in more detail, the binding activity of various purified antibodies was measured by using BIAcore 2000 (manufactured by BIACORE) as follows. In this case, HBS-EP (manufactured by BIACORE) was used as the buffer for diluting samples and for the measurement. First, 5 µl of 0.01 µg/ml solution of Compound 1 as a biotinylated CCR4 partial peptide was added to a sensor tip SA (manufactured by BIACORE) at a flow rate of 5 µl/minute and immobilized on the sensor tip.

To the prepared biotinylated compound 1-immobilized sensor tip, 20 µl of 4 µg/ml solution of each of the purified antibodies was added at a flow rate of 5 µl/minute, and after completion of the addition, the dissociation reaction was monitored for 4 minutes and then the sensor tip surface was regenerated by adding 5 µl of 10 mM HCl twice. In this way, a binding reaction curve (sensorgram) for Compound 1 was obtained.

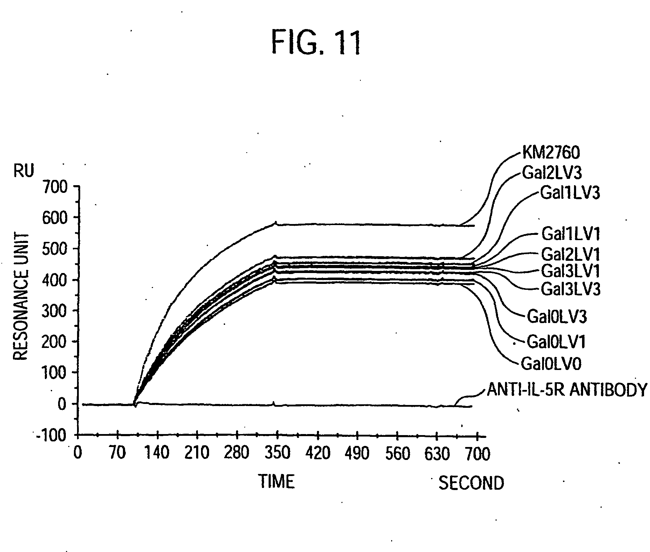

The results are shown in Fig. 11. The ordinate represents a resonance unit (RU) which means a mass change on the sensor tip. For example, 1,000 RU corresponds to a mass change of about 1 ng/mm² protein. It was shown that KM 2760 has a markedly stable and high binding activity, because it showed a time-dependent binding activity for Compound 1 and dissociation of its binding was hardly found by the dissociation reaction. On the other hand, each of the anti-CCR4 CDR-grafted antibodies showed almost the same dissociation reaction as the human chimeric antibody KM2760, but a slight decrease in the activity was found in the binding reaction to the CCR4 partial peptide, Compound 1. The anti-CCR4 CDR-grafted antibody Gal0LV0 in which CDR alone was grafted showed the lowest binding activity, and the binding activity was increased by modifying the amino acid residue of FR. The results show that an anti-CCR4 CDR-grafted antibody which maintains the antigen binding activity and binding specificity of a mouse antibody can be prepared by grafting CDR of the mouse antibody KM2160 to an appropriate human antibody FR, and that an anti-CCR4 CDR-grafted antibody having higher binding activity can be prepared by identifying FR amino acid residues important for the binding activity based on the three-dimensional structure and the like of antibody V regions and grafting them together with the CDR. It is expected that the anti-CCR4 CDR-grafted antibodies prepared in this Reference Example have high binding activity to CCR4, have decreased immunogenicity in human in comparison with that of mouse antibodies and also that of human chimeric antibodies, and have high safety and high therapeutic effects.

### INDUSTRIAL APPLICABILITY

The present invention provides a preventive agent, a diagnostic agent or a therapeutic agent for interstitial pneumonia, particularly a preventive agent, a diagnostic agent or a therapeutic agent for the interstitial pneumonia in usual idiopathic interstitial pneumonia and collagen disease, and a diagnostic agent for discriminating between usual idiopathic interstitial pneumonia and non-specific idiopathic interstitial pneumonia. Also, the present invention provides a method for discriminating between usual idiopathic interstitial pneumonia and non-specific idiopathic interstitial pneumonia.

### Free Text in Sequence Listing:

SEQUENCE LISTING

<110> KYOWA HAKKO KOGYO CO., LTD.

<120> Agent for diagnosing and treating interstitial pneumonia

<130> P044055

<150> JP 2002-54764
<151> 2002-02-28

<160> 63

<170> PatentIn Ver. 2.1

<210> 1
<211> 360
<212> PRT
<213> Homo sapiens

<400> 1
Met Asn Pro Thr Asp Ile Ala Asp Thr Thr Leu Asp Glu Ser Ile Tyr
  1               5                  10                  15

Ser Asn Tyr Tyr Leu Tyr Glu Ser Ile Pro Lys Pro Cys Thr Lys Glu
             20                  25                  30

Gly Ile Lys Ala Phe Gly Glu Leu Phe Leu Pro Pro Leu Tyr Ser Leu
         35                  40                  45

Val Phe Val Phe Gly Leu Leu Gly Asn Ser Val Val Val Leu Val Leu
     50                  55                  60

Phe Lys Tyr Lys Arg Leu Arg Ser Met Thr Asp Val Tyr Leu Leu Asn
 65                  70                  75                  80

Leu Ala Ile Ser Asp Leu Leu Phe Val Phe Ser Leu Pro Phe Trp Gly
                 85                  90                  95

Tyr Tyr Ala Ala Asp Gln Trp Val Phe Gly Leu Gly Leu Cys Lys Met
            100                 105                 110

Ile Ser Trp Met Tyr Leu Val Gly Phe Tyr Ser Gly Ile Phe Phe Val
        115                 120                 125

Met Leu Met Ser Ile Asp Arg Tyr Leu Ala Ile Val His Ala Val Phe
    130                 135                 140

Ser Leu Arg Ala Arg Thr Leu Thr Tyr Gly Val Ile Thr Ser Leu Ala
145                 150                 155                 160

Thr Trp Ser Val Ala Val Phe Ala Ser Leu Pro Gly Phe Leu Phe Ser
                165                 170                 175

Thr Cys Tyr Thr Glu Arg Asn His Thr Tyr Cys Lys Thr Lys Tyr Ser
            180                 185                 190

Leu Asn Ser Thr Thr Trp Lys Val Leu Ser Ser Leu Glu Ile Asn Ile
        195                 200                 205

Leu Gly Leu Val Ile Pro Leu Gly Ile Met Leu Phe Cys Tyr Ser Met
    210                 215                 220

Ile Ile Arg Thr Leu Gln His Cys Lys Asn Glu Lys Lys Asn Lys Ala
225                 230                 235                 240

Val Lys Met Ile Phe Ala Val Val Val Leu Phe Leu Gly Phe Trp Thr
                245                 250                 255

Pro Tyr Asn Ile Val Leu Phe Leu Glu Thr Leu Val Glu Leu Glu Val
            260                 265                 270

Leu Gln Asp Cys Thr Phe Glu Arg Tyr Leu Asp Tyr Ala Ile Gln Ala
        275                 280                 285

Thr Glu Thr Leu Ala Phe Val His Cys Cys Leu Asn Pro Ile Ile Tyr
    290                 295                 300

Phe Phe Leu Gly Glu Lys Phe Arg Lys Tyr Ile Leu Gln Leu Phe Lys
305                 310                 315                 320

Thr Cys Arg Gly Leu Phe Val Leu Cys Gln Tyr Cys Gly Leu Leu Gln
                325                 330                 335

Ile Tyr Ser Ala Asp Thr Pro Ser Ser Ser Tyr Thr Gln Ser Thr Met
            340                 345                 350

Asp His Asp Leu His Asp Ala Leu
        355

<210> 2
<211> 5
<212> PRT
<213> Mus musculus

<400> 2
Asn Tyr Gly Met Ser
1               5

<210> 3
<211> 17
<212> PRT
<213> Mus musculus

<400> 3
Thr Ile Ser Ser Ala Ser Thr Tyr Ser Tyr Tyr Pro Asp Ser Val Lys
1               5                   10                  15

Gly

<210> 4
<211> 10
<212> PRT
<213> Mus musculus

<400> 4
His Ser Asp Gly Asn Phe Ala Phe Gly Tyr
1               5                   10

<210> 5
<211> 16
<212> PRT
<213> Mus musculus

<400> 5
Arg Ser Ser Arg Asn Ile Val His Ile Asn Gly Asp Thr Tyr Leu Glu
1               5                   10                  15

<210> 6
<211> 7
<212> PRT
<213> Mus musculus

<400> 6
Lys Val Ser Asn Arg Phe Ser
1               5

<210> 7
<211> 9
<212> PRT
<213> Mus musculus

<400> 7
Phe Gln Gly Ser Leu Leu Phe Trp Thr
1               5

<210> 8
<211> 119
<212> PRT
<213> Mus musculus

<400> 8
Glu Val Gln Leu Val Glu Ser Gly Gly Asp Leu Met Lys Pro Gly Gly
1               5                   10                  15

Ser Leu Lys Ile Ser Cys Ala Ala Ser Gly Phe Ile Phe Ser Asn Tyr
            20                  25                  30

Gly Met Ser Trp Val Arg Gln Thr Pro Asp Met Arg Leu Glu Trp Val
        35                  40                  45

Ala Thr Ile Ser Ser Ala Ser Thr Tyr Ser Tyr Tyr Pro Asp Ser Val
    50                  55                  65

Lys Gly Arg Phe Thr Ile Ser Arg Asp Asn Ala Glu Asn Ser Leu Tyr
70                  75                  80                  85

Leu Gln Met Asn Ser Leu Arg Ser Glu Asp Thr Gly Ile Tyr Tyr Cys
                85                  90                  95

Gly Arg His Ser Asp Gly Asn Phe Ala Phe Gly Tyr Trp Gly Arg Gly
            100                 105                 110

Thr Leu Val Thr Val Ser Ala
        115

<210> 9
<211> 113
<212> PRT
<213> Mus mus ulus

<400> 9
Asp Val Leu Met Thr Gln Thr Pro Leu Ser Leu Pro Val Ser Leu Gly
1               5                   10                  15

Asp Gln Ala Ser Ile Ser Cys Arg Ser Ser Arg Asn Ile Val His Ile
            20                  25                  30

Asn Gly Asp Thr Tyr Leu Glu Trp Tyr Leu Gln Arg Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser Leu Leu Pro Trp Thr Phe Gly Gly Gly Thr Arg Leu Glu Ile Arg
            100                 105                 110

Arg

<210> 10
<211> 119
<212> PRT
<213> Artifi ial Se uen e

<220>
<223> Des ription of Artifi ial Se uen e Syntheti Peptide

<400> 10
Glu Val Gln Leu Val Glu Ser Gly Gly Asp Leu Val Gln Pro Gly Arg
1               5                   10                  15

Ser Leu Arg Leu Ser Cys Ala Ala Ser Gly Phe Ile Phe Ser Asn Tyr
            20                  25                  30

Gly Met Ser Trp Val Arg Gln Ala Pro Gly Lys Gly Leu Glu Trp Val
        35                  40                  45

Ala Thr Ile Ser Ser Ala Ser Thr Tyr Ser Tyr Tyr Pro Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Val Glu Asp Thr Ala Leu Tyr Tyr Cys
                85                  90                  95

Ala Arg His Ser Asp Gly Asn Phe Ala Phe Gly Tyr Trp Gly Gln Gly
            100                 105                 110

Thr Leu Val Thr Val Ser Ser
        115


<210> 11
<211> 119
<212> PRT
<213> Artifi ial Se uen e

<220>
<223> Des ription of Artifi ial Se uen e  Syntheti  Peptide

<400> 11
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Gly Met Ser Trp Val Arg Gln Ala Pro Gly Lys Gly Leu Glu Trp Val
        35                  40                  45

Ser Thr Ile Ser Ser Ala Ser Thr Tyr Ser Tyr Tyr Pro Asp Ser Val
50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Ser Asp Gly Asn Phe Ala Phe Gly Tyr Trp Gly Gln Gly
            100                 105                 110

Thr Leu Val Thr Val Ser Ser
        115

<210> 12
<211> 112
<212> PRT
<213> Artifi ial Se uen e

<220>
<223> Des ription of Artifi ial Se uen e  Syntheti  Peptide

<400> 12
Asp Ile Val Met Thr Gln Ser Pro Leu Ser Leu Pro Val Thr Pro Gly
1               5                   10                  15

Glu Pro Ala Ser Ile Ser Cys Arg Ser Ser Arg Asn Ile Val His Ile
            20                  25                  30

Asn Gly Asp Thr Tyr Leu Glu Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Gly
                85                  90                  95

Ser Leu Leu Pro Trp Thr Phe Gly Gln Gly Thr Lys Val Glu Ile Lys
            100                 105                 110

<210> 13
<211> 119
<212> PRT
<213> Artifi ial Se uen e

<220>
<223> Des ription of Artifi ial Se uen e  Syntheti  Peptide

<400> 13
Glu Val Gln Leu Val Glu Ser Gly Gly Asp Leu Val Gln Pro Gly Arg
 1               5                  10                  15

Ser Leu Arg Leu Ser Cys Ala Ala Ser Gly Phe Ile Phe Ser Asn Tyr
            20                  25                  30

Gly Met Ser Trp Val Arg Gln Ala Pro Gly Lys Gly Leu Glu Trp Val
        35                  40                  45

Ala Thr Ile Ser Ser Ala Ser Thr Tyr Ser Tyr Tyr Pro Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Val Glu Asp Thr Ala Leu Tyr Tyr Cys
                85                  90                  95

Gly Arg His Ser Asp Gly Asn Phe Ala Phe Gly Tyr Trp Gly Gln Gly
            100                 105                 110

Thr Leu Val Thr Val Ser Ser
        115

<210> 14
<211> 119
<212> PRT
<213> Artifi ial Se uen e

<220>

<223> Des ription of Artifi ial Se uen e  Syntheti  Peptide

<400> 14
Glu Val Gln Leu Val Glu Ser Gly Gly Asp Leu Val Gln Pro Gly Arg
1               5                   10                  15

Ser Leu Arg Leu Ser Cys Ala Ala Ser Gly Phe Ile Phe Ser Asn Tyr
            20                  25                  30

Gly Met Ser Trp Val Arg Gln Ala Pro Asp Lys Arg Leu Glu Trp Val
        35                  40                  45

Ala Thr Ile Ser Ser Ala Ser Thr Tyr Ser Tyr Tyr Pro Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Val Glu Asp Thr Ala Leu Tyr Tyr Cys
                85                  90                  95

Ala Arg His Ser Asp Gly Asn Phe Ala Phe Gly Tyr Trp Gly Gln Gly
            100                 105                 110

Thr Leu Val Thr Val Ser Ser
        115


<210> 15
<211> 119
<212> PRT
<213> Artifi ial Se uen e

<220>
<223> Des ription of Artifi ial Se uen e  Syntheti  Peptide

<400> 15
Glu Val Gln Leu Val Glu Ser Gly Gly Asp Leu Val Gln Pro Gly Arg
1               5                   10                  15

Ser Leu Arg Leu Ser Cys Ala Ala Ser Gly Phe Ile Phe Ser Asn Tyr
            20                  25                  30

Gly Met Ser Trp Val Arg Gln Ala Pro Asp Lys Arg Leu Glu Trp Val

        35                  40                  45

Ala Thr Ile Ser Ser Ala Ser Thr Tyr Ser Tyr Tyr Pro Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Val Glu Asp Thr Ala Leu Tyr Tyr Cys
                85                  90                  95

Gly Arg His Ser Asp Gly Asn Phe Ala Phe Gly Tyr Trp Gly Gln Gly
            100                 105                 110

Thr Leu Val Thr Val Ser Ser
        115

<210> 16
<211> 119
<212> PRT
<213> Artifi ial Se uen e

<220>
<223> Des ription of Artifi ial Se uen e Syntheti Peptide

<400> 16
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ile Phe Ser Asn Tyr
            20                  25                  30

Gly Met Ser Trp Val Arg Gln Ala Pro Gly Lys Gly Leu Glu Trp Val
        35                  40                  45

Ser Thr Ile Ser Ser Ala Ser Thr Tyr Ser Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Ser Asp Gly Asn Phe Ala Phe Gly Tyr Trp Gly Gln Gly
            100                 105                 110

Thr Leu Val Thr Val Ser Ser
        115

<210> 17
<211> 119
<212> PRT
<213> Artifi ial Se uen e

<220>
<223> Des ription of Artifi ial Se uen e  Syntheti  Peptide

<400> 17
Glu Val Gln Leu Val Glu Ser Gly Gly Gly Leu Val Gln Pro Gly Gly
  1               5                  10                  15

Ser Leu Arg Leu Ser Cys Ala Ala Ser Gly Phe Thr Phe Ser Asn Tyr
             20                  25                  30

Gly Met Ser Trp Val Arg Gln Ala Pro Gly Lys Gly Leu Glu Trp Val
         35                  40                  45

Ser Thr Ile Ser Ser Ala Ser Thr Tyr Ser Tyr Tyr Pro Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Gly Arg His Ser Asp Gly Asn Phe Ala Phe Gly Tyr Trp Gly Gln Gly
            100                 105                 110

Thr Leu Val Thr Val Ser Ser
        115

<210> 18
<211> 119

<212> PRT
<213> Artifi ial Se uen e

<220>
<223> Des ription of Artifi ial Se uen e  Syntheti  Peptide

<400> 18
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ile Phe Ser Asn Tyr
            20                  25                  30

Gly Met Ser Trp Val Arg Gln Ala Pro Gly Lys Gly Leu Glu Trp Val
        35                  40                  45

Ser Thr Ile Ser Ser Ala Ser Thr Tyr Ser Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Gly Arg His Ser Asp Gly Asn Phe Ala Phe Gly Tyr Trp Gly Gln Gly
            100                 105                 110

Thr Leu Val Thr Val Ser Ser
        115

<210> 19
<211> 112
<212> PRT
<213> Artifi ial Se uen e

<220>
<223> Des ription of Artifi ial Se uen e  Syntheti  Peptide

<400> 19
Asp Val Val Met Thr Gln Ser Pro Leu Ser Leu Pro Val Thr Pro Gly
1               5                   10                  15

Glu Pro Ala Ser Ile Ser Cys Arg Ser Ser Arg Asn Ile Val His Ile
            20                  25                  30

Asn Gly Asp Thr Tyr Leu Glu Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Gly
                85                  90                  95

Ser Leu Leu Pro Trp Thr Phe Gly Gln Gly Thr Lys Val Glu Ile Lys
            100                 105                 110


<210> 20
<211> 112
<212> PRT
<213> Artifi ial Se uen e

<220>
<223> Des ription of Artifi ial Se uen e  Syntheti  Peptide

<400> 20
Asp Ile Leu Met Thr Gln Ser Pro Leu Ser Leu Pro Val Thr Pro Gly
1               5                   10                  15

Glu Pro Ala Ser Ile Ser Cys Arg Ser Ser Arg Asn Ile Val His Ile
            20                  25                  30

Asn Gly Asp Thr Tyr Leu Glu Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Gly

85                  90                  95

Ser Leu Leu Pro Trp Thr Phe Gly Gln Gly Thr Lys Val Glu Ile Lys
            100                 105                 110


<210> 21
<211> 112
<212> PRT
<213> Artifi ial Se uen e

<220>
<223> Des ription of Artifi ial Se uen e  Syntheti  Peptide

<400> 21
Asp Val Leu Met Thr Gln Ser Pro Leu Ser Leu Pro Val Thr Pro Gly
 1               5                  10                  15

Glu Pro Ala Ser Ile Ser Cys Arg Ser Ser Arg Asn Ile Val His Ile
            20                  25                  30

Asn Gly Asp Thr Tyr Leu Glu Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Gly
                85                  90                  95

Ser Leu Leu Pro Trp Thr Phe Gly Gln Gly Thr Lys Val Glu Ile Lys
            100                 105                 110


<210> 22
<211> 28
<212> PRT
<213> Homo sapiens

<400> 22
Asn Pro Thr Asp Ile Ala Asp Thr Thr Leu Asp Glu Ser Ile Tyr Ser

1               5                   10                  15

Asn Tyr Tyr Leu Tyr Glu Ser Ile Pro Lys Pro Cys
            20                  25

<210> 23
<211> 18
<212> PRT
<213> Homo sapiens

<400> 23
Asp Glu Ser Ile Tyr Ser Asn Tyr Tyr Leu Tyr Glu Ser Ile Pro Lys
1               5                   10                  15

Pro Cys

<210> 24
<211> 357
<212> DNA
<213> Artificial Sequence

<220>
<221> CDS
<222> (1)..(357)
<223> Description of Artificial Sequence: Synthetic DNA

<400> 24
gag gtg cag ctg gtg gag tct ggg gga gac ttg gta cag cct ggg agg     48
Glu Val Gln Leu Val Glu Ser Gly Gly Asp Leu Val Gln Pro Gly Arg
1               5                   10                  15

tcc ctg aga ctc tcc tgt gca gcc tct gga ttc att ttc agt aat tat     96
Ser Leu Arg Leu Ser Cys Ala Ala Ser Gly Phe Ile Phe Ser Asn Tyr
            20                  25                  30

ggc atg tct tgg gtc cgc cag gct cca ggg aag ggg ctg gag tgg gtc     144
Gly Met Ser Trp Val Arg Gln Ala Pro Gly Lys Gly Leu Glu Trp Val
        35                  40                  45

gca acc att agt agt gct agc act tat tcc tat tat cca gac agt gtg     192
Ala Thr Ile Ser Ser Ala Ser Thr Tyr Ser Tyr Tyr Pro Asp Ser Val
    50                  55                  60

aag gga cga ttc acc atc tcc aga gac aat gcc aag aac tcc ctg tat 240
Lys Gly Arg Phe Thr Ile Ser Arg Asp Asn Ala Lys Asn Ser Leu Tyr
65                  70                  75                  80

ctg cag atg aac agc ctg aga gtc gag gac acg gcc ctg tat tac tgt 288
Leu Gln Met Asn Ser Leu Arg Val Glu Asp Thr Ala Leu Tyr Tyr Cys
                85                  90                  95

gcg aga cat agc gat gga aac ttc gcg ttt ggt tat tgg ggc cag gga 336
Ala Arg His Ser Asp Gly Asn Phe Ala Phe Gly Tyr Trp Gly Gln Gly
            100                 105                 110

acc ctg gtc acc gtc tcc tca 357
Thr Leu Val Thr Val Ser Ser
        115

<210> 25
<211> 357
<212> DNA
<213> Artificial Sequence

<220>
<221> CDS
<222> (1)..(357)
<223> Description of Artificial Sequence: Synthetic DNA

<400> 25
gag gtg cag ctg gtg gag tct ggg gga ggc ttg gta cag cct ggg ggg 48
Glu Val Gln Leu Val Glu Ser Gly Gly Gly Leu Val Gln Pro Gly Gly
1               5                   10                  15

tcc ctg aga ctc tcc tgt gca gcc tct gga ttc acc ttc agt aat tat 96
Ser Leu Arg Leu Ser Cys Ala Ala Ser Gly Phe Thr Phe Ser Asn Tyr
            20                  25                  30

ggc atg tct tgg gtc cgc cag gct cca ggg aag ggg ctg gag tgg gtc 144
Gly Met Ser Trp Val Arg Gln Ala Pro Gly Lys Gly Leu Glu Trp Val
        35                  40                  45

tca acc att agt agt gct agc act tat tcc tat tat cca gac agt gtg 192
Ser Thr Ile Ser Ser Ala Ser Thr Tyr Ser Tyr Tyr Pro Asp Ser Val
    50                  55                  60

aag gga cga ttc acc atc tcc aga gac aat tcc aag aac acg ctg tat     240
Lys Gly Arg Phe Thr Ile Ser Arg Asp Asn Ser Lys Asn Thr Leu Tyr
65                  70                  75                  80

ctg cag atg aac agc ctg aga gcc gag gac acg gcc gtg tat tac tgt     288
Leu Gln Met Asn Ser Leu Arg Ala Glu Asp Thr Ala Val Tyr Tyr Cys
                85                  90                  95

gcg aga cat agc gat gga aac ttc gcg ttt ggt tat tgg ggc cag gga     336
Ala Arg His Ser Asp Gly Asn Phe Ala Phe Gly Tyr Trp Gly Gln Gly
            100                 105                 110

acc ctg gtc acc gtc tcc tca                                         357
Thr Leu Val Thr Val Ser Ser
        115

<210> 26
<211> 336
<212> DNA
<213> Artificial Sequence

<220>
<221> CDS
<222> (1)..(336)
<223> Description of Artificial Sequence: Synthetic DNA

<400> 26
gat atc gtg atg act cag tct cca ctc tcc ctg ccc gtc acc cct gga     48
Asp Ile Val Met Thr Gln Ser Pro Leu Ser Leu Pro Val Thr Pro Gly
1               5                   10                  15

gag ccg gcc tcc atc tcc tgc aga tct agt cgg aac att gtt cat att     96
Glu Pro Ala Ser Ile Ser Cys Arg Ser Ser Arg Asn Ile Val His Ile
            20                  25                  30

aat ggt gac aca tat tta gaa tgg tac ctg cag aag cca ggc cag tct     144
Asn Gly Asp Thr Tyr Leu Glu Trp Tyr Leu Gln Lys Pro Gly Gln Ser
        35                  40                  45

cca cag ctc ctg atc tat aaa gtt tcc aac cga ttt tct ggg gtc cca     192
Pro Gln Leu Leu Ile Tyr Lys Val Ser Asn Arg Phe Ser Gly Val Pro
    50                  55                  60

gac agg ttc agt ggc agt ggg tca ggc aca gat ttc aca ctg aaa atc     240
Asp Arg Phe Ser Gly Ser Gly Ser Gly Thr Asp Phe Thr Leu Lys Ile
 65                  70                  75                  80

agc aga gtg gag gct gag gat gtt ggg gtt tat tac tgc ttt caa ggt     288
Ser Arg Val Glu Ala Glu Asp Val Gly Val Tyr Tyr Cys Phe Gln Gly
                 85                  90                  95

tca ctt ctt ccg tgg acg ttc ggc caa ggg acc aag gtg gaa atc aaa     336
Ser Leu Leu Pro Trp Thr Phe Gly Gln Gly Thr Lys Val Glu Ile Lys
            100                 105                 110

<210> 27
<211> 357
<212> DNA
<213> Artificial Sequence

<220>
<221> CDS
<222> (1)..(357)
<223> Description of Artificial Sequence: Synthetic DNA

<400> 27
gag gtg cag ctg gtg gag tct ggg gga gac ttg gta cag cct ggg agg     48
Glu Val Gln Leu Val Glu Ser Gly Gly Asp Leu Val Gln Pro Gly Arg
  1               5                  10                  15

tcc ctg aga ctc tcc tgt gca gcc tct gga ttc att ttc agt aat tat     96
Ser Leu Arg Leu Ser Cys Ala Ala Ser Gly Phe Ile Phe Ser Asn Tyr
             20                  25                  30

ggc atg tct tgg gtc cgc cag gct cca ggg aag ggg ctg gag tgg gtc     144
Gly Met Ser Trp Val Arg Gln Ala Pro Gly Lys Gly Leu Glu Trp Val
         35                  40                  45

gca acc att agt agt gct agc act tat tcc tat tat cca gac agt gtg     192
Ala Thr Ile Ser Ser Ala Ser Thr Tyr Ser Tyr Tyr Pro Asp Ser Val
     50                  55                  60

aag gga cga ttc acc atc tcc aga gac aat gcc aag aac tcc ctg tat     240
Lys Gly Arg Phe Thr Ile Ser Arg Asp Asn Ala Lys Asn Ser Leu Tyr
 65                  70                  75                  80

ctg cag atg aac agc ctg aga gtc gag gac acg gcc ctg tat tac tgt    288
Leu Gln Met Asn Ser Leu Arg Val Glu Asp Thr Ala Leu Tyr Tyr Cys
                85                  90                  95

gga aga cat agc gat gga aac ttc gcg ttt ggt tat tgg ggc cag gga    336
Gly Arg His Ser Asp Gly Asn Phe Ala Phe Gly Tyr Trp Gly Gln Gly
            100                 105                 110

acc ctg gtc acc gtc tcc tca                                        357
Thr Leu Val Thr Val Ser Ser
        115

<210> 28
<211> 357
<212> DNA
<213> Artificial Sequence

<220>
<221> CDS
<222> (1)..(357)
<223> Description of Artificial Sequence: Synthetic DNA

<400> 28
gag gtg cag ctg gtg gag tct ggg gga gac ttg gta cag cct ggg agg    48
Glu Val Gln Leu Val Glu Ser Gly Gly Asp Leu Val Gln Pro Gly Arg
  1               5                  10                  15

tcc ctg aga ctc tcc tgt gca gcc tct gga ttc att ttc agt aat tat    96
Ser Leu Arg Leu Ser Cys Ala Ala Ser Gly Phe Ile Phe Ser Asn Tyr
             20                  25                  30

ggc atg tct tgg gtc cgc cag gct cca gac aag agg ctg gag tgg gtc    144
Gly Met Ser Trp Val Arg Gln Ala Pro Asp Lys Arg Leu Glu Trp Val
         35                  40                  45

gca acc att agt agt gct agc act tat tcc tat tat cca gac agt gtg    192
Ala Thr Ile Ser Ser Ala Ser Thr Tyr Ser Tyr Tyr Pro Asp Ser Val
     50                  55                  60

aag gga cga ttc acc atc tcc aga gac aat gcc aag aac tcc ctg tat    240
Lys Gly Arg Phe Thr Ile Ser Arg Asp Asn Ala Lys Asn Ser Leu Tyr
 65                  70                  75                  80

ctg cag atg aac agc ctg aga gtc gag gac acg gcc ctg tat tac tgt    288
Leu Gln Met Asn Ser Leu Arg Val Glu Asp Thr Ala Leu Tyr Tyr Cys
                85                  90                  95

gcg aga cat agc gat gga aac ttc gcg ttt ggt tat tgg ggc cag gga    336
Ala Arg His Ser Asp Gly Asn Phe Ala Phe Gly Tyr Trp Gly Gln Gly
            100                 105                 110

acc ctg gtc acc gtc tcc tca                                        357
Thr Leu Val Thr Val Ser Ser
        115

<210> 29
<211> 357
<212> DNA
<213> Artificial Sequence

<220>
<221> CDS
<222> (1)..(357)
<223> Description of Artificial Sequence: Synthetic DNA

<400> 29
gag gtg cag ctg gtg gag tct ggg gga gac ttg gta cag cct ggg agg    48
Glu Val Gln Leu Val Glu Ser Gly Gly Asp Leu Val Gln Pro Gly Arg
 1               5                  10                  15

tcc ctg aga ctc tcc tgt gca gcc tct gga ttc att ttc agt aat tat    96
Ser Leu Arg Leu Ser Cys Ala Ala Ser Gly Phe Ile Phe Ser Asn Tyr
            20                  25                  30

ggc atg tct tgg gtc cgc cag gct cca gac aag agg ctg gag tgg gtc    144
Gly Met Ser Trp Val Arg Gln Ala Pro Asp Lys Arg Leu Glu Trp Val
        35                  40                  45

gca acc att agt agt gct agc act tat tcc tat tat cca gac agt gtg    192
Ala Thr Ile Ser Ser Ala Ser Thr Tyr Ser Tyr Tyr Pro Asp Ser Val
    50                  55                  60

aag gga cga ttc acc atc tcc aga gac aat gcc aag aac tcc ctg tat    240
Lys Gly Arg Phe Thr Ile Ser Arg Asp Asn Ala Lys Asn Ser Leu Tyr
65                  70                  75                  80

ctg cag atg aac agc ctg aga gtc gag gac acg gcc ctg tat tac tgt 288
Leu Gln Met Asn Ser Leu Arg Val Glu Asp Thr Ala Leu Tyr Tyr Cys
                85                  90                  95

gga aga cat agc gat gga aac ttc gcg ttt ggt tat tgg ggc cag gga 336
Gly Arg His Ser Asp Gly Asn Phe Ala Phe Gly Tyr Trp Gly Gln Gly
            100                 105                 110

acc ctg gtc acc gtc tcc tca 357
Thr Leu Val Thr Val Ser Ser
        115

<210> 30
<211> 336
<212> DNA
<213> Artificial Sequence

<220>
<221> CDS
<222> (1)..(336)
<223> Description of Artificial Sequence: Synthetic DNA

<400> 30
gat gtt gtg atg act cag tct cca ctc tcc ctg ccc gtc acc cct gga 48
Asp Val Val Met Thr Gln Ser Pro Leu Ser Leu Pro Val Thr Pro Gly
1               5                   10                  15

gag ccg gcc tcc atc tcc tgc aga tct agt cgg aac att gtt cat att 96
Glu Pro Ala Ser Ile Ser Cys Arg Ser Ser Arg Asn Ile Val His Ile
            20                  25                  30

aat ggt gac aca tat tta gaa tgg tac ctg cag aag cca ggc cag tct 144
Asn Gly Asp Thr Tyr Leu Glu Trp Tyr Leu Gln Lys Pro Gly Gln Ser
        35                  40                  45

cca cag ctc ctg atc tat aaa gtt tcc aac cga ttt tct ggg gtc cca 192
Pro Gln Leu Leu Ile Tyr Lys Val Ser Asn Arg Phe Ser Gly Val Pro
    50                  55                  60

gac agg ttc agt ggc agt ggg tca ggc aca gat ttc aca ctg aaa atc 240
Asp Arg Phe Ser Gly Ser Gly Ser Gly Thr Asp Phe Thr Leu Lys Ile
65                  70                  75                  80

agc aga gtg gag gct gag gat gtt ggg gtt tat tac tgc ttt caa ggt     288
Ser Arg Val Glu Ala Glu Asp Val Gly Val Tyr Tyr Cys Phe Gln Gly
                85                  90                  95

tca ctt ctt ccg tgg acg ttc ggc caa ggg acc aag gtg gaa atc aaa     336
Ser Leu Leu Pro Trp Thr Phe Gly Gln Gly Thr Lys Val Glu Ile Lys
            100                 105                 110

<210> 31
<211> 336
<212> DNA
<213> Artificial Sequence

<220>
<221> CDS
<222> (1)..(336)
<223> Description of Artificial Sequence: Synthetic DNA

<400> 31
gat atc ttg atg act cag tct cca ctc tcc ctg ccc gtc acc cct gga     48
Asp Ile Leu Met Thr Gln Ser Pro Leu Ser Leu Pro Val Thr Pro Gly
  1               5                  10                  15

gag ccg gcc tcc atc tcc tgc aga tct agt cgg aac att gtt cat att     96
Glu Pro Ala Ser Ile Ser Cys Arg Ser Ser Arg Asn Ile Val His Ile
            20                  25                  30

aat ggt gac aca tat tta gaa tgg tac ctg cag aag cca ggc cag tct     144
Asn Gly Asp Thr Tyr Leu Glu Trp Tyr Leu Gln Lys Pro Gly Gln Ser
        35                  40                  45

cca cag ctc ctg atc tat aaa gtt tcc aac cga ttt tct ggg gtc cca     192
Pro Gln Leu Leu Ile Tyr Lys Val Ser Asn Arg Phe Ser Gly Val Pro
    50                  55                  60

gac agg ttc agt ggc agt ggg tca ggc aca gat ttc aca ctg aaa atc     240
Asp Arg Phe Ser Gly Ser Gly Ser Gly Thr Asp Phe Thr Leu Lys Ile
 65                  70                  75                  80

agc aga gtg gag gct gag gat gtt ggg gtt tat tac tgc ttt caa ggt     288
Ser Arg Val Glu Ala Glu Asp Val Gly Val Tyr Tyr Cys Phe Gln Gly
                85                  90                  95

tca ctt ctt ccg tgg acg ttc ggc caa ggg acc aag gtg gaa atc aaa    336
Ser Leu Leu Pro Trp Thr Phe Gly Gln Gly Thr Lys Val Glu Ile Lys
            100                 105                 110

<210> 32
<211> 336
<212> DNA
<213> Artificial Sequence

<220>
<221> CDS
<222> (1)..(336)
<223> Description of Artificial Sequence: Synthetic DNA

<400> 32
gat gtt ttg atg act cag tct cca ctc tcc ctg ccc gtc acc cct gga    48
Asp Val Leu Met Thr Gln Ser Pro Leu Ser Leu Pro Val Thr Pro Gly
  1               5                  10                  15

gag ccg gcc tcc atc tcc tgc aga tct agt cgg aac att gtt cat att    96
Glu Pro Ala Ser Ile Ser Cys Arg Ser Ser Arg Asn Ile Val His Ile
             20                  25                  30

aat ggt gac aca tat tta gaa tgg tac ctg cag aag cca ggc cag tct    144
Asn Gly Asp Thr Tyr Leu Glu Trp Tyr Leu Gln Lys Pro Gly Gln Ser
         35                  40                  45

cca cag ctc ctg atc tat aaa gtt tcc aac cga ttt tct ggg gtc cca    192
Pro Gln Leu Leu Ile Tyr Lys Val Ser Asn Arg Phe Ser Gly Val Pro
     50                  55                  60

gac agg ttc agt ggc agt ggg tca ggc aca gat ttc aca ctg aaa atc    240
Asp Arg Phe Ser Gly Ser Gly Ser Gly Thr Asp Phe Thr Leu Lys Ile
 65                  70                  75                  80

agc aga gtg gag gct gag gat gtt ggg gtt tat tac tgc ttt caa ggt    288
Ser Arg Val Glu Ala Glu Asp Val Gly Val Tyr Tyr Cys Phe Gln Gly
                 85                  90                  95

tca ctt ctt ccg tgg acg ttc ggc caa ggg acc aag gtg gaa atc aaa    336
Ser Leu Leu Pro Trp Thr Phe Gly Gln Gly Thr Lys Val Glu Ile Lys
            100                 105                 110

<210> 33
<211> 357
<212> DNA
<213> Artificial Sequence

<220>
<221> CDS
<222> (1)..(357)
<223> Description of Artificial Sequence: Synthetic DNA

<400> 33
gag gtg cag ctg gtg gag tct ggg gga ggc ttg gta cag cct ggg ggg 48
Glu Val Gln Leu Val Glu Ser Gly Gly Gly Leu Val Gln Pro Gly Gly
1               5                   10                  15

tcc ctg aga ctc tcc tgt gca gcc tct gga ttc att ttc agt aat tat 96
Ser Leu Arg Leu Ser Cys Ala Ala Ser Gly Phe Ile Phe Ser Asn Tyr
            20                  25                  30

ggc atg tct tgg gtc cgc cag gct cca ggg aag ggg ctg gag tgg gtc 144
Gly Met Ser Trp Val Arg Gln Ala Pro Gly Lys Gly Leu Glu Trp Val
        35                  40                  45

tca acc att agt agt gct agc act tat tcc tat tat cca gac agt gtg 192
Ser Thr Ile Ser Ser Ala Ser Thr Tyr Ser Tyr Tyr Pro Asp Ser Val
    50                  55                  60

aag gga cga ttc acc atc tcc aga gac aat tcc aag aac acg ctg tat 240
Lys Gly Arg Phe Thr Ile Ser Arg Asp Asn Ser Lys Asn Thr Leu Tyr
65                  70                  75                  80

ctg cag atg aac agc ctg aga gcc gag gac acg gcc gtg tat tac tgt 288
Leu Gln Met Asn Ser Leu Arg Ala Glu Asp Thr Ala Val Tyr Tyr Cys
                85                  90                  95

gcg aga cat agc gat gga aac ttc gcg ttt ggt tat tgg ggc cag gga 336
Ala Arg His Ser Asp Gly Asn Phe Ala Phe Gly Tyr Trp Gly Gln Gly
            100                 105                 110

acc ctg gtc acc gtc tcc tca 357
Thr Leu Val Thr Val Ser Ser
        115

<210> 34
<211> 357
<212> DNA
<213> Artificial Sequence

<220>
<221> CDS
<222> (1)..(357)
<223> Description of Artificial Sequence: Synthetic DNA

<400> 34
gag gtg cag ctg gtg gag tct ggg gga ggc ttg gta cag cct ggg ggg 48
Glu Val Gln Leu Val Glu Ser Gly Gly Gly Leu Val Gln Pro Gly Gly
1               5                   10                  15

tcc ctg aga ctc tcc tgt gca gcc tct gga ttc acc ttc agt aat tat 96
Ser Leu Arg Leu Ser Cys Ala Ala Ser Gly Phe Thr Phe Ser Asn Tyr
            20                  25                  30

ggc atg tct tgg gtc cgc cag gct cca ggg aag ggg ctg gag tgg gtc 144
Gly Met Ser Trp Val Arg Gln Ala Pro Gly Lys Gly Leu Glu Trp Val
        35                  40                  45

tca acc att agt agt gct agc act tat tcc tat tat cca gac agt gtg 192
Ser Thr Ile Ser Ser Ala Ser Thr Tyr Ser Tyr Tyr Pro Asp Ser Val
    50                  55                  60

aag gga cga ttc acc atc tcc aga gac aat tcc aag aac acg ctg tat 240
Lys Gly Arg Phe Thr Ile Ser Arg Asp Asn Ser Lys Asn Thr Leu Tyr
65                  70                  75                  80

ctg cag atg aac agc ctg aga gcc gag gac acg gcc gtg tat tac tgt 288
Leu Gln Met Asn Ser Leu Arg Ala Glu Asp Thr Ala Val Tyr Tyr Cys
                85                  90                  95

gga aga cat agc gat gga aac ttc gcg ttt ggt tat tgg ggc cag gga 336
Gly Arg His Ser Asp Gly Asn Phe Ala Phe Gly Tyr Trp Gly Gln Gly
            100                 105                 110

acc ctg gtc acc gtc tcc tca 357
Thr Leu Val Thr Val Ser Ser
        115

<210> 35
<211> 357
<212> DNA
<213> Artificial Sequence

<220>
<221> CDS
<222> (1)..(357)
<223> Description of Artificial Sequence: Synthetic DNA

<400> 35
gag gtg cag ctg gtg gag tct ggg gga ggc ttg gta cag cct ggg ggg 48
Glu Val Gln Leu Val Glu Ser Gly Gly Gly Leu Val Gln Pro Gly Gly
1               5                   10                  15

tcc ctg aga ctc tcc tgt gca gcc tct gga ttc att ttc agt aat tat 96
Ser Leu Arg Leu Ser Cys Ala Ala Ser Gly Phe Ile Phe Ser Asn Tyr
            20                  25                  30

ggc atg tct tgg gtc cgc cag gct cca ggg aag ggg ctg gag tgg gtc 144
Gly Met Ser Trp Val Arg Gln Ala Pro Gly Lys Gly Leu Glu Trp Val
        35                  40                  45

tca acc att agt agt gct agc act tat tcc tat tat cca gac agt gtg 192
Ser Thr Ile Ser Ser Ala Ser Thr Tyr Ser Tyr Tyr Pro Asp Ser Val
    50                  55                  60

aag gga cga ttc acc atc tcc aga gac aat tcc aag aac acg ctg tat 240
Lys Gly Arg Phe Thr Ile Ser Arg Asp Asn Ser Lys Asn Thr Leu Tyr
65                  70                  75                  80

ctg cag atg aac agc ctg aga gcc gag gac acg gcc gtg tat tac tgt 288
Leu Gln Met Asn Ser Leu Arg Ala Glu Asp Thr Ala Val Tyr Tyr Cys
                85                  90                  95

gga aga cat agc gat gga aac ttc gcg ttt ggt tat tgg ggc cag gga 336
Gly Arg His Ser Asp Gly Asn Phe Ala Phe Gly Tyr Trp Gly Gln Gly
            100                 105                 110

acc ctg gtc acc gtc tcc tca 357
Thr Leu Val Thr Val Ser Ser

<210> 36
<211> 19
<212> PRT
<213> Mus musculus

<400> 36
Met Asn Leu Gly Leu Ser Leu Ile Phe Leu Ala Leu Ile Leu Lys Gly
1               5                   10                  15

Val Gln Cys


<210> 37
<211> 98
<212> DNA
<213> Artificial Sequence

<220>
<223> Description of Artificial Sequence: Synthetic DNA

<400> 37
aacagctatg accatggcgg ccgcgacccc tcaccatgaa cctcgggctc agtttgattt   60

tccttgccct cattttaaaa ggtgtccagt gtgaggtg                           98


<210> 38
<211> 97
<212> DNA
<213> Artificial Sequence

<220>
<223> Description of Artificial Sequence: Synthetic DNA

<400> 38
atgaatccag aggctgcaca ggagagtctc agggacctcc caggctgtac caagtctccc   60

ccagactcca ccagctgcac ctcacactgg acacctt                            97


<210> 39
<211> 98
<212> DNA

<213> Artificial Sequence

<220>
<223> Description of Artificial Sequence: Synthetic DNA

<400> 39
tgtgcagcct ctggattcat tttcagtaat tatggcatgt cttgggtccg ccaggctcca 60

gggaaggggc tggagtgggt cgcaaccatt agtagtgc 98


<210> 40
<211> 98
<212> DNA
<213> Artificial Sequence

<220>
<223> Description of Artificial Sequence: Synthetic DNA

<400> 40
acagggagtt cttggcattg tctctggaga tggtgaatcg tcccttcaca ctgtctggat 60

aataggaata agtgctagca ctactaatgg ttgcgacc 98


<210> 41
<211> 98
<212> DNA
<213> Artificial Sequence

<220>
<223> Description of Artificial Sequence: Synthetic DNA

<400> 41
acaatgccaa gaactccctg tatctgcaga tgaacagcct gagagtcgag gacacggccc 60

tgtattactg tgcgagacat agcgatggaa acttcgcg 98


<210> 42
<211> 96
<212> DNA
<213> Artificial Sequence

<220>
<223> Description of Artificial Sequence: Synthetic DNA

<400> 42
gtaaaacgac ggccagtggg cccttggtgg aggctgagga gacggtgacc agggttccct 60

ggccccaata accaaacgcg aagtttccat cgctat 96


<210> 43
<211> 17
<212> DNA
<213> Artificial Sequence

<220>
<223> Description of Artificial Sequence: Synthetic DNA

<400> 43
gatggttcac gtagtgg 17


<210> 44
<211> 61
<212> DNA
<213> Artificial Sequence

<220>
<223> Description of Artificial Sequence: Synthetic DNA

<400> 44
ctgtatctgc agatgaacag cctgagagtc gaggacacgg ccctgtatta ctgtggaaga 60

c 61


<210> 45
<211> 56
<212> DNA
<213> Artificial Sequence

<220>
<223> Description of Artificial Sequence: Synthetic DNA

<400> 45

gaataagtgc tagcactact aatggttgcg acccactcca gcctcttgtc tggagc 56

<210> 46
<211> 97
<212> DNA
<213> Artificial Sequence

<220>
<223> Description of Artificial Sequence: Synthetic DNA

<400> 46
gtgaatccag aggctgcaca ggagagtctc agggaccccc caggctgtac caagcctccc 60

ccagactcca ccagctgcac ctcacactgg acacctt 97

<210> 47
<211> 98
<212> DNA
<213> Artificial Sequence

<220>
<223> Description of Artificial Sequence: Synthetic DNA

<400> 47
tgtgcagcct ctggattcac cttcagtaat tatggcatgt cttgggtccg ccaggctcca 60

gggaaggggc tggagtgggt ctcaaccatt agtagtgc 98

<210> 48
<211> 98
<212> DNA
<213> Artificial Sequence

<220>
<223> Description of Artificial Sequence: Synthetic DNA

<400> 48
acagcgtgtt cttggaattg tctctggaga tggtgaatcg tcccttcaca ctgtctggat 60

aataggaata agtgctagca ctactaatgg ttgagacc 98

<210> 49
<211> 98
<212> DNA
<213> Artificial Sequence

<220>
<223> Description of Artificial Sequence: Synthetic DNA

<400> 49
acaattccaa gaacacgctg tatctgcaga tgaacagcct gagagccgag gacacggccg 60

tgtattactg tgcgagacat agcgatggaa acttcgcg 98


<210> 50
<211> 97
<212> DNA
<213> Artificial Sequence

<220>
<223> Description of Artificial Sequence: Synthetic DNA

<400> 50
atgaatccag aggctgcaca ggagagtctc agggaccccc caggctgtac caagcctccc 60

ccagactcca ccagctgcac ctcacactgg acacctt 97


<210> 51
<211> 98
<212> DNA
<213> Artificial Sequence

<220>
<223> Description of Artificial Sequence: Synthetic DNA

<400> 51
tgtgcagcct ctggattcat tttcagtaat tatggcatgt cttgggtccg ccaggctcca 60

gggaaggggc tggagtgggt ctcaaccatt agtagtgc 98


<210> 52

<211> 98
<212> DNA
<213> Artificial Sequence

<220>
<223> Description of Artificial Sequence: Synthetic DNA

<400> 52
acaattccaa gaacacgctg tatctgcaga tgaacagcct gagagccgag gacacggccg 60

tgtattactg tggaagacat agcgatggaa acttcgcg 98


<210> 53
<211> 19
<212> PRT
<213> Mus musculus

<400> 53
Met Lys Leu Pro Val Arg Leu Leu Val Leu Met Phe Trp Ile Pro Ala
1               5                   10                  15

Ser Ser Ser


<210> 54
<211> 92
<212> DNA
<213> Artificial Sequence

<220>
<223> Description of Artificial Sequence: Synthetic DNA

<400> 54
aacagctatg accatggaat tcgcctcttc aaaatgaagt tgcctgttag gctgttggtg 60

ctgatgttct ggattcctgc ttccagcagt ga 92


<210> 55
<211> 94
<212> DNA
<213> Artificial Sequence

<220>
<223> Description of Artificial Sequence: Synthetic DNA

<400> 55
tagatctgca ggagatggag gccggctctc caggggtgac gggcagggag agtggagact 60

gagtcatcac gatatcactg ctggaagcag gaat 94


<210> 56
<211> 92
<212> DNA
<213> Artificial Sequence

<220>
<223> Description of Artificial Sequence: Synthetic DNA

<400> 56
ctccatctcc tgcagatcta gtcggaacat tgttcatatt aatggtgaca catatttaga 60

atggtacctg cagaagccag gccagtctcc ac 92


<210> 57
<211> 93
<212> DNA
<213> Artificial Sequence

<220>
<223> Description of Artificial Sequence: Synthetic DNA

<400> 57
tgtgcctgac ccactgccac tgaacctgtc tgggacccca gaaaatcggt tggaaacttt 60

atagatcagg agctgtggag actggcctgg ctt 93


<210> 58
<211> 92
<212> DNA
<213> Artificial Sequence

<220>
<223> Description of Artificial Sequence: Synthetic DNA

<400> 58
tggcagtggg tcaggcacag atttcacact gaaaatcagc agagtggagg ctgaggatgt 60

tggggtttat tactgctttc aaggttcact tc 92


<210> 59
<211> 91
<212> DNA
<213> Artificial Sequence

<220>
<223> Description of Artificial Sequence: Synthetic DNA

<400> 59
gtaaaacgac ggccagtctc gagcgtacgt ttgatttcca ccttggtccc ttggccgaac 60

gtccacggaa gaagtgaacc ttgaaagcag t 91


<210> 60
<211> 17
<212> DNA
<213> Artificial Sequence

<220>
<223> Description of Artificial Sequence: Synthetic DNA

<400> 60
gtaaaacgac ggccagt 17


<210> 61
<211> 94
<212> DNA
<213> Artificial Sequence

<220>
<223> Description of Artificial Sequence: Synthetic DNA

<400> 61
tagatctgca ggagatggag gccggctctc caggggtgac gggcagggag agtggagact 60

gagtcatcac aacatcactg ctggaagcag gaat 94

<210> 62
<211> 94
<212> DNA
<213> Artificial Sequence

<220>
<223> Description of Artificial Sequence: Synthetic DNA

<400> 62
tagatctgca ggagatggag gccggctctc caggggtgac gggcagggag agtggagact 60

gagtcatcaa gatatcactg ctggaagcag gaat 94

<210> 63
<211> 94
<212> DNA
<213> Artificial Sequence

<220>
<223> Description of Artificial Sequence: Synthetic DNA

<400> 63
tagatctgca ggagatggag gccggctctc caggggtgac gggcagggag agtggagact 60

gagtcatcaa aacatcactg ctggaagcag gaat 94

SEQ ID NO:10-Explanation of artificial sequence: Synthetic peptide
SEQ ID NO:11-Explanation of artificial sequence: Synthetic peptide
SEQ ID NO:12-Explanation of artificial sequence: Synthetic peptide
SEQ ID NO:13-Explanation of artificial sequence: Synthetic peptide
SEQ ID NO:14-Explanation of artificial sequence: Synthetic peptide
SEQ ID NO:15-Explanation of artificial sequence: Synthetic peptide
SEQ ID NO:16-Explanation of artificial sequence: Synthetic peptide
SEQ ID NO:17-Explanation of artificial sequence: Synthetic peptide
SEQ ID NO:18-Explanation of artificial sequence: Synthetic peptide
SEQ ID NO:19-Explanation of artificial sequence: Synthetic peptide
SEQ ID NO:20-Explanation of artificial sequence: Synthetic peptide
SEQ ID NO:21-Explanation of artificial sequence: Synthetic peptide
SEQ ID NO:24-Explanation of artificial sequence: Synthetic DNA
SEQ ID NO:25-Explanation of artificial sequence: Synthetic DNA
SEQ ID NO:26-Explanation of artificial sequence: Synthetic DNA
SEQ ID NO:27-Explanation of artificial sequence: Synthetic DNA
SEQ ID NO:28-Explanation of artificial sequence: Synthetic DNA
SEQ ID NO:29-Explanation of artificial sequence: Synthetic DNA
SEQ ID NO:30-Explanation of artificial sequence: Synthetic DNA
SEQ ID NO:31-Explanation of artificial sequence: Synthetic DNA
SEQ ID NO:32-Explanation of artificial sequence: Synthetic DNA
SEQ ID NO:33-Explanation of artificial sequence: Synthetic DNA
SEQ ID NO:34-Explanation of artificial sequence: Synthetic DNA
SEQ ID NO:35-Explanation of artificial sequence: Synthetic DNA
SEQ ID NO:37-Explanation of artificial sequence: Synthetic DNA
SEQ ID NO:38-Explanation of artificial sequence: Synthetic DNA
SEQ ID NO:39-Explanation of artificial sequence: Synthetic DNA

SEQ ID NO:40-Explanation of artificial sequence: Synthetic DNA
SEQ ID NO:41-Explanation of artificial sequence: Synthetic DNA
SEQ ID NO:42-Explanation of artificial sequence: Synthetic DNA
SEQ ID NO:43-Explanation of artificial sequence: Synthetic DNA
SEQ ID NO:44-Explanation of artificial sequence: Synthetic DNA
SEQ ID NO:45-Explanation of artificial sequence: Synthetic DNA
SEQ ID NO:46-Explanation of artificial sequence: Synthetic DNA
SEQ ID NO:47-Explanation of artificial sequence: Synthetic DNA
SEQ ID NO:48-Explanation of artificial sequence: Synthetic DNA
SEQ ID NO:49-Explanation of artificial sequence: Synthetic DNA
SEQ ID NO:50-Explanation of artificial sequence: Synthetic DNA
SEQ ID NO:51-Explanation of artificial sequence: Synthetic DNA
SEQ ID NO:52-Explanation of artificial sequence: Synthetic DNA
SEQ ID NO:54-Explanation of artificial sequence: Synthetic DNA
SEQ ID NO:55-Explanation of artificial sequence: Synthetic DNA
SEQ ID NO:56-Explanation of artificial sequence: Synthetic DNA
SEQ ID NO:57-Explanation of artificial sequence: Synthetic DNA
SEQ ID NO:58-Explanation of artificial sequence: Synthetic DNA
SEQ ID NO:59-Explanation of artificial sequence: Synthetic DNA
SEQ ID NO:60-Explanation of artificial sequence: Synthetic DNA
SEQ ID NO:61-Explanation of artificial sequence: Synthetic DNA
SEQ ID NO:62-Explanation of artificial sequence: Synthetic DNA
SEQ ID NO:63-Explanation of artificial sequence: Synthetic DNA

## Claims

1. A preventive agent, a diagnostic agent or a therapeutic agent for interstitial pneumonia, which comprises an anti-CCR4 antibody and/or an anti-CXCR3 antibody as an active ingredient.

2. The preventive agent, the diagnostic agent or the therapeutic agent according to claim 1, wherein the interstitial pneumonia is usual idiopathic interstitial pneumonia or non-specific idiopathic interstitial pneumonia.

3. The preventive agent, the diagnostic agent or the therapeutic agent according to claim 1, wherein the interstitial pneumonia is interstitial pneumonia in collagen disease.

4. A diagnostic agent for discriminating between usual idiopathic interstitial pneumonia and non-specific idiopathic interstitial pneumonia, which comprises an anti-CCR4 antibody and an anti-CXCR3 antibody as an active ingredient.

5. The preventive agent, the diagnostic agent or the therapeutic agent according to any one of claims 1 to 4, wherein the anti-CCR4 antibody is an antibody which specifically binds to an extracellular region of CCR4.

6. The preventive agent, the diagnostic agent or the therapeutic agent according to claim 5, wherein the extracellular region is an extracellular region selected from the group consisting of positions 1 to 39, 98 to 112, 176 to 206 and 271 to 284 in the amino acid sequence represented by SEQ ID NO:1.

7. The preventive agent, the diagnostic agent or the therapeutic agent according to claim 5, wherein the extracellular region is an epitope present in positions 2 to 29 in the amino acid sequence represented by SEQ ID NO:1.

8. The preventive agent, the diagnostic agent or the therapeutic agent according to claim 5, wherein the extracellular region is an epitope present in positions 13 to 29 in the amino acid sequence represented by SEQ ID NO:1.

9. The preventive agent, the diagnostic agent or the therapeutic agent according to any one of claims 1 to 8, wherein the anti-CCR4 antibody is a polyclonal antibody, a monoclonal antibody or an antibody fragment thereof.

10. The preventive agent, the diagnostic agent or the therapeutic agent according to any one of claims 1 to 8, wherein the anti-CCR4 antibody is a recombinant antibody or an antibody fragment thereof.

11. The preventive agent, the diagnostic agent or the therapeutic agent according to claim 10, wherein the recombinant antibody is an antibody selected from the group consisting of a humanized antibody and an antibody fragment thereof.

12. The preventive agent, the diagnostic agent or the therapeutic agent according to claim 11, wherein the humanized antibody or the antibody fragment thereof comprises:
complementarity determining region (CDR) 1, CDR2 and CDR3 of an antibody heavy chain (H chain) variable region (V region) having the amino acid sequences represented by SEQ ID NOs:2, 3 and 4, respectively, and/or
CDR1, CDR2 and CDR3 of an antibody light chain (L chain) V region having the amino acid sequences represented by SEQ ID NOs:5, 6 and 7, respectively.

13. The preventive agent, the diagnostic agent or the therapeutic agent according to claim 11 or 12, wherein the humanized antibody is an antibody selected from the group consisting of a human chimeric antibody, a human complementarity determining region (CDR)-grafted antibody, and an antibody fragment thereof.

14. The preventive agent, the diagnostic agent or the therapeutic agent according to claim 13, wherein the human chimeric antibody or the antibody fragment thereof comprises:
a heavy chain (H chain) variable region (V region) and a light chain (L chain) V region of a monoclonal antibody against CCR4, and
an H chain constant region (C region) and an L chain C region of a human antibody.

15. The preventive agent, the diagnostic agent or the therapeutic agent according to claim 13 or 14, wherein the human chimeric antibody or the antibody fragment thereof comprises:
CDR1, CDR2 and CDR3 of an antibody heavy chain (H chain) variable region (V region) having the amino acid sequences represented by SEQ ID NOs:2, 3 and 4, respectively, and/or
CDR1, CDR2 and CDR3 of an antibody light chain (L chain) V region having the amino acid sequences represented by SEQ ID NOs:5, 6 and 7, respectively.

16. The preventive agent, the diagnostic agent or the therapeutic agent according to any one of claims 13 to 15, wherein the human chimeric antibody or the antibody fragment thereof comprises:
a heavy chain (H chain) variable region (V region) of an antibody comprising the amino acid sequence represented by SEQ ID NO:8, and/or
a light chain (L chain) V region of an antibody comprising the amino acid sequence represented by SEQ ID NO:9.

17. The preventive agent, the diagnostic agent or the therapeutic agent according to any one of claims 13 to 16, wherein the human chimeric antibody or the antibody fragment thereof is an antibody KM2760 produced by a transformant KM2760 (FERM BP-7054).

18. The preventive agent, the diagnostic agent or the therapeutic agent according to claim 13, wherein the human CDR-grafted antibody or the antibody fragment thereof comprises CDRs of a heavy chain (H chain) variable region (V region) and a light chain (L chain) V region of a monoclonal antibody against CCR4.

19. The preventive agent, the diagnostic agent or the therapeutic agent according to claim 13, wherein the human CDR-grafted antibody or the antibody fragment thereof comprises:
CDRs of a heavy chain (H chain) variable region (V region) and a light chain (L chain) V region of a monoclonal antibody against CCR4, and
framework regions (FRs) of an H chain V region and an L chain V region of a human antibody.

20. The preventive agent, the diagnostic agent or the therapeutic agent according to claim 13, wherein the human CDR-grafted antibody or the antibody fragment thereof comprises:
CDRs of a heavy chain (H chain) variable region (V region) and a light chain (L chain) V region of a monoclonal antibody against CCR4,
framework regions (FRs) of an H chain V region and an L chain V region of a human antibody, and
an H chain constant region (C region) and an L chain C region of a human antibody.

21. The preventive agent, the diagnostic agent or the therapeutic agent according to any one of claims 13 and 18 to 20, wherein the human CDR-grafted antibody or the antibody fragment thereof comprises:
CDR1, CDR2 and CDR3 of an antibody heavy chain (H chain) variable region (V region) having the amino acid sequences represented by SEQ ID NOs:2, 3 and 4, respectively, and/or
CDR1, CDR2 and CDR3 of an antibody light chain (L chain) V region having the amino acid sequences represented by SEQ ID NOs:5, 6 and 7, respectively.

22. The preventive agent, the diagnostic agent or the therapeutic agent according to any one of claims 13 and 18 to 21, wherein the human CDR-grafted antibody or the antibody fragment thereof comprises:
a heavy chain (H chain) variable region (V region) of an antibody comprising an amino acid sequence in which at least one amino acid residue selected from Ala at position 40, Gly at position 42, Lys at position 43, Gly at position 44, Lys at position 76 and Ala at position 97 in the amino acid sequence represented by SEQ ID NO:10 is substituted with other amino acid, and/or
a light chain (L chain) variable region (V region) of an antibody comprising an amino acid sequence in which at least one amino acid residue selected from Ile at position 2, Val at position 3, Gln at position 50 and Val at position 88 in the amino acid sequence represented by SEQ ID NO:12 is substituted with other amino acid.

23. The preventive agent, the diagnostic agent or the therapeutic agent according to any one of claims 13 and 18 to 21, wherein the human CDR-grafted antibody or the antibody fragment thereof comprises:
a heavy chain (H chain) V region of an antibody comprising an amino acid sequence in which at least one amino acid residue selected from Thr at position 28 and Ala at position 97 in the amino acid sequence represented by SEQ ID NO:11 is substituted with other amino acid, and/or
a light chain (L chain) variable region (V region) of an antibody comprising an amino acid sequence in which at least one amino acid residue selected from Ile at position 2, Val at position 3, Gln at position 50 and Val at position 88 in the amino acid sequence represented by SEQ ID NO:12 is substituted with other amino acid.

24. The preventive agent, the diagnostic agent or the therapeutic agent according to any one of claims 13 and 18 to 21, wherein the human CDR-grafted antibody or the antibody fragment thereof comprises:
a heavy chain (H chain) variable region (V region) of an antibody comprising the amino acid sequence selected from SEQ ID NOs:10, 11 and 13 to 18, and/or
a light chain (L chain) V region of an antibody comprising the amino acid sequence selected from SEQ ID NOs:12 and 19 to 21.

25. The preventive agent, the diagnostic agent or the therapeutic agent according to any one of claims 13 and 18 to 21, wherein the human CDR-grafted antibody or the antibody fragment thereof comprises:
a heavy chain (H chain) variable region (V region) of an antibody comprising the amino acid sequence represented by SEQ ID NO:13, and/or
a light chain (L chain) V region of an antibody comprising the amino acid sequence represented by SEQ ID NO:21.

26. The preventive agent, the diagnostic agent or the therapeutic agent according to any one of claims 13 and 18 to 21, wherein the human CDR-grafted antibody or the antibody fragment thereof comprises:
a heavy chain (H chain) variable region (V region) of an antibody comprising the amino acid sequence represented by SEQ ID NO:14, and/or
a light chain (L chain) V region of an antibody comprising the amino acid sequence represented by SEQ ID NO:21.

27. The preventive agent, the diagnostic agent or the therapeutic agent according to any one of claims 13 and 18 to 21, wherein the human CDR-grafted antibody or the antibody fragment thereof is a human CDR-grafted antibody produced by a transformant selected from the group consisting of FERM BP-8129 and FERM BP-8130 or an antibody fragment thereof.

28. The preventive agent, the diagnostic agent or the therapeutic agent according to any one of claims 9 to 27, wherein the antibody fragment is an antibody fragment selected from Fab, Fab', F(ab')₂, a single chain antibody (scFv), a dimerized variable region (V region) fragment (Diabody), a disulfide-stabilized V region fragment (dsFv) and a peptide comprising CDR.

29. The preventive agent, the diagnostic agent or the therapeutic agent according to any one of claims 9 to 28, wherein the antibody or the antibody fragment thereof according to any one of claims 9 to 28 is chemically or genetically bound to a radioisotope, a protein or an agent.

30. A method for discriminating between usual idiopathic interstitial pneumonia and non-specific idiopathic interstitial pneumonia, which comprises detecting and/or determining a Th2 cell and a Th1 cell in a sample by using an anti-CCR4 antibody and an anti-CXCR3 antibody.

31. The method according to claim 30, wherein the anti-CCR4 antibody is an antibody which specifically binds to an extracellular region of CCR4.

32. The method according to claim 31, wherein the extracellular region is an extracellular region selected from the group consisting of positions 1 to 39, 98 to 112, 176 to 206 and 271 to 284 in the amino acid sequence represented by SEQ ID NO:1.

33. The method according to claim 31, wherein the extracellular region is an epitope present in positions 2 to 29 in the amino acid sequence represented by SEQ ID NO:1.

34. The method according to claim 31, wherein the extracellular region is an epitope present in positions 13 to 29 in the amino acid sequence represented by SEQ ID NO:1.

35. The method according to any one of claims 31 to 34, wherein the anti-CCR4 antibody is a polyclonal antibody, a monoclonal antibody or an antibody fragment thereof.

36. The method according to any one of claims 31 to 34, wherein the anti-CCR4 antibody is a recombinant antibody or an antibody fragment thereof.

37. The method according to claim 36, wherein the recombinant antibody is an antibody selected from a humanized antibody and an antibody fragment thereof.

38. The method according to claim 37, wherein the humanized antibody or the antibody fragment thereof comprises:
complementarity determining region (CDR) 1, CDR2 and CDR3 of an antibody heavy chain (H chain) variable region (V region) having the amino acid sequences represented by SEQ ID NOs:2, 3 and 4, respectively, and/or
CDR1, CDR2 and CDR3 of an antibody light chain (L chain) V region having the amino acid sequences represented by SEQ ID NOs:5, 6 and 7, respectively.

39. The method according to claim 37 or 38, wherein the humanized antibody is an antibody selected from a human chimeric antibody, a human complementarity determining region (CDR)-grafted antibody, and an antibody fragment thereof.

40. The method according to claim 39, wherein the human chimeric antibody or the antibody fragment thereof comprises:
a heavy chain (H chain) variable region (V region) and a light chain (L chain) V region of a monoclonal antibody against CCR4, and
an H chain constant region (C region) and an L chain C region of a human antibody.

41. The method according to claim 39 or 40, wherein the human chimeric antibody or the antibody fragment thereof comprises:
CDR1, CDR2 and CDR3 of an antibody heavy chain (H chain) variable region (V region) having the amino acid sequences represented by SEQ ID NOs:2, 3 and 4, respectively, and/or
CDR1, CDR2 and CDR3 of an antibody light chain (L chain) V region having the amino acid sequences represented by SEQ ID NOs:5, 6 and 7, respectively.

42. The method according to any one of claims 39 to 41, wherein the human chimeric antibody or the antibody fragment thereof comprises:
a heavy chain (H chain) variable region (V region) of an antibody comprising the amino acid sequence represented by SEQ ID NO:8, and/or
a light chain (L chain) V region of an antibody comprising the amino acid sequence represented by SEQ ID NO:9.

43. The method according to any one of claims 39 to 42, wherein the human chimeric antibody or the antibody fragment thereof is an antibody KM2760 produced by a transformant KM2760 (FERM BP-7054).

44. The method according to claim 39, wherein the human CDR-grafted antibody or the antibody fragment thereof comprises CDRs of a heavy chain (H chain) variable region (V region) and a light chain (L chain) V region of a monoclonal antibody against CCR4.

45. The method according to claim 39, wherein the human CDR-grafted antibody or the antibody fragment thereof comprises:
CDRs of a heavy chain (H chain) variable region (V region) and a light chain (L chain) V region of a monoclonal antibody against CCR4, and
framework regions (FRs) of an H chain V region and an L chain V region of a human antibody.

46. The method according to claim 39, wherein the human CDR-grafted antibody or the antibody fragment thereof comprises:
CDRs of a heavy chain (H chain) variable region (V region) and a light chain (L chain) V region of a monoclonal antibody against CCR4,
framework regions (FRs) of an H chain V region and an L chain V region of a human antibody, and
an H chain constant region (C region) and an L chain C region of a human antibody.

47. The method according to any one of claims 39 and 44 to 46, wherein the human CDR-grafted antibody or the antibody fragment thereof comprises:
CDR1, CDR2 and CDR3 of an antibody heavy chain (H chain) variable region (V region) having the amino acid sequences represented by SEQ ID NOs:2, 3 and 4, respectively, and/or
CDR1, CDR2 and CDR3 of an antibody light chain (L chain) V region having the amino acid sequences represented by SEQ ID NOs:5, 6 and 7, respectively.

48. The method according to any one of claims 39 and 44 to 47, wherein the human CDR-grafted antibody or the antibody fragment thereof comprises:
a heavy chain (H chain) variable region (V region) of an antibody comprising an amino acid sequence in which at least one amino acid residue selected from Ala at position 40, Gly at position 42, Lys at position 43, Gly at position 44, Lys at position 76 and Ala at position 97 in the amino acid sequence represented by SEQ ID NO:10 is substituted with other amino acid, and/or
a light chain (L chain) variable region (V region) of an antibody comprising an amino acid sequence in which at least one amino acid residue selected from Ile at position 2, Val at position 3, Gln at position 50 and Val at position 88 in the amino acid sequence represented by SEQ ID NO:12 is substituted with other amino acid.

49. The method according to any one of claims 39 and 44 to 47, wherein the human CDR-grafted antibody or the antibody fragment thereof comprises:
a heavy chain (H chain) variable region (V region) of an antibody comprising an amino acid sequence in which at least one amino acid residue selected from Thr at position 28 and Ala at position 97 in the amino acid sequence represented by SEQ ID NO:11 is substituted with other amino acid, and/or
a antibody light chain (L chain) variable region (V region) of an antibody comprising an amino acid sequence in which at least one amino acid residue selected from Ile at position 2, Val at position 3, Gln at position 50 and Val at position 88 in the amino acid sequence represented by SEQ ID NO:12 is substituted with other amino acid.

50. The method according to any one of claims 39 and 44 to 47, wherein the human CDR-grafted antibody or the antibody fragment thereof comprises:
a heavy chain (H chain) variable region (V region) of an antibody comprising the amino acid sequence selected from SEQ ID NOs:10, 11 and 13 to 18, and/or
a light chain (L chain) V region of an antibody comprising the amino acid sequence selected from SEQ ID NOs:12 and 19 to 21.

51. The method according to any one of claims 39 and 44 to 47, wherein the human CDR-grafted antibody or the antibody fragment thereof comprises:
a heavy chain (H chain) variable region (V region) of an antibody comprising the amino acid sequence represented by SEQ ID NO:13, and/or
a light chain (L chain) V region of an antibody comprising the amino acid sequence represented by SEQ ID NO:21.

52. The method according to any one of claims 39 and 44 to 47, wherein the human CDR-grafted antibody or the antibody fragment thereof comprises:
a heavy chain (H chain) variable region (V region) of an antibody comprising the amino acid sequence represented by SEQ ID NO:14, and/or
a light chain (L chain) V region of an antibody comprising the amino acid sequence represented by SEQ ID NO:21.

53. The method according to any one of claims 39 and 44 to 47, wherein the human CDR-grafted antibody or the antibody fragment thereof is a human CDR-grafted antibody produced by a transformant selected from the group consisting of FERM BP-8129 and FERM BP-8130 or an antibody fragment thereof.

54. The method according to any one of claims 35 to 53, wherein the antibody fragment is an antibody fragment selected from Fab, Fab', F(ab')₂, a single chain antibody (scFv), a dimerized variable region (V region) fragment (Diabody), a disulfide-stabilized V region fragment (dsFv) and a peptide comprising CDR.

55. The method according to any one of claims 35 to 54, wherein the antibody or the antibody fragment thereof according to any one of claims 35 to 54 is chemically or genetically bound to a radioisotope, a protein or an agent.
